# EUROPEAN PATENT SPECIFICATION

(11) **EP 3 223 833 B1**
(45) Date of publication and mention of the grant of the patent: **15.05.2019**
(21) Application number: 15804915.5
(22) Date of filing: 24.11.2015
(51) Int. Cl.: A61K 35/644, A61P 17/02

(54) **PREVENTION AND TREATMENT OF MICROBIAL INFECTIONS**
VORBEUGUNG UND BEHANDLUNG VON MIKROBIELLEN INFEKTIONEN
PRÉVENTION ET TRAITEMENT D'INFECTIONS MICROBIENNES

(30) Priority: 24.11.2014 GB 201420856; 14.05.2015 GB 201508313; 03.06.2015 GB 201509654; 01.07.2015 GB 201511576; 23.07.2015 GB 201513047; 15.10.2015 GB 201518252; 04.11.2015 GB 201519483
(43) Date of publication of application: 04.10.2017
(73) Proprietor: Matoke Holdings Limited, Abingdon, Oxfordshire OX13 5HR (GB)
(72) Inventor: PATTON, Thomas, Collooney County Sligo (IE); BRENNAN, James, Hazelwood Co. Sligo (IE); STAPLES, Ian, Abingdon Oxfordshire OX13 5HR (GB); ELDER, Iain, Abingdon Oxfordshire OX13 5HR (GB); CALLAGHAN, Annette, Abingdon Oxfordshire OX13 5HR (GB); DRYDEN, Matthew, Abingdon Oxfordshire OX13 5HR (GB); BARRETT, John, Reginald, Templemore Co Tipperary (IE); KERSHAW, David, Abingdon Oxfordshire OX13 5HR (GB); SALIB, Rami., Horton Heath Eastleigh SO50 7FD (GB)
(74) Representative: Carridge, Andrew Edward
(86) International application number: PCT/GB2015/053584
(87) International publication number: WO 2016/083798

(56) References cited:
- WO-A1-2007/137881
- WO-A1-2012/030231
- WO-A1-2015/166197
- US-A1- 2010 098 645
- Fenella Halstead ET AL: "The in vitro antibacterial activity of engineered honey (Surgihoney(TM)) against important biofilm-forming burn wound pathogens BACKGROUND", Public Health England, 23 November 2014 (2014-11-23), XP055239578, Retrieved from the Internet: URL:http://www.surgihoney.com/pdf/pubmedpa pers/11.pdf [retrieved on 2016-01-08]
- MOLAN P C: "The antibacterial activity of honey 1. The nature of the antibacterial activity", BEE WORLD, BEE RESEARCH ASSOCIATION, GERRARDS CROSS, GB, vol. 1-2, 1 January 1992 (1992-01-01), pages 5-29, XP002902515, ISSN: 0005-772X
- JONATHAN COOKE ET AL: "The antimicrobial activity of prototype modified honeys that generate reactive oxygen species (ROS) hydrogen peroxide", BMC RESEARCH NOTES, BIOMED CENTRAL LTD, GB, vol. 8, no. 1, 28 January 2015 (2015-01-28), page 20, XP021210561, ISSN: 1756-0500, DOI: 10.1186/S13104-014-0960-4

## Description

This invention relates to the prevention and treatment of microbial infections, in particular microbial infections that include a biofilm , and treatment of chronic wounds, such as chronic skin and burn wounds.

Chronic wounds are those wounds which fail to progress as expected through the typical healing processes in a timely manner. A chronic wound has been defined as a wound that has been in existence for more than three weeks, or that has failed to proceed through an orderly and timely process to produce anatomic and functional integrity or to proceed through the repair process without establishing a sustained and functional result (Lazarus et al, Arch Dermatol. 1994;130(4):489-493).

Chronic wounds are a significant health problem (Cowan et al, Ulcers 2013, Article ID 487024). Health care costs related to the management and treatment of chronic wounds in the USA has been reported to exceed $20 billion annually. The treatment and management of non-healing wounds is challenging. Traditionally, basic wound care has consisted of surgical debridement, manual irrigation, moisture retentive dressings, and topical and/or systemic antimicrobial therapy. Although there has been tremendous progress in the science of wound healing, the prevalence and incidence of chronic wounds and their associated complications continue to escalate.

The presence and complexity of bacterial biofilms in chronic wounds have recently been recognized as key aspects of non-healing wounds. Bacterial biofilms are sessile colonies of polymicrobial organisms (bacterial, fungal, and possibly, viral), which are often symbiotic. These biofilm colonies produce a protective coating to protect the colonies from host defenses. The character of this protective substance unique to biofilms is dynamic, and the production of its components seems to be triggered by hostile environments in the wound bed (such as the presence of topical antibiotics). Biofilms have been shown to have survival and defense mechanisms that inhibit the healing aspects of inflammatory cells, resist antibiotics (topical and systemic) and other therapies, and initiate cell-to-cell communication pathways (quorum sensing), which facilitate new biofilm growth, resulting in recalcitrant non-healing wounds.

A bacterial biofilm is characterized as an aggregated bacteria attached to a surface or formed at a surface interface and organized as a complex community embedded in a self-secreted extracellular polymeric substance (EPS). These dynamic bacterial communities may consist predominately of single bacterial or fungal species or, more commonly, may be polymicrobial, containing multiple diverse species that are continuously changing.

All biofilms, regardless of their location, share several common features. These include the synthesis of an extracellular polymeric matrix that holds the bacterial cells together, and an increase in resistance to killing by host defenses and antimicrobial agents compared with the resistance exhibited by free-living or 'planktonic' cells. The inherent protective nature of the biofilm colony makes most biofilm-associated infections difficult or impossible to eradicate.

Biofilm development can be divided into three distinct stages (Kaplan, J Dent Res 89(3) 2010: 205-218): attachment of cells to a surface, growth of the cells into a sessile biofilm colony, and detachment of cells from the colony into the surrounding medium. The initial, reversible interaction between a bacterial cell and a surface is mediated by non-specific Lifshitz-van der Waals, Lewis acid-base, and electrostatic forces. This transient attachment is reinforced by host- and tissue-specific adhesins that are located on the bacterial cell surface or on cellular appendages such as pili and fimbriae. This results in the irreversible attachment of the bacterial cell to the surface.

The second stage of biofilm development involves the multiplication of bacteria on the surface and the concomitant synthesis of an extracellular polymeric matrix. The matrix holds the bacterial cells together in a mass and firmly attaches the bacterial mass to the underlying surface. Some examples of polymeric biofilm matrix components include glucan polysaccharides, proteinaceous fimbriae, and extracellular, double-stranded DNA. In addition to providing a structural 'scaffold' for the biofilm colony, the matrix also contributes to biofilm-mediated antimicrobial resistance, either by acting as a diffusion barrier, or by binding directly to antimicrobial agents and preventing their access to the biofilm cells.

Continued growth of bacterial cells on a surface leads to the development of mature biofilm colonies containing millions of tightly packed cells gathered into pillar- and mushroom-shaped masses that project outward into the surrounding medium for hundreds of microns. These structures are interspersed with fluid-filled channels which act as a primitive circulatory system, allowing for the exchange of nutrients and waste products with the bulk fluid phase. In addition, masses of biofilm cells often contain demarcated internal spaces that are devoid of cells. Thus, mature biofilm colonies are complex, highly differentiated structures. Numerous microenvironments that differ with respect to pH, oxygen concentration, nutrient availability, and cell density exist within the biofilm colony. This results in a great deal of heterogeneity in metabolic and reproductive activity among cells located in different parts of the colony. Metabolically inactive cells located in the interior of the colony may be resistant to the actions of antimicrobial agents that target actively growing cells.

The final stage of biofilm development is the detachment of cells from the biofilm colony and their dispersal ('seeding') into the environment. This is an essential stage of the biofilm life cycle that contributes to biological dispersal, bacterial survival, and disease transmission. Like other stages of biofilm development, dispersal can be a complex process that involves numerous environmental signals, signal transduction pathways, and effectors. No single mechanism of biofilm dispersal is utilized by all bacteria.

Biofilms have been identified on various surfaces of the body including the teeth (plaque), endocardium, gastrointestinal and genitourinary mucosa, and nasal epithelium, as well as foreign objects such as orthopedic prosthetics and invasive catheters.

Evidence suggests that biofilms are strongly associated with impaired wound healing in chronic skin wounds. Wound biofilms trigger a chronic inflammatory response resulting in accumulation of neutrophils and macrophages surrounding biofilms. The neutrophils and macrophages secrete high levels of reactive oxygen species (ROS) that affect the biofilm and the surrounding tissues. Inflammatory cells also secrete high levels of proteases (matrix metalloproteinases and elastase) that can help break down the attachments between biofilms and the affected tissue, dislodging the biofilms from the tissue. However, the ROS and proteases also have the capacity to damage the normal surrounding tissue, proteins, immune cells, and tissue cells, delaying healing.

In vulnerable tissue, biofilms are created by planktonic bacteria attaching and forming a protective community before they are killed by the patient's immune system, antibiotics, or by debridement. Several conditions which impair the immune system or reduce the effectiveness of antibiotic drugs encourage the development and spread of biofilms in wounds. These include ischemia or necrosis of tissues, nutritional deficits or compromise, and comorbidities that impair the body's immune function, such as HIV, diabetes, major physical trauma, radiation treatment, or treatment with immune-suppressing drugs.

It has been suggested that the processes employed by biofilms include molecular mechanisms which enable bacteria to attach to host cells and inject proteins to reorganize host cellular pathways. For some bacterial species, the injected bacterial proteins reorganize the host cellular cytoskeleton and prevent migration and mitosis, and inhibit apoptosis. As bacteria begin to form a biofilm, their molecular mechanisms may attract other bacteria to form a sustainable polymicrobial system. A biofilm colony is thought to possess an expanded diverse gene pool representing numerous species of bacteria. Long-term biofilm survival is often directly related to the genetic diversity of the biofilms, resulting in chronic infections that become recalcitrant to treatment. Survival of a bacterial biofilm requires gene expression to ensure attachment to the host, cellular senescence of the host to prevent shedding and to cause local inflammation, and stimulation of the production of plasma in the wound bed to nourish the biofilm colony.

Microorganisms that have the ability to form biofilms also possess quorum-sensing molecules to direct the focus and organization of the biofilm. Directed secretion of molecules and organization of the colonies in biofilms maximize the availability of nutrients and other essential molecules while minimizing the opposing effects of waste products, toxins of competitors, and other environmental hazards on the biofilms. Polymicrobial biofilms likely incorporate quorum-sensing molecules that can regulate pathways and also perform bidirectional signaling. Biofilm organisms have the ability to sense and communicate with many quorum-sensing pathways.

Biofilms have numerous defenses and can be resistant to treatment, limiting the effectiveness of antibiotics. Antibiotics and antiseptics kill single bacteria very easily, but the biofilm barrier blocks most antibiotics and antiseptics from reaching the bacteria, particularly towards the center of the wound matrix. Wound biofilms are resistant to antibodies, antibiotics, disinfectants, and phagocytic inflammatory cells.

There is, therefore, a need to provide effective therapies to prevent and treat microbial infections that include biofilms or microbes capable of forming biofilms, particularly in chronic wounds, such as chronic skin and burn wounds.

Chronic rhinosinusitis (CRS) is a common disorder characterized by mucosal inflammation of the nose and paranasal sinuses with sinonasal symptoms persisting for greater than 12 weeks. The most simplified classification divides CRS into those patients who have nasal polyps (CRSwNP) and those without (CRSsNP). CRS causes significant physical impairment, adversely impacting patients' quality of life and health care expenditure. Medical therapy of CRS is a key strategy, with surgery playing a vital adjunctive role.

The role of microbes as causative agents in CRS is not clear, but microbial infection and biofilms may contribute to the propagation of CRS. *S. aureus* is the most common bacterial pathogen identified in CRS patients in Western countries. Coagulase-negative *Staphylococcus* and anaerobic and Gram-negative bacteria are also commonly cultured from CRS patients. In post-surgical patients, *Pseudomonas, Klebsiella, Enterobacter,* and *Staphylococcus* species predominate. Bacterial biofilms, which are largely absent in controls, have been recovered from both CRSsNP and CRSwNP patients, with reported rates varying between 30% and 100%.

There is no standard management of CRS. Treatment strategies differ based on divergent etiologies of the various CRS subclasses. A variety of systemic and topical therapeutic agents are commonly employed. These include corticosteroids, antimicrobials, and immune modulating medications. As CRS is a chronic disease, there are concerns related to the use of systemic agents over prolonged periods. Long-term use of corticosteroids and antibiotics may lead to adverse effects, drug interactions, and antimicrobial resistance. The development of topical therapies delivered directly to the sinonasal cavity have created alternative treatment strategies. Many therapeutic agents can now be delivered into the sinonasal cavity by a variety of delivery methods, such as irrigations, sprays, and aerosols.

There is also a need, therefore, to provide effective therapies to prevent and treat CRS, in particular to prevent and treat biofilms or microbes capable of forming biofilms that are associated with CRS.

The Applicant has found that compositions that are able to release hydrogen peroxide at the site of a microbial infection are particularly effective at preventing or inhibiting formation of biofilms, and in preventing or inhibiting growth or seeding of existing biofilms. The Applicant has found that such compositions are effective against formation of biofilms, and growth or seeding of existing biofilms, produced by bacteria of burn wounds and other chronic wounds, and are superior to several commercially available wound dressings.

According to the invention, there is provided a composition for generating anti-microbial activity for use in the treatment of a microbial infection that comprises a biofilm, wherein the composition comprises an enzyme that is able to convert a substrate to release hydrogen peroxide, and a substance that includes a substrate for the enzyme, wherein the composition does not include sufficient free water to allow the enzyme to convert the substrate, and wherein the enzyme is additional to any enzyme activity able to convert the substrate to release hydrogen peroxide that may be present in the substance.

According to the disclosure there is provided a composition for generating anti-microbial activity for use in the prevention or treatment of a microbial infection that comprises a biofilm, or a microbe that is capable of forming a biofilm, wherein the composition comprises an enzyme that is able to convert a substrate to release hydrogen peroxide, and a substance that includes a substrate for the enzyme.

According to the disclosure there is also provided use of a composition for generating anti-microbial activity in the manufacture of a medicament for use in the prevention or treatment of a microbial infection that comprises a biofilm, or a microbe that is capable of forming a biofilm, wherein the composition comprises an enzyme that is able to convert a substrate to release hydrogen peroxide, and a substance that includes a substrate for the enzyme.

There is also provided according to the disclosure a method of preventing or treating a microbial infection that includes a biofilm, or a microbe that is capable of forming a biofilm, wherein the method comprises administering an effective amount of a composition for generating anti-microbial activity to a site of the infection, wherein the composition comprises an enzyme that is able to convert a substrate to release hydrogen peroxide, and a substance that includes a substrate for the enzyme.

There is also provided according to the disclosure an *in vitro* method for preventing or inhibiting formation of a biofilm, which comprises contacting an effective amount of a composition with a microbe that is capable of forming a biofilm, wherein the composition comprises an enzyme that is able to convert a substrate to release hydrogen peroxide, and a substance that includes a substrate for the enzyme.

There is further provided according to the disclosure an *in vitro* method for preventing or inhibiting growth or seeding of an existing biofilm, which comprises contacting an effective amount of a composition with an existing biofilm, wherein the composition comprises an enzyme that is able to convert a substrate to release hydrogen peroxide, and a substance that includes a substrate for the enzyme.

The Examples below describe *in vitro* methods that can be used to test whether a microbe is capable of forming a biofilm, for detecting the presence and amount of a biofilm (using crystal violet), and for determining whether formation of a biofilm, or growth or seeding of a biofilm, has been prevented or inhibited.

The composition may prevent or inhibit growth or seeding of the biofilm, or prevent or inhibit formation of a biofilm by the microbe.

The microbial infection may be a chronic microbial infection, for example an infection that has been in existence for more than three weeks, for example more than a month, or more than two or three months, or a year.

The microbial infection may be a wound infection, such as a skin wound infection, or a burn wound infection.

A wound occurs when the integrity of any tissue is compromised (e.g. skin breaks, muscle tears, burns, or bone fractures). A wound may be caused by an act (a trauma) or surgical procedure, by an infectious disease, or by an underlying condition.

The microbial infection may be present in a chronic wound, for example a wound that has been in existence for more than three weeks, or that has failed to proceed through an orderly and timely process to produce anatomic and functional integrity or to proceed through the repair process without establishing a sustained and functional result (Lazarus et al, Arch Dermatol. 1994;130(4):489-493).

Chronic wounds include burn wounds, venous ulcers, arterial ulcers, diabetic ulcers, and pressure ulcers. The microbial infection may comprise a bacterium, preferably a Gram-negative bacterium, that is capable of forming a biofilm.

The biofilm may comprise any of the following species of bacteria, or the microbe capable of forming a biofilm may be any of the following species of bacteria: *Pseudomonas aeruginosa; Acinetobacter baumannii.*

The Applicant has found that compositions of the invention are also effective at treating CRS-associated biofilms, in particular a CRS-associated *Staphylococcus aureus* biofilm (Methicillin-resistant *Staphylococcus aureus* (MRSA) biofilm, or Methicillin-sensitive *Staphylococcus aureus* (MSSA) biofilm).

CRS is diagnosed when specific sinonasal symptoms lasting 12 or more weeks are confirmed by nasal endoscopy or radiographic imaging: a duration of 12 or more weeks of 2 or more of the following: mucopurulent drainage, nasal obstruction, facial pain/pressure/fullness, decreased sense of smell; and inflammation by one or more objective criteria: endoscopy: pus, mucosal edema or polyps; imaging showing inflammation of the paranasal sinuses (Infection and Drug Resistance 2013:6,1-14).

Accordingly, the microbial infection may be a sinus infection, such as CRS, particularly a CRS-associated biofilm.

The sinus or CRS microbial infection may comprise bacteria of any of the following species: *Pseudomonas; Klebsiella; Enterobacter; Staphylococcus.* The biofilm may comprise any of the following species of bacteria, or the microbe capable of forming a biofilm may be any of the following species of bacteria: *Pseudomonas; Klebsiella; Enterobacter; Staphylococcus.*

Compositions described herein may be used to treat wounds that are critically colonized. The term "critically colonized" is often used to refer to a wound that has reached a critical point at which bacteria begin to negatively affect the wound and begin to elicit signs of their presence. A critically colonized wound may indicate the presence of a biofilm. A bacterial load of greater than 10⁵ organisms/gram of tissue is often accepted as impeding wound healing (Siddiqui AR, Bernstein JM (2010) Chronic wound infection: Facts and controversies. Clinics in Dermatology 28: 519-26; Edmonds, M., & Foster, A. (2004). The use of antibiotics in the diabetic foot. Am J Surg, 187(5A), 25S-28S).

Consequently, according to the disclosure there is provided a composition for generating anti-microbial activity for use in the treatment of a wound having a bacterial load of greater than 10⁵ organisms/gram of tissue, wherein the composition comprises an enzyme that is able to convert a substrate to release hydrogen peroxide, and a substance that includes a substrate for the enzyme.

According to the disclosure there is also provided use of a composition for generating anti-microbial activity in the manufacture of a medicament for use in the treatment of a wound having a bacterial load of greater than 10⁵ organisms/gram of tissue, wherein the composition comprises an enzyme that is able to convert a substrate to release hydrogen peroxide, and a substance that includes a substrate for the enzyme.

There is also provided according to the disclosure n a method of treating a wound having greater than 10⁵ organisms/gram of tissue, wherein the method comprises administering an effective amount of a composition for generating anti-microbial activity to the wound, wherein the composition comprises an enzyme that is able to convert a substrate to release hydrogen peroxide, and a substance that includes a substrate for the enzyme.

The enzyme of the composition for use according to the invention is additional (i.e. added as a result of human intervention) to any enzyme activity able to convert the substrate to release hydrogen peroxide (referred to herein as "substrate conversion activity") that may be present in the substance. The composition may be a storage-stable composition which does not include sufficient free water to allow the enzyme to convert the substrate.

The composition may be a storage-stable composition for generating antimicrobial activity, which comprises: a purified enzyme that is able to convert a substrate to release hydrogen peroxide; and a substance that includes a substrate for the enzyme; wherein the composition does not include sufficient free water to allow the enzyme to convert the substrate.

The composition may be a storage-stable composition for generating antimicrobial activity, which comprises: an enzyme that is able to convert a substrate to release hydrogen peroxide; and a substance that lacks catalase activity and that includes a substrate for the enzyme; wherein the composition does not include sufficient free water to allow the enzyme to convert the substrate.

In the presence of sufficient water, the enzyme of the storage-stable composition is able to convert the substrate and release hydrogen peroxide. Hydrogen peroxide is known to be effective against a wide variety of different microbes. Thus, antimicrobial activity is generated following dilution of a storage-stable composition of the invention.

If a storage-stable composition is used, this may be diluted by liquid present at the site of administration (for example, by exudate from a wound), leading to release of hydrogen peroxide at the administration site.

Catalase is an enzyme that catalyses the decomposition of hydrogen peroxide to water and oxygen. The use of a substance that lacks catalase activity means that there is no variability in the amount of this activity between similar substances from different sources, or from different harvests from the same source. This reduces the variability in antimicrobial activity that can be generated from such substances. Alternatively, if the substance does include catalase activity, and it is not possible or desirable to inactivate the catalase activity in the substance prior to contacting the substance with the enzyme, then sufficient enzyme may be used such that the effect of catalase activity on the hydrogen peroxide that can be generated from the substance is reduced. This also reduces the variability in antimicrobial activity that can be generated from the substance. In some embodiments, the substance may lack catalase activity.

Catalase is present in many plants and animals. Catalase activity may be removed during processing or extraction of the substance, or inactivated before use of the substance in the composition. Catalase activity may be heat inactivated, for example by pasteurisation. A suitable temperature for heat inactivation of catalase activity is at least 60°C, 70°C, or 80°C, preferably for at least 2 minutes.

The term "storage-stable" is used herein to mean that the composition can be stored at ambient temperature for at least several days, suitably at least a week or at least one or two months, whilst retaining the ability to generate antimicrobial activity following dilution of the composition. The storage temperature may be below 37°C, preferably 20-25°C. Preferably compositions are stored away from exposure to light.

Hydrogen peroxide is generally unstable at ambient temperature. The lack of sufficient free water in a storage-stable composition for use according to the invention prevents the enzyme converting the substrate to release hydrogen peroxide, and thus helps to maintain the stability of the composition for extended periods at ambient temperature. A storage-stable composition for use according to the invention may include some water provided that there is not sufficient free water to allow the enzyme to convert the substrate. Suitable amounts of water will vary depending on the precise components of the composition. However, typically, a storage-stable composition for use according to the invention comprises less than 20% total water content, for example, 10%-19%, water.

Hydrogen peroxide may be released for a sustained period following dilution of the composition, depending on the amount of substrate present in the composition, and the activity of the enzyme. It will be appreciated that the amount of substrate and/or the activity of enzyme in the composition may be selected to provide for release of a relatively high level of hydrogen peroxide for a short period, or for release of a lower level of hydrogen peroxide for a longer period, following dilution of the composition. Suitably the composition provides for sustained release of hydrogen peroxide for a period of at least twenty four hours, more preferably at least forty eight hours, following dilution of the composition. Suitably the composition provides for sustained release of hydrogen peroxide at a level of less than 2 mmol/litre for a period of at least twenty four hours, following dilution of the composition.

A composition for use according to the invention, may comprise sufficient enzyme and substrate to provide for sustained release of at least 0.1, 0.5, 1 or 1.5 mmol/litre hydrogen peroxide for a period of at least 24 hours, more preferably 48 hours.

The enzyme present in a composition for use according to the invention is additional to any enzyme activity able to convert the substrate to release hydrogen peroxide (referred to herein as "substrate conversion activity") that may be present in the substance, i.e. the compositions comprise the substance and added enzyme. In some embodiments there may be no substrate conversion activity in the substance.

It will be appreciated that there should be sufficient enzyme present in a storage-stable composition for use according to the invention to convert the substrate and form hydrogen peroxide as needed following dilution of the composition.

In view of the importance of generation of hydrogen peroxide by storage-stable compositions for use according to the invention in the presence of sufficient water, it will be appreciated that the compositions should not contain any added peroxidase.

In some examples, compositions of the disclosure or compositions for use in the disclosure may contain sufficient free water to allow the enzyme to convert the substrate, The composition may be an aqueous mixture. In some examples, such compositions may be able to produce hydrogen peroxide for an extended period of time. For example, the composition may be capable of producing hydrogen peroxide at a level described herein (e.g. at least 0.1, 0.5, 1 or 1.5 mmol/litre hydrogen peroxide) for at least a year, preferably at least 3 years. In some compositions that have sufficient free water to allow the enzyme to convert the substrate, there may be a reduction in the level of hydrogen peroxide produced, over time. This may result from fermentation of the substrate by fungi. Fermentation may reduce the amount of the substrate available for hydrogen peroxide production. In some examples, fermentation may be reduced by sealing the composition in a container or sachet that prevents any fungi from entering the composition and then treating the composition to kill fungi in the composition. For instance, this may be achieved by gamma irradiation.

In some embodiments the enzyme is a purified enzyme. The term "purified enzyme" is used herein to include an enzyme preparation in which the enzyme has been separated from at least some of the impurities originally present when the enzyme was produced. Preferably impurities that have been removed or reduced include those that would otherwise interfere with the ability of the enzyme to convert the substrate to release hydrogen peroxide.

It may not always be necessary or desirable that the purified enzyme is at a high level of purity provided that the enzyme is able to convert the substrate to release hydrogen peroxide. In some circumstances, it may be desirable to used a relatively crude enzyme preparation. Examples of suitable purity levels include at least 10%, 20%, 30%, 40%, 50%, 60%, 70%, 80%, or 90% pure.

It is preferred, however, that the amount of any catalase that may originally have been present when the enzyme was produced has been reduced. The enzyme may have been produced by recombinant or non-recombinant means, and may be a recombinant or non-recombinant enzyme. The enzyme may be purified from a microbial source, preferably from a non genetically modified microbe.

The level of purity of the enzyme may be selected as appropriate depending on the intended use of the composition. For example, if the composition is intended for medical use, a medical grade or medical device grade of purity should be used.

In some embodiments the enzyme is an oxidoreductase enzyme. Examples of oxidoreductase enzymes that can convert a substrate to release hydrogen peroxide include glucose oxidase, hexose oxidase, cholesterol oxidase, galactose oxidase, pyranose oxidase, choline oxidase, pyruvate oxidase, glycollate oxidase, and amioacid oxidase. The corresponding substrates for these oxidoreductase enzymes are D-glucose, hexose, cholesterol, D-galactose, pyranose, choline, pyruvate, glycollate and aminoacid, respectively.

A mixture of one or more oxidoreductase enzymes and one or more substrates for the oxidoreductase enzymes may be present in a composition for use according to the invention.

The oxidoreductase enzyme may be glucose oxidase, and the substrate may be D-glucose.

The substance may be any substance that includes a substrate for the enzyme. In some embodiments the substance lacks catalase activity. The substance may be an unrefined substance. The term "unrefined" is used herein to refer to substances that have not been processed into a pure form. Unrefined substances include substances that may have been concentrated, for example by drying or boiling.

The substance may include one or more substrates from a natural source (termed herein a "natural substance"). Examples of natural substances include substances from a plant source, including from sap, roots, nectar, flowers, seeds, fruit, leaves, or shoots. The substance may be an unrefined natural substance.

Suitably the substance comprises one or more of the following substrates: D-glucose, hexose, cholesterol, D-galactose, pyranose, choline, pyruvate, glycollate or aminoacid.

The substance may be a sugar substance. The term "sugar substance" is used herein to mean any substance that includes one or more sugars. The term "sugar" is used herein to refer to a carbohydrate with the general formula Cₘ(H₂O)ₙ. Preferred sugars include monosaccharides, such as D-glucose, hexose, or D-galactose. The sugar substance may include one or more sugars from a natural source (termed herein a "natural sugar substance"). The natural sugar substance may be an unrefined natural sugar substance. The unrefined natural sugar substance may be (or be derived from) a natural sugar product. In some embodiments, the unrefined natural sugar product is a honey. In some embodiments, the honey is a honey that has been treated to remove or inactivate catalase activity.

As discussed above, the substance itself may preferably lack an enzyme activity that is able to convert the substrate to release hydrogen peroxide (referred to as "substrate conversion activity"). Absence of substrate conversion activity from the substance has the advantage that there is then no variability in the amount of this activity between similar substances from different sources, or from different harvests from the same source. This further reduces the variability in antimicrobial activity that can be generated from such substances. Substrate conversion activity is then provided only by the enzyme that is contacted with the substance, and so the amount of substrate conversion activity present in the composition can be controlled.

Substrate conversion activity may be removed during processing or extraction of the substance, or inactivated before use of the substance in a composition for use according to the invention. Substrate conversion activity may be inactivated by heat inactivation, for example by pasteurisation. A suitable temperature for heat inactivation of substrate conversion activity is at least 80°C, preferably for at least two minutes. An advantage of heat inactivation is that both catalase activity and substrate conversion activity can be inactivated in a single heat inactivation step.

In some embodiments of the invention, the substance is a processed, extracted, or refined substance (i.e. a substance in which impurities or unwanted elements have been removed by processing). Preferably impurities that have been removed or reduced include those that would otherwise interfere with the ability of the enzyme to convert the substrate to release hydrogen peroxide.

In some embodiments of the invention, the substance comprises a purified substrate for the enzyme. The term "purified substrate" is used herein to include a substrate preparation in which the substrate has been separated from at least some of the impurities originally present when the substrate was obtained or produced. The purified substrate may be obtained from a natural source or may be synthetically produced. The purified substrate may be a processed, extracted, or refined substrate (i.e. a substrate in which impurities or unwanted elements have been removed by processing).

It may not always be necessary or desirable that the purified substrate is at a high level of purity provided that the enzyme is able to convert the substrate to release hydrogen peroxide. In some circumstances, it may be desirable to used a relatively crude substrate preparation. Examples of suitable purity levels include at least 10%, 20%, 30%, 40%, 50%, 60%, 70%, 80%, 90%, 95%, or 99% pure. However, in some embodiments, it may be desirable that the purified substrate is a medical grade, medical device grade, or pharmaceutical grade substrate.

In particular embodiments, the purified substrate is or comprises a purified sugar substance. The purified sugar substance may be obtained from a natural source (for example a processed, extracted, or refined natural sugar substance), or be synthetically produced. The purified sugar substance may be at least 10%, 20%, 30%, 40%, 50%, 60%, 70%, 80%, 90%, 95%, or 99% pure. The purified sugar substance may be a medical grade, medical device grade, or pharmaceutical grade sugar substance,. The purified sugar substance may include one or more purified sugar substances, for example purified D-glucose, hexose, or D-galactose. For example the purified sugar substance may be medical grade, medical device grade, or pharmaceutical grade D-glucose, hexose, or D-galactose.

In particular embodiments, the enzyme and the substrate are purified, for example purified glucose oxidase and purified D-glucose, suitably medical grade, medical device grade, or pharmaceutical grade glucose oxidase and D-glucose.

There is also provided according to the disclosure a composition for generating anti-microbial activity, wherein the composition comprises an enzyme that is able to convert a substrate to release hydrogen peroxide, and a substance that includes a purified substrate for the enzyme.

The enzyme of the composition is additional (i.e. added as a result of human intervention) to any enzyme activity able to convert the substrate to release hydrogen peroxide (referred to herein as "substrate conversion activity") that may be present in the substance. The composition may be a storage-stable composition which does not include sufficient free water to allow the enzyme to convert the substrate.

The composition may be a storage-stable composition for generating antimicrobial activity, which comprises: a purified enzyme that is able to convert a substrate to release hydrogen peroxide; and a substance that includes a purified substrate for the enzyme; wherein the composition does not include sufficient free water to allow the enzyme to convert the substrate.

The composition may be a storage-stable composition for generating antimicrobial activity, which comprises: an enzyme that is able to convert a substrate to release hydrogen peroxide; and a substance that lacks catalase activity and that includes a purified substrate for the enzyme; wherein the composition does not include sufficient free water to allow the enzyme to convert the substrate.

In the presence of sufficient water, the enzyme of the storage-stable composition is able to convert the substrate and release hydrogen peroxide.

There is also provided according to the disclosure, a pharmaceutical composition comprising a composition of the disclosure and a pharmaceutically acceptable carrier, excipient or diluent.

There is also provided according to the disclosure a composition of the disclosure for use as a medicament.

There is further provided according to the disclosure a composition of the disclosure for use in the prevention or treatment of a microbial infection, for example a microbial infection that comprises a biofilm, or a microbe that is capable of forming a biofilm. So, there may be provided a composition for use in the prevention or treatment of a microbial infection that comprises a biofilm or a microbe that is capable of forming a biofilm, wherein the composition comprises an enzyme that is able to convert a substrate to release hydrogen peroxide, and a substance that includes a purified substrate for the enzyme, wherein the enzyme is additional to any enzyme activity able to convert the substrate to release hydrogen peroxide that may be present in the substance.

There is also provided according to the disclosure use of a composition of the disclosure in the manufacture of a medicament for the prevention or treatment of a microbial infection, for example a microbial infection that comprises a biofilm, or a microbe that is capable of forming a biofilm. So, there may be provided use of a composition in the manufacture of a medicament for the prevention or treatment of a microbial infection that comprises a biofilm or a microbe that is capable of forming a biofilm, wherein the composition comprises an enzyme that is able to convert a substrate to release hydrogen peroxide, and a substance that includes a purified substrate for the enzyme, wherein the enzyme is additional to any enzyme activity able to convert the substrate to release hydrogen peroxide that may be present in the substance.

The disclosure also provides a method of preventing or treating a microbial infection, for example a microbial infection that comprises a biofilm, or a microbe that is capable of forming a biofilm, wherein the method comprises administering an effective amount of a composition of the disclosure to a site of the infection. So, there may be provided a method of preventing or treating a microbial infection that comprises a biofilm, or a microbe that is capable of forming a biofilm, wherein the method comprises administering an effective amount of a composition of the disclosure to a site of the infection, wherein the composition comprises an enzyme that is able to convert a substrate to release hydrogen peroxide, and a substance that includes a purified substrate for the enzyme, wherein the enzyme is additional to any enzyme activity able to convert the substrate to release hydrogen peroxide that may be present in the substance.

A storage-stable composition for use according to the invention may include an antimicrobial agent. For example, hydrogen peroxide may be present if the storage-stable composition is formed by contacting the enzyme with the substance in aqueous solution under conditions for conversion of the substrate by the enzyme, and then drying the composition to reduce its water content to a level where there is insufficient free water to allow the enzyme to convert the substrate. Preferably, however, the storage-stable antimicrobial composition does not include any detectable hydrogen peroxide. Such composition may be formed, for example, by contacting the enzyme with the substrate in the absence of sufficient free water to allow the enzyme to convert the substrate. Examples of other antimicrobial agents that may be present in a storage-stable composition of the invention include: an antibiotic, an antiviral agent, or an anti-fungal agent.

The composition may be a medical grade or medical device grade composition, or a pharmaceutical grade composition.

Each component of the composition may be a natural substance (i.e. each component is derived or purified from a natural source). Compositions for use according to the invention which contain only natural ingredients provide an attractive alternative to drug-based antimicrobial formulations.

Advantageously the substance is a honey. The honey may be a medical grade or medical device grade honey. In some embodiments, the honey is a honey that has been treated to remove or inactivate catalase activity originally present in the honey. According to an embodiment of the invention, the substance is a pasteurised honey, and the enzyme is a glucose oxidase. According to some embodiments, the substance is a medical grade or medical device grade honey, and the enzyme is a medical grade or medical device grade enzyme, suitably glucose oxidase.

Honey is a natural product made by honey bees using nectar from flowers. It is a saturated or super-saturated solution of sugars. Honey is defined in the Codex Alimentarius international food standard as "the natural sweet substance produced by honey bees from the nectar of plants or from secretions of living parts of plants or excretions of plant sucking insects on the living parts of plants, which the bees collect, transform by combining with specific substances of their own, deposit, dehydrate, store and leave in the honey comb to ripen and mature" (Revised Codex Standard for Honey, 2001).

Nectar typically includes approximately 14% simple sugars (w/w), 1% phenol compounds, and 85% water. The phenol compounds give the honey its taste, aroma and colour. In the warm conditions of the hive, typically 36°C, the nectar would very quickly ferment. To prevent this, the nectar is mixed with secretions, containing enzymes, from the salivary and hypopharyngeal glands of foraging bees. In the hive the nectar is passed from bee to bee and more secretions are added before it is stored in the cells of the hive. The amount of enzymes present varies with the age, diet and physiological stage of the bees (when a bee is a forager its glands produce more digestive enzymes), strength of the colony, temperature of the hive, and the nectar flow and its sugar content.

The enzymes added to nectar by bees include diastase, which catalyses the conversion of starch to dextrin and sugar, Invertase, which catalyses the conversion of sucrose to fructose and glucose, and glucose oxidase, which catalyses the conversion of glucose to hydrogen peroxide and gluconic acid. Low doses of hydrogen peroxide prevent the growth of yeasts that would quickly ferment the nectar. As the bees progressively dry the nectar to form honey, the gluconic acid makes the honey acidic (between pH 3.5 and 4.5). Water is effectively trapped to the sugar molecules in the honey and is not available for further chemical reactions. The amount of 'free' water in honey is measured as the water activity (a_{w}). The range of a_{w} found in honey has been reported to be 0.47-0.70, with mean values of 0.562 and 0.589 (RCIEGG, M; BLANC, B, 1981, The water activity of honey and related sugar solutions. Lebensmittel-Wissenschaft und Technologie 14: 1-6). The a_{w} of ripened honey is too low to support the growth of any species, with no fermentation occurring if the water content is below 17.1% (Molan, P. C. (1992). The antibacterial activity of honey: 1. The nature of the antibacterial activity. Bee World, 73(1), 5-28). The acidity of the honey and the lack of free water prevent the further risk of fermentation, and stop the glucose oxidase working. Honey also contains variable amounts of catalase originating from the nectar.

A typical chemical composition of blossom honey is:

**Table 1**

| Component | Blossom honey | |
|---|---|---|
| | Average (% w/w) | Min-Max (% w/w) |
| Water content | 17,2 | 15 - 20 |
| Fructose | 38,2 | 30 - 45 |
| Glucose | 31,3 | 24 - 40 |
| Sucrose | 0,7 | 0,1 - 4,8 |
| Other disaccharides | 5 | |
| Total sugars | 79,7 | |
| Minerals | 0,2 | 0,1 - 0,5 |
| Amino acids, Proteins | 0,3 | 0,2 - 0,8 |
| Acids | 0,5 | 0,2 -0,8 |
| pH | 3,9 | 3,5 - 4,5 |

In addition, trace amounts of pollen are present, which can be used to identify the botanical origin of the honey, as well as the enzymes invertase, diastase, catalase, and glucose oxidase. There is also phytochemical component. This varies but is typically up to ∼1%, depending on the source of the honey.

Once diluted, the glucose oxidase present in natural honey is able to convert glucose substrate in the diluted honey to release hydrogen peroxide. However, the variability in the content of honey (particularly in the content of glucose oxidase activity, glucose, and catalase activity) means that honeys from different sources, or different harvests of honey from the same source, can be very variable in their antimicrobial effectiveness.

According to an embodiment of the invention, the honey may be pasteurised. Pasteurisation of honey inactivates the catalase and glucose oxidase activity present in the honey. Optionally, the pasteurised honey may be filtered to remove any particles (such as wax particles and bee wings) that may be in the honey post harvest. To form a storage-stable composition of the invention, a glucose oxidase is contacted with the pasteurised honey once it has cooled to a temperature (suitably 35-40°C) that will not inactivate the added glucose oxidase and at which the honey remains sufficiently liquid to facilitate mixing with glucose oxidase.

Honey can be pasteurised at a temperature that is sufficient for the heat inactivation of catalase activity. A suitable minimum temperature is from 60°C to 80°C. This temperature should be maintained preferably for at least two minutes.

The control of the heat process may be important, since a bi-product of heating honey is the formation of HMF (HydroxyMethylFurfuraldehyde) which is used as an indicator of heat and storage changes in honey. HMF is formed by the breakdown of fructose in the presence of acid. Heat increases the speed of this reaction. The increase in speed is exponential with increasing heat. For every degree that the honey is raised above 40°C, close to the normal hive ambient temperature, HMF increases rapidly. HMF is not a harmful product. Jams, Molasses, Golden Syrup etc. can have levels of HMF 10 to 100 times that of honey. However HMF levels are used as an indication of degradation of honey and under the Codex Alimentarius Standard 40 mg/l is the maximum permissible level in the EU for table honey.

To prevent the build up of HMF it is preferred that the honey is raised rapidly to temperature levels to inactivate the catalase and then the honey is brought quickly down in temperature to a maximum of between 40 and 45°C using a heat exchange mechanism.

No water is added during the process of this preferred embodiment, and so the resulting composition does not include sufficient free water to allow the glucose oxidase to convert the glucose present to release hydrogen peroxide. The storage-stable composition comprises: pasteurised honey, and added glucose oxidase. There is no detectable hydrogen peroxide present. The composition can be stored at ambient temperature for at least several days.

In other embodiments of the invention, the honey may be unpasteurised.

According to some preferred embodiments, the honey (pasteurised or unpasteurised) is a creamed honey. Creamed honey is a honey that has been processed to control crystallization. Creamed honey contains a large number of small crystals, which prevent the formation of larger crystals that can occur in unprocessed honey. A method for producing creamed honey was described in U.S. Patent 1,987,893. In this process, raw honey is first pasteurised, then previously processed creamed honey is added to the pasteurized honey to produce a mixture of 10% creamed honey and 90% pasteurised honey. The mixture is then allowed to rest at a controlled temperature of 14°C. This method produces a batch of creamed honey in about one week. A seed batch can be made by allowing normal honey to crystallize and crushing the crystals to the desired size. Large scale producers have modified this process by using paddles to stir the honey mixture while holding the mixture at 14°C. In alternative creaming methods, the pasteurisation step may be omitted, with the honey instead being slowly warmed to 37°C.

In other embodiments of the invention, the honey (pasteurised or unpasteurised) is an uncreamed honey. For example, the honey may be a pasteurised, uncreamed honey.

The glucose oxidase may be a purified natural glucose oxidase preparation which is of medical grade or medical device grade for medical applications. The activity of the glucose oxidase may be selected depending on the desired rate of production of hydrogen peroxide following dilution of the storage-stable composition. Several glucose oxidase preparations are commercially available (glucose oxidase is identified by the reference CAS:9001-37-0). Common microbial sources for glucose oxidase from non genetically modified organisms include selected strains of *Aspergillus niger, Penicillium amagasakiense, Penicillium variabile, Penicillium notatum.* Medical device grade glucose oxidase, from GMO *Aspergillus niger,* is available from Biozyme UK, activity 240iu/mg. Food standard glucose oxidase, from *Aspergillus niger,* is available from BIO-CAT INC, activity 15,000 Units/g. Non-Genetically Modified glucose oxidase is available from BIO-CAT INC, activity 12,000/g. Glucose oxidase (GO3B2), from *Apsergillus niger,* is available from BBI Enzymes Limited, activity 360 Units/mg. Contaminants: alpha amylase no greater than 0.05%, Saccharase no greater than 0.05%, maltase no greater than 0.05% and GO/Cat no less than 2000.

The enzyme activity (for example, the glucose oxidase activity) may range, for example, from 1-400 IU/mg, or 1-300 IU/mg, for example 250-280 IU/mg. The amount of enzyme used is likely to depend on several factors, including the desired use of the composition, the amount of any catalase activity present in the substance, the amount of substrate present in the substance, the desired level of hydrogen peroxide release, and the desired length of time for hydrogen peroxide release. A suitable amount of enzyme can readily be determined by a person of ordinary skill in the art, if necessary using a well diffusion assay, to determine the extent of hydrogen peroxide release for different amounts of enzyme. Suitable amounts of enzyme (such as glucose oxidase) may be from 0.0001% to 0.5% w/w of the composition. The amount of enzyme used may be selected so as to produce a composition for generating antimicrobial activity that is equivalent to a selected phenol standard (for example a 10%, 20%, or 30% phenol standard).

Compositions for use according to the invention, particularly compositions in which the substance is honey (for example, unpasteurised honey), and the enzyme is glucose oxidase that is able to convert D-glucose in the honey to release hydrogen peroxide, may comprise at least 1 unit, and preferably up to 1500 units, of glucose oxidase per gram of the composition. The glucose oxidase is additional (i.e. added as a result of human intervention) to any glucose oxidase activity that may naturally be present in the substance.

A "unit" is defined herein as the amount of enzyme causing the oxidation of 1 micromole of glucose per minute at 25 degrees centigrade at pH 7.0.

The Applicant has found that the antimicrobial potency of compositions for use according to the invention may be increased simply by increasing the amount of glucose oxidase activity present in the composition.

In some embodiments of the invention, a composition for use according to the invention comprises more than 15 units, for example at least 30 units, at least 50 units, or at least 100 units, and suitably less than 685 units, for example 100-500 units, of glucose oxidase per gram of the composition. Such compositions have been found to have superior antimicrobial properties than compositions with up to 15 units of glucose oxidase per gram of the composition. In particular, such compositions have increased potency against a wide range of microorganisms, including MSSA, MRSA, Group A and B *Streptococci, Enterococcus, E.coli, E.coli* ESBL, *Serr.liquefaciens* Amp C, *Kleb.pneumoniae, Pseudomonas aeruginosa, Acinetobacter baumannii,* and *Candida albicans.*

In other embodiments of the invention, a composition for use according to the invention comprises at least 500 units, for example 500-1000 units, or 685-1000 units, of glucose oxidase per gram of the composition. Such compositions have been found to have even more superior antimicrobial properties. In particular such compositions have further increased potency against a wide range of microorganisms, including *Staphylococcus aureus,* MSSA, MRSA, Group A and B *Streptococci, Enterococcus, E.coli, E.coli* ESBL, *Serr.liquefaciens* Amp C, *Kleb.pneumoniae, Pseudomonas aeruginosa, Acinetobacter baumannii,* and *Candida albicans.*

The pasteurisation process inactivates any enzyme activity present in the honey, and so there is no variability in catalase and substrate conversion activity between pasteurised honeys from different sources, or between different harvests of honey from the same source. The amount of substrate conversion activity can be controlled by addition of a purified glucose oxidase preparation with a defined amount and activity of the enzyme. Thus, the inherent variability in antimicrobial properties between different types and harvests of honey is considerably reduced, and the antimicrobial properties of honeys with low antimicrobial potency are improved.

For wound healing applications, compositions for use according to the invention may be administered at an appropriate frequency determined by the healthcare provider. Suitably compositions for use according to the invention may be administered at least every several days, for example every week, but preferably every day or every other day.

The amount of a composition for use according to the invention administered will depend on many factors, such as the strength of the antimicrobial properties of the composition, and other wound healing properties of the composition, on the size of the wound, and on the age and condition of the subject to be treated. However, for many applications it is expected that each administration comprises 2-100g, or 5-100g of a composition for use according to the invention, preferably 10-50g.

According to preferred embodiments of the invention, a composition for use according to the invention is sterile. Sterile compositions are preferably used for medical applications such as wound healing.

Compositions for use according to the invention may be sterilised by any suitable means. The Applicant has found that compositions for use according to the invention retain glucose oxidase activity (and, therefore, the ability to release hydrogen peroxide on dilution) following sterilisation by exposure to gamma irradiation. A suitable level of gamma irradiation is 10-70 kGy, preferably 25-70 kGy, more preferably 35-70 kGy.

Since ozone has not been authorised by the US FDA for sterilisation of honey-based products for use in wound healing, compositions for use according to the invention preferably have not been sterilized by ozonation, and do not include ozone, or any components that have been subjected to sterilisation by ozonation. In particular, compositions for use according to the invention should not comprise ozonized honey or ozonated oil.

Preferred compositions for medical use according to the invention are sterile, single use compositions.

Sterilised compositions for use according to the invention that are stored away from exposure to light are expected to retain stability for at least six months. For example, such compositions may be packaged in high-density polyethylene/low-density polyethylene (HDPE/LDPE) tubes or in polyester-aluminium-polyethylene (PET/AI/PE) sachets.

A composition for use according to the invention is preferably a medical grade or medical device grade composition. Preferably the unrefined natural substance is a honey, suitably a medical grade or medical device grade honey.

Preferably a composition for use according to the invention comprises a creamed honey, more preferably a creamed unpasteurised honey. Such compositions can readily be administered topically because the presence or number of large crystals has been minimised by the creaming process.

For compositions for use according to the invention that comprise honey, it will be appreciated that there may be no need to use pasteurised honey in the composition if the composition is sterilised. It may instead be preferable to use unpasteurised honey (preferably creamed honey) or other unrefined natural substance. In some embodiments, compositions for use according to the invention comprise unpasteurised honey, and added purified glucose oxidase.

Thus, a storage-stable composition for generating antimicrobial activity for use according to the invention may comprise unpasteurized honey, and added purified glucose oxidase that, in the presence of sufficient free water, is able to convert D-glucose in the honey to release hydrogen peroxide, wherein the composition does not include sufficient free water to allow the glucose oxidase to convert the D-glucose.

Such compositions may comprise at least 1 unit, and for example up to 1500 units, of glucose oxidase per gram of the composition. Suitably such compositions comprise more than 15 units of glucose oxidase per gram of the composition, for example at least 100 units, or 100-500 units, of glucose oxidase per gram of the composition, or at least 500 units, or 500-1000 units, of glucose oxidase per gram of the composition.

The honey of such compositions may comprise a creamed unpasteurized honey.

A composition for use according to the invention may be a pharmaceutical composition comprising a pharmaceutically acceptable carrier, excipient, or diluent.

A composition for use according to the invention may be provided with a dressing. Suitable dressings include gauzes, bandages, tissues, films, gels, foams, hydrocolloids, alginates, hydrogels, or polysaccharide pastes, granules or beads. The composition may be present together with a wound-dressing matrix, such as a collagen or collagen-glycosaminoglycan matrix.

The composition may be in the form of a solid or semi-solid preparation. Examples of solid or semi-solid preparations include capsules, pellets, gel caps, powders, hydrogels, pills, pillules, or globules. Alternatively, the composition may be in the form of a liquid preparation. Examples of liquid preparations include a syrup, paste, spray, drop, ointment, cream, lotion, oil, liniment, or gels. A typical gel includes an alcoholic gel such as an isopropanol, ethanol, or propanol gel, or a hydrogel.

A composition for use according to the invention may be in a form suitable for administration to a human or animal subject. Suitable forms include forms adapted for topical or oral administration. Forms suitable for topical administration include a topical ointment, cream, lotion, oil, liniment, liquid, gel, or a dissolvable strip. Forms suitable for oral administration include a capsule, pellet, gel cap, pill, pillule, globule, lozenge, dental floss, toothpaste, mouthwash, dissolvable film strips. If a storage-stable composition is used, this may be diluted by liquid present at the site of administration (for example, by saliva for oral administration, or by exudate from a wound), leading to release of hydrogen peroxide at the administration site.

A composition for use according to the invention may be present with at least one suitable antimicrobial or immunostimulatory component, excipient or adjuvant, or any other suitable component where it is desired to provide ability to generate antimicrobial activity. Preferably, however, the compositions do not include any antibiotic.

An example of a suitable composition for use according to the invention is "Surgihoney". Surgihoney is unpasteurised honey with added purified glucose oxidase. Three different preparations of Surgihoney have been made with different antimicrobial potencies:
SH1 Surgihoney: unpasteurised honey with 0.1% (w/w) added glucose oxidase. The enzyme used was food grade glucose oxidase, from *Aspergillus niger,* from BIO-CAT, INC, activity 15,000 Units/g. Sealed sachets of the SH1 Surgihoney were gamma irradiated at a target dose of 11/6-14.2 kGy.

SH2 Surgihoney: unpasteurised honey with 0.1% (w/w) added glucose oxidase. The enzyme used was glucose oxidase (GO3B2), from *Aspergillus niger,* from BBI Enzymes Limited, activity 274 Units/mg. Unit Definition: the amount of enzyme causing the oxidation of 1 micromole of glucose per minute at 25 degrees centigrade at pH 7.0. Contaminants: alpha amylase no greater than 0.05%, Saccharase no greater than 0.05%, maltase no greater than 0.05% and GO/Cat no less than 2000.

SH3 Surgihoney: unpasteurised honey with 0.25% (w/w) added glucose oxidase. The enzyme used was glucose oxidase (GO3B2) from BBI Enzymes Limited, activity 274 Units/mg.

Thus, SH1 Surgihoney contains 15 units of glucose oxidase per gram of the composition, SH2 Surgihoney contains 274 units of glucose oxidase per gram of the composition, and SH3 Surgihoney contains 685 units of glucose oxidase per gram of the composition.

Compositions of the invention may be used to treat sinus infections, such as CRS, for example as part of a nasal douche.

According to the disclosure there is provided a composition for generating anti-microbial activity that comprises: an enzyme that is able to convert a substrate to release hydrogen peroxide; a substance that includes a substrate for the enzyme; and a salt.

Alternatively, the salt may be provided in a kit separately from the rest of the composition. Accordingly, there is also provided according to the disclosure a kit comprising: a composition for generating anti-microbial activity that comprises an enzyme that is able to convert a substrate to release hydrogen peroxide, and a substance that includes a substrate for the enzyme; and, separately, a salt. The kit may further include instructions, for example, for mixing of the components of the kit, and their use to treat a microbial infection.

The composition, or the composition of the kit, may be provided in a form that does not include sufficient free water to allow the enzyme to convert the substrate. For example, the composition may comprise less than 20% water, for example 10-19% water. Such compositions (referred to below as compositions in 'dry' form), when mixed with water, are suitable for use as a nasal douche.

The salt may be provided in dry form, or in aqueous solution. The salt may comprise sodium chloride.

The composition in dry form may comprise a ratio of the composition for generating anti-microbial activity to the salt, for example, of from 1:2 to 5:1, or from 2:3 to 3:2.

The composition may comprise, for example, 1-99%, 1-80%, 1-70%, 1-60%, 1-50%, 1-40%, 1-30%, 1-20%, or 1-10%, by weight, of the composition for generating anti-microbial activity.

The composition may comprise, for example, 1-99%, 1-80%, 1-70%, 1-60%, 1-50%, 1-40%, 1-30%, 1-20%, or 1-10%, by weight, of the salt.

The composition, kit, or mixture may further comprise a buffering agent, such as sodium bicarbonate, to adjust the pH of the solution, for example to close to physiological pH (suitably pH 7.3-7.5).

The composition may comprise, for example, 1-99%, 1-80%, 1-70%, 1-60%, 1-50%, 1-40%, 1-30%, 1-20%, or 1-10%, by weight, of the buffering agent.

The composition may be provided as an aqueous mixture. The aqueous mixture may be an isotonic or hypertonic mixture. The aqueous mixture may comprise, for example, 0.1-20% w/v salt, suitably 0.25-10%, 0.25-10%, 0.25-5%, 0.25-3%, 0.5-10%, 0.5-5%, or 0.5-3%, for example 0.9% w/v salt. The aqueous mixture may comprise, for example, 1-300%, 1-250%, 1-200%, 1-150%, 1-100%, 1-50%, 1-40%, 1-30%, 1-20%, or 1-10% w/v of the composition for generating anti-microbial activity. The aqueous mixture may comprise, for example, 10-300%, 10-250%, 10-200%, 10-150%, 10-100%, 10-50%, 10-40%, 10-30%, or 10-20% w/v of the composition for generating anti-microbial activity. The aqueous mixture may comprise, for example, 50-300%, 50-250%, 50-200%, 50-150%, or 50-100% w/v of the composition for generating anti-microbial activity. The aqueous mixture may comprise, for example, 0.1-20%, 0.1-10%, 0.1-5%, 0.1-1% w/v of sodium bicarbonate.

Compositions, kits, or mixtures, in particular those comprising salt, may be used as a nasal douche, for example to prevent or treat nasal microbial infection, sinusitis, rhinitis, CRS, nasal allergy, cold or flu symptoms, congestion, or dryness. The compositions, kits, or mixtures may be used to prevent or treat a microbial infection, for example a microbial infection that comprises a biofilm, or a microbe that is capable of forming a biofilm. The microbial infection that comprises a biofilm may be a nasal microbial infection, or the microbe that is capable of forming a biofilm may be part of a nasal microbial infection.

To use a composition or mixture of the invention as a nasal douche, it may be poured into one nostril and allowed to run out through the other, while the mouth is kept open to breathe, using gravity as an aid. Alternatively, some form of positive pressure may be applied to facilitate rinsing. For example, bottles made of flexible plastic, optionally with special tips to fit the nostril, can be squeezed to exert positive pressure of the mixture flowing through the sinuses while the mouth is kept open at all times in order to breathe and prevent snorting the liquid down the throat. Irrigation machines that utilize electric motor-driven pumps are also available. Some nasal irrigation systems that apply pressure have an anti-backwash valve to prevent used saltwater solution from flowing back into the nasal cavity.

Compositions or mixtures of the invention may be provided in a neti pot, a container used to administer nasal douche. Neti pots are typically made of metal, glass, ceramic or plastic. They rely on gravity, along with head positioning and repeated practice in order to rinse the outer sinus cavities. Typically they have a spout attached near the bottom, sometimes with a handle on the opposite side.

There is also provided according to the disclosure, a method of preventing or treating a sinus infection, such as CRS, which comprises administering an effective amount of a composition or mixture of the disclosure to a subject in need of such prevention or treatment.

Compositions or mixtures of the invention may also be administered to the lungs to prevent or treat a microbial infection, for example a microbial infection that comprises a biofilm, or a microbe that is capable of forming a biofilm, in lung tissue. For example, compositions or mixtures of the invention may be administered to the lungs to prevent or treat tuberculosis, or to prevent or treat a microbial infection associated with cystic fibrosis (CF). *Mycobacterium tuberculosis* is the causative agent of tuberculosis. Compositions or mixtures of the invention may be used to prevent or treat respiratory infection, for example respiratory infection in a subject suffering from respiratory disease, such as COPD, cystic fibrosis, bronchiectasis, or asthma, or an HIV/AIDS-associated respiratory infection, or respiratory infection associated with terminal disease.

The genetic basis for CF is a well-characterized, severe monogenic recessive disorder, found predominantly in Caucasian populations of European ancestry, that arises from mutations in the cystic fibrosis transmembrane conductance regulator (CFTR) gene. While the gene defect results in a myriad of medical problems for the patient, a significant clinical feature of the disease is chronic pulmonary infection with *Pseudomonas aeruginosa.* One aspect of the pathogenesis of chronic lung infection in CF is the ability of *P*. *aeruginosa* to grow as a biofilm. Ultimately, 80 to 95% of patients with CF succumb to respiratory failure brought on by chronic bacterial infection and concomitant airway inflammation. Other microbial infections associated with cystic fibrosis include *Staphylococcus aureus* and *Haemophilus influenzae* (Lyczak, et al., Clinical Microbiology Reviews, Apr. 2002, p. 194-222).

Compositions of the invention may be used to prevent or treat a microbial infection in a patient with cystic fibrosis, especially a microbial infection in a patient with cystic fibrosis that comprises a biofilm, or a microbe that is capable of forming a biofilm. The infection may be a pulmonary infection. For example, a composition of the invention may be used to prevent or treat a pulmonary *Pseudomonas aeruginosa* infection in a subject with cystic fibrosis.

There is also provided according to the disclosure a method of preventing or treating a microbial lung infection, which comprises administering an effective amount of a composition of the disclosure to a subject in need of such prevention or treatment.

Bronchiectasis is a permanent dilatation and thickening of the airways characterised by chronic cough, excessive sputum production, bacterial colonisation, and recurrent acute infections. It may be widespread throughout the lungs (diffuse) or more localised (focal). It is caused by chronic inflammation of the airways, and is associated with, or caused by, a large number of diseases. It may develop after lung infections, particularly in childhood and in association with underlying problems, such as immunodeficiency and cystic fibrosis.

Bronchiectasis can be classified into the following forms morphologically (all three forms may be present in the same patient):
Cylindrical bronchiectasis: bronchi are enlarged and cylindrical;
Varicose bronchiectasis: bronchi are irregular with areas of dilatation and constriction;
Saccular or cystic: dilated bronchi form clusters of cysts. This is the most severe form of bronchiectasis and is often found in patients with cystic fibrosis.

The affected airways are inflamed and easily collapse. There is an impairment of airflow and drainage of secretions, leading to the accumulation of a large amount of mucus in the lungs. The mucus collects bacteria, predisposing to frequent and often severe lower respiratory tract infections.

Respiratory tract infections associated with bronchiectasis include infections caused by the following bacteria: *Staphylococcus aureus, Haemophilus influenzae, Pseudomonas aeruginosa, Streptococcus pneumoniae,* non-tuberculous mycobacteria.

The severity of bronchiectasis used to be classified according to the volume of sputum produced but this has now largely been superseded by using the radiological appearance on a computerised tomography (CT) scan.

Chronic obstructive pulmonary disease (COPD) is an umbrella term for people with chronic bronchitis, emphysema, or both. With COPD the airflow to the lungs is restricted. COPD is usually caused by smoking. Symptoms include cough and breathlessness. Lower respiratory tract infections, both acute and chronic, occur with increased frequency. As these infections contribute considerably to the clinical course of the patient with COPD, they constitute a significant comorbidity in COPD. *Enterobacteriaceae, P. aeruginosa* and *Staphylococcus aureus* have been implicated in COPD exacerbations. Various microbial pathogens have been implicated in chronic infection in COPD. These include typical bacteria such as non-typeable H. *influenzae* and *P*. *aeruginosa,* atypical bacterium such as *C. pneumoniae,* viruses such as adenovirus and possibly respiratory syncytial virus, and a fungus, *Pneumocystis jiroveci.*

Some of the most common opportunistic infectious lung diseases seen in HIV-positive or AIDS patients are *pneumocystis carinii pneumonia,* tuberculosis (caused by *Mycobacterium tuberculosis*), mycobacterium avium complex (*Mycobacterium avium-M. intracellulare* complex (MAIC)), fungal infections (such as candidiasis or coccidioidomycosis) and viral and bacterial pneumonia (such as bacterial pneumonia caused by *Haemophilus influenzae*)*.*

Compositions of the invention may be used for the prevention or treatment of infections, particularly respiratory infections, caused by any of the following microbes: *Pseudomonas aeruginosa; Staphylococcus aureus; Haemophilus influenza; Streptococcus pneumoniae,* non-tuberculous mycobacteria; *Enterobacteriaceae; C. pneumonia;* adenovirus; respiratory syncytial virus; *Pneumocystis jiroveci; pneumocystis carinii pneumonia; Mycobacterium tuberculosis; Mycobacterium avium-M. intracellulare* complex (MAIC); *candida; Coccidioides immitis; Mycobacterium abscessus.*

Compositions of the invention may be used to treat lower genital tract infections. For example, compositions of the invention may be applied topically to treat such infections. Examples of such infections include bacterial vaginosis and general bacterial vaginal discharge. Compositions of the invention may be applied to an insertion device, such as a tampon.

Compositions of the invention may be used to treat infections comprising Carbapenem-resistant enterobacteriaceae (CRE) or Carbapenemase-producing Enterobacteriaceae (CPE) bacteria,

A composition of the invention may be administered or applied by spraying, by injection, by inhalation, or applying the composition to a patient's nasal cavity or paranasal sinus. The composition may be administered or applied externally or internally, to a patient.

A composition of the invention may be provided in an inhaler, for example, a metered-dose inhaler, a dry powder inhaler, a nebulizer, for delivery of the composition into the lungs, or in a nasal inhaler.

According to the disclosure there is also provided a composition for generating anti-microbial activity, wherein the composition comprises: an enzyme that is able to convert a substrate to release hydrogen peroxide; an unrefined natural substance that includes a substrate for the enzyme, wherein the enzyme is additional to any enzyme activity able to convert the substrate to release hydrogen peroxide that may be present in the unrefined natural substance; and a polymer. Optionally, the composition may further comprise a salt. If the composition further comprises a salt, optionally the composition may further comprise a buffering agent, such as sodium bicarbonate.

A composition for generating anti-microbial activity may comprise: an enzyme that is able to convert a substrate to release hydrogen peroxide; a substance that comprises a purified substrate for the enzyme; and a polymer. The enzyme is additional (i.e. added as a result of human intervention) to any enzyme activity able to convert the substrate to release hydrogen peroxide that may be present in the substance. Optionally, the composition may further comprise a salt. If the composition further comprises a salt, optionally the composition may further comprise a buffering agent, such as sodium bicarbonate.

In particular embodiments, the purified substrate is or comprises a purified sugar substance. As described above, the purified sugar substance may be obtained from a natural source (for example a processed, extracted, or refined natural sugar substance), or be synthetically produced. The purified sugar substance may be at least 10%, 20%, 30%, 40%, 50%, 60%, 70%, 80%, 90%, 95%, or 99% pure. The purified sugar substance may be a medical grade, medical device grade, or pharmaceutical grade sugar substance,. The purified sugar substance may include one or more purified sugar substances, for example purified D-glucose, hexose, or D-galactose. For example the purified sugar substance may be medical grade, medical device grade, or pharmaceutical grade D-glucose, hexose, or D-galactose. In particular embodiments, the enzyme and the substrate are purified, for example purified glucose oxidase and purified D-glucose, suitably medical grade, medical device grade, or pharmaceutical grade glucose oxidase and D-glucose.

The composition may be a sprayable or atomisable composition. For example, the composition may have rheological properties that permit spraying or atomisation.

The composition may be an injectable composition. For example, the composition may have rheological properties that permit application through a syringe.

Without the addition of the polymer to the composition, an unrefined natural substance, such as honey, may not have the requisite rheological properties to permit effective spraying or injection. For example, the unrefined natural substance may be too viscous.

The polymer in the composition may be any medically acceptable polymer, such as any Food and Drug Administration-approved (FDA-approved) polymer.

In some embodiments, the polymer may be a synthetic polymer. In some embodiments, the polymer is a natural polymer.

Optionally, the polymer is water soluble. The polymer may be soluble in an organic, or non-aqueous, solvent. The polymer may be soluble in a mixture of an aqueous and non-aqueous solvent. The polymer may be biodegradable or bioerodable. The polymer may be a co-polymer. In some embodiments, the polymer is selected from polyethylene oxide (or polyethylene glycol), polyvinyl alcohol and polyvinylpyrrolidone.

Other polymers may include poly(lactic-co-glycolic acid), polyglycolic acid, polylactic acid, polycaprolactone or polymeric surfactants. Another suitable polymer may be phosphinocarboxylic acid (PCA).

Further polymers may include polysaccharides such as cellulose (which includes derivatives such as hydroxypropyl methyl cellulose and hydroxypropyl cellulose), alginate, gelatin or cyclodextrins. Suitable polymers may also include chitosan or hyaluronic acid.

The composition may comprise up to 50%, 25%, 10% or 5% by weight of the polymer. For example, the composition may comprise from 0.5 to 3% by weight of the polymer. Optionally, the polymer may be from 0.5 to 50% by weight of the composition. The composition may comprise up to 30%, 20% or 10% by weight of the unrefined natural substance.

The composition may be formed by mixing a polymer solution with the unrefined natural substance, or the substance that comprises a purified substrate. For example, the polymer solution may comprise up to 50%, 25%, 10% or 5%, by weight, of the polymer. The composition may be formed by mixing the polymer solution with the unrefined natural substance, or the substance that comprises a purified substrate, in a weight ratio (polymer solution:unrefined natural substance, or polymer:substance that comprises a purified substrate) of 90%:10% to 60%:40%. For example, the composition may be formed from a mixture of 80% by weight polymer solution and 20% by weight unrefined natural substance, or substance that comprises a purified substrate.

Preferably, the composition is a storage-stable composition. In some embodiments, the composition does not include sufficient free water to allow the enzyme to convert the substrate. In some embodiments, the solution or composition does comprise sufficient free water to allow the enzyme to convert the substrate.

Storage-stable compositions of the invention may include an antimicrobial agent. For example, hydrogen peroxide may be present if the storage-stable composition is formed by contacting the enzyme with the substance in aqueous solution under conditions for conversion of the substrate by the enzyme, and then drying the composition to reduce its water content to a level where there is insufficient free water to allow the enzyme to convert the substrate. Preferably, however, the storage-stable composition does not include any detectable hydrogen peroxide. It may be said that the composition contains substantially no hydrogen peroxide. Such a composition may be formed, for example, by contacting the enzyme with the substrate in the absence of sufficient free water to allow the enzyme to convert the substrate. Examples of other antimicrobial agents that may be present in a storage-stable composition of the invention include: an antibiotic, an antiviral agent, or an anti-fungal agent.

The composition may comprise non-aqueous solvents.

The viscosity of the composition may be adjusted according to the desired application of the composition. For example, viscosity may be adjusted by varying the type and/or amount of polymer in the composition or by adjusting the solvent content of the composition.

The composition may be a gel. Preferably, the composition is a liquid.

According to the disclosure, there is also provided a device for delivering a composition to a patient, the device comprising a composition, the composition comprising: an enzyme that is able to convert a substrate to release hydrogen peroxide; an unrefined natural substance that includes a substrate for the enzyme, wherein the enzyme is additional to any enzyme activity able to convert the substrate to release hydrogen peroxide that may be present in the unrefined natural substance; and a polymer.

There is also provided according to the disclosure a device for delivering a composition to a patient, the device comprising a composition, the composition comprising: an enzyme that is able to convert a substrate to release hydrogen peroxide; a substance that comprises a purified substrate for the enzyme, wherein the enzyme is additional to any enzyme activity able to convert the substrate to release hydrogen peroxide that may be present in the substance; and a polymer.

The device may be a spraying or atomising device, such as a pump-action spray or an aerosol spray. The device may be an inhaler, for example, a metered-dose inhaler, a dry powder inhaler, a nebulizer, for delivery of the composition into the lungs, or a nasal inhaler.

A nebuliser is a device that converts liquid into aerosol droplets suitable for inhalation. Nebulisers use oxygen, compressed air or ultrasonic power to break up medication solutions and deliver a therapeutic dose of aerosol particles directly to the lungs. A wide variety of nebulisers is available. Nebulisers can be driven by compressed gas (jet nebuliser) or by an ultrasonically vibrating crystal (ultrasonic nebuliser).

In order to produce small enough particles from solution in 5-10 minutes, gas flow rates of at least 6 L/minute are usually necessary. Ultrasonic nebulisers use a rapidly vibrating piezoelectric crystal to produce aerosol particles. Ultrasonic nebuliser machines are often smaller and quieter.

Many nebulisers deliver only 10% of the prescribed drug dose to the lungs. Much of the drug is caught on the internal apparatus or wasted during exhalation. The efficiency of drug delivery depends on the type and volume of nebuliser chamber and the flow rate at which it is driven. Some chambers have reservoir and valve systems to increase efficiency of particle delivery during inspiration and reduce environmental losses during expiration. Breath-assisted open vent systems improve drug delivery but are dependent on the patient having an adequate expiratory flow. Face masks or mouthpieces may be used for administration of aerosol particles.

Nebulisers are used for the treatment of many respiratory diseases. Indications for nebuliser use include the management of exacerbations and long-term treatment of chronic obstructive pulmonary disease (COPD), management of cystic fibrosis, bronchiectasis, asthma, HIV/AIDS and symptomatic relief in palliative care.

Nebulised compositions may be used to prevent or treat a microbial infection, for example a microbial infection that comprises a biofilm, or a microbe that is capable of forming a biofilm, in a subject suffering from respiratory disease, such as COPD, cystic fibrosis, bronchiectasis, or asthma, or an HIV/AIDS-associated respiratory infection, or respiratory infection associated with terminal disease.

The device may be for external use, such as for applying the composition to a patient's skin.

The device may be for internal application to a patient. For example, the device may be an inhaler or nebuliser for administering the composition to the patient's respiratory tract. The device may be a douche. The device may be a device for injecting the composition into the patient, such as a syringe.

According to the disclosure there is provided a kit, the kit comprising: i) a composition, the composition comprising an enzyme that is able to convert a substrate to release hydrogen peroxide, an unrefined natural substance that includes a substrate for the enzyme, wherein the enzyme is additional to any enzyme activity able to convert the substrate to release hydrogen peroxide that may be present in the unrefined natural substance, and a polymer; and ii) a device for delivering a composition to a patient. The composition may be separate from the device.

According to the disclosure there is also provided a kit, the kit comprising: i) a composition, the composition comprising an enzyme that is able to convert a substrate to release hydrogen peroxide, a substance that comprises a purified substrate for the enzyme, wherein the enzyme is additional to any enzyme activity able to convert the substrate to release hydrogen peroxide that may be present in the substance, and a polymer; and ii) a device for delivering a composition to a patient. The composition may be separate from the device.

The compositions may be for prophylactic applications, or for treatment of a condition associated with microbial infection.

For example, there may be provided a prophylactic spray for pre-operation skin sterilisation. Such a spray may comprise a low-viscosity composition. When applied, the composition may be non-sticky. The spray may allow delivery of a reactive oxygen laminar to the skin. The laminar may continue to deliver reactive oxygen over an extended period of time which could sterilise skin prior to surgical incision and provide prophylactic protection during and after the surgical procedure.

There may be provided an atomising spray for inhalation. An atomising spray for inhalation may be used to treat infections in a patient's airways. An atomising spray may require a composition with low viscosity.

A spray could be used to apply to burn or wound tissue prior to applying a dressing. This may require a more viscous composition.

A spray could be applied internally to a patient, post-surgery, to prevent organ infection and septicaemia. This may require a more viscous composition.

The composition may be diluted with water prior to being used with a device, such as a douche.

Preferably, the composition is substantially homogenous.

According to the disclosure, there is provided a method of applying or administering a composition to a patient, the composition comprising: an enzyme that is able to convert a substrate to release hydrogen peroxide; an unrefined natural substance that includes a substrate for the enzyme, wherein the enzyme is additional to any enzyme activity able to convert the substrate to release hydrogen peroxide that may be present in the unrefined natural substance; and a polymer, wherein the method comprises spraying the composition, injecting the composition, inhaling the composition or applying the composition to a patient's nasal cavity or paranasal sinus.

According to the disclosure, there is also provided a method of applying or administering a composition to a patient, the composition comprising: an enzyme that is able to convert a substrate to release hydrogen peroxide; a substance that comprises a purified substrate for the enzyme, wherein the enzyme is additional to any enzyme activity able to convert the substrate to release hydrogen peroxide that may be present in the substance; and a polymer, wherein the method comprises spraying the composition, injecting the composition, inhaling the composition or applying the composition to a patient's nasal cavity or paranasal sinus.

According to the disclosure, there is provided a composition for use as a medicament, the composition comprising: an enzyme that is able to convert a substrate to release hydrogen peroxide; an unrefined natural substance that includes a substrate for the enzyme, wherein the enzyme is additional to any enzyme activity able to convert the substrate to release hydrogen peroxide that may be present in the unrefined natural substance; and a polymer.

There is also provided according to the disclosure, a composition for use as a medicament, the composition comprising: an enzyme that is able to convert a substrate to release hydrogen peroxide; a substance that includes a purified substrate for the enzyme, wherein the enzyme is additional to any enzyme activity able to convert the substrate to release hydrogen peroxide that may be present in the substance; and a polymer. The composition may be administered or applied by spraying, by injection, by inhalation, or applying the composition to a patient's nasal cavity or paranasal sinus. The composition may be administered or applied externally or internally, to a patient.

The composition may be administered or applied by spraying, by injection, by inhalation, or applying the composition to a patient's nasal cavity or paranasal sinus. The composition may be administered or applied externally or internally, to a patient.

Compositions of the invention may be sterile. The compositions may be sterilised by any suitable method, for example by exposure to gamma irradiation as described above.

In some embodiments, the composition (in particular, a composition of the invention that comprises an unrefined natural substance and a polymer) is not, or does not comprise, the following:

### Aqueous Spray

25 ml plastic bottle containing:

| | |
|---|---|
| Active honey | 10 g |
| Triton CF | 0.1g |
| Maltodextrin, or corn Starch | 1g |

wherein "Active honey" is honey with added glucose oxidase.

In some embodiments, the composition (in particular, a composition of the invention that comprises an unrefined natural substance and a polymer) is not, or does not comprise, the following:

### Non-Aqueous spray

| | |
|---|---|
| Active honey | 70% |
| Proplyene Glycol | 30% |

In some embodiments, the composition (in particular, a composition of the invention that comprises an unrefined natural substance and a polymer) is not, or does not comprise, the following:

### Powder

Active honey
Maltodextrin
Ibuprofen
Microcrystalline Cellulose (CMC)
Polyvinyl Pyrrolidone (PVP)

In some embodiments, the composition (in particular, a composition of the invention that comprises an unrefined natural substance and a polymer) is not, or does not comprise, the following:

### Alcoholic Gel

| | |
|---|---|
| Carbopol 940 | 0.3 % |
| Triethanolamine | 0.4 % (needed for pH and stability control) |
| Active honey (5+) | 65% |
| Ethanol | 25.0 % |
| water qs | |

In preferred embodiments, the composition (in particular, a composition of the invention that comprises an unrefined natural substance and a polymer) is not, or does not comprise the following:

### Fungal Nail Treatment

Plaster with Active Honey (5+) in a foam well
Hydroxypropylcellulose
Glycerol
Isopropyl Alcohol
Citric Acid
Monohydrate

In some embodiments, the composition (in particular, a composition of the invention that comprises an unrefined natural substance and a polymer) is not, or does not comprise the following:

### Cream

| | |
|---|---|
| Honey | 15% |
| Carbomer | 2.63% |
| Dimethicone | 0.13% |
| Disodium Lauryl Sulphosuccinate | 0.05% |
| Disodium Edetate | 0.13% |
| Glycerol | 5.26% |
| Silica Colloidal Hydrated | 0.33% |
| Poloxamer | 0.26% |
| Sodium Hydroxide | 0.41% |
| Purified Water | 85.03% |

In some embodiments, the composition (in particular, a composition of the invention that comprises an unrefined natural substance and a polymer) does not comprise one or more of the following: maltodextrin, microcrystalline cellulose, Polyvinyl Pyrrolidone, carbomer, polaxomer, hydroxypropyl cellulose, carbopol 940.

There is also provided according to the disclosure a composition for generating anti-microbial activity that comprises: an enzyme that is able to convert a substrate to release hydrogen peroxide; and a substance that includes a purified substrate for the enzyme; wherein the composition is a storage-stable composition that does not include sufficient free water to allow the enzyme to convert the substrate, and wherein the composition provides for sustained release of hydrogen peroxide at a level of less than 2 mmol/litre for a period of at least 24 hours following dilution of the composition.

There is further provided according to the disclosure an aqueous mixture, which comprises: an enzyme that is able to convert a substrate to release hydrogen peroxide; and a substance that includes a purified substrate for the enzyme; wherein the mixture provides for sustained release of hydrogen peroxide at a level of less than 2 mmol/litre for a period of at least 24 hours.

The composition or mixture may comprise sufficient enzyme and substrate to provide for sustained release of at least 0.1 mmol/litre hydrogen peroxide for a period of at least 24 hours. The composition or mixture may further comprise a salt.

There is also provided according to the disclosure a composition or mixture of the disclosure for use as a medicament.

The disclosure also provides a composition or mixture of the disclosure for use in the prevention or treatment of a microbial infection.

There is further provided according to the disclosure use of a composition or mixture of the disclosure in the manufacture of a medicament for the prevention or treatment of a microbial infection.

The disclosure further provides a method of preventing or treating a microbial infection, wherein the method comprises administering an effective amount of a composition or mixture of the disclosure to a site of the infection.

The microbial infection may include a biofilm, or a microbe that is capable of forming a biofilm.

It is estimated that about one out of every 100 people in the U.S. are colonised with MRSA (i.e. have the organisms in or on their body but not causing infection), and these individuals may transmit MRSA bacteria to others. Another term for people colonised with MRSA is "carrier" which means the person carries the organism in or on the body and may transfer the organism to another person who subsequently may become infected. A common place for carriers to harbour MRSA organisms is the nose.

Mupirocin (Bactroban) is a topical antibacterial treatment that is effective against Gram-positive bacteria, including MRSA, and is currently used as part of the decolonisation regime. However, recent evidence indicates increasing resistance of MRSA to Mupriocin (Jones et al., Clinical Infectious Diseases. 2007;45(5):541-547; Patel et al., Clinical Infectious Diseases. 2009;49(6):935-941).

Compositions for use according to the invention, as defined above, may be used to prevent or treat an MRSA colonisation or infection, for example, as a topical anti-MRSA treatment to decolonise MRSA in MRSA-positive subjects, such as MRSA carriers (particularly nasal carriers), or surgical patients with MRSA-infected wounds.

Thus, according to the invention there is provided a composition for use according to the invention, for use in the prevention or treatment of an MRSA colonisation or infection.

There is also provided according to the disclosure use of a composition or mixture of the disclosure, or a composition for use according to the disclosure, in the manufacture of a medicament for the prevention or treatment of an MRSA colonisation or infection.

There is further provided according to the disclosure, a method of preventing or treating an MRSA colonisation or infection, which comprises administering an effective amount of a composition or mixture of the disclosure, or a composition for use according to the disclosure, to a subject in need of such prevention or treatment.

Compositions, or compositions for use according to the disclosure may comprise an enzyme that is able to convert a substrate to release hydrogen peroxide, and a substance that includes a substrate for the enzyme. In some embodiments, the enzyme may be a purified enzyme, as defined above. Alternatively, or additionally the substance may be a processed, extracted, or refined substance, or the substrate may be an unrefined substance (such as an unrefined, natural substrate), or may comprise a purified substrate, as defined above.

The enzyme of a composition of the invention is additional (i.e. added as a result of human intervention) to any enzyme activity able to convert the substrate to release hydrogen peroxide (referred to herein as "substrate conversion activity") that may be present in the substance. The composition may be a storage-stable composition which does not include sufficient free water to allow the enzyme to convert the substrate.

A composition of the invention may be contained within a water-soluble container, such as a sachet or pouch. So, there may be provided a water-soluble container enclosing, or containing, a composition of the invention. Advantageously, this may allow a precise amount of the composition to be delivered for a particular therapeutic application by adding a measured amount of solvent, such as water or saline. For example, a composition of the invention contained in a water-soluble sachet may be used to form a nasal douche solution, or a solution that is to be nebulised or atomised. Consequently, contacting the soluble sachet containing the composition, with an aqueous solvent, may result in formation of an aqueous mixture of the invention. The aqueous mixture may contain sufficient free water to allow the enzyme to convert the substrate and produce hydrogen peroxide.

The water-soluble sachet is preferably manufactured from a medical grade material. The sachet may be dissolvable in water at 38°C. Preferably, the water-soluble sachet is non-toxic, anti-static, resistant to degradation by ultraviolet light and resistant to degradation by gases, oils and greases. In one example, the soluble sachet may be manufactured from a polymer or plastics material, such as polyvinyl alcohol.

Compositions of the invention contained in water-soluble sachets may useful for treating one or more of the following conditions: nasal conditions, such as sinusitis and rhinosinusitis; respiratory tract infections, such as upper respiratory tract infections (e.g. tonsillitis, laryngitis and sinusitis or lower respiratory tract infections (e.g. bronchitis, pneumonia, bronchiolitis and tuberculosis); chronic obstructive pulmonary disease; and cystic fibrosis.

There may be provided a wound dressing which comprises a composition of the invention, and a water-soluble material which may function as a barrier or layer. The water-soluble material may be in contact with, or adjacent to, the composition. The composition of the invention may be enclosed by, or contained within, the water-soluble material. The water-soluble material may thus form a sachet, pouch or enclosure. The composition may be positioned between layers of the water-soluble material. The water-soluble material may be manufactured from a medical grade material. The water-soluble material may be dissolvable in water at 38°C. Preferably, the water-soluble material is non-toxic, anti-static, resistant to degradation by ultraviolet light and resistant to degradation by gases, oils and greases. In one example, the water-soluble material may be manufactured from a polymer or plastics material, such as polyvinyl alcohol. In use, the dressing may be applied to a wound, and the water-soluble material may thus dissolve in wound exudate, allowing the composition of the invention to contact the wound and deliver reactive oxygen to the wound. Advantageously, a measurable and accurate dose of reactive oxygen may thus be delivered to a wound. The water-soluble material may control release of the composition from the wound dressing.

The water-soluble material and the composition may be attached to the surface of a conventional aseptic wound dressing.

In some embodiments, it is advantageous for compositions for use in the invention, to have a particularly low water content. This may be the case, for example, if the composition is to be contained within a water-soluble container or sachet. If the water content is too high, it may only be possible to contain the composition in a water-soluble container for a short time. Reducing the water content may thus enable a longer shelf-life. A three-year shelf life, for instance, is particularly desirable.

In some embodiments, there is 12% or less by weight of water in the composition. In other embodiments, there is 10% by weight or less of water in the composition. In some embodiments, there is 5% by weight or less, or even 3% by weight or less of water in the composition. In some embodiments, the composition with reduced water content is in a liquid form. In some embodiments, the composition is in a solid form. Suitable solid forms include powders, flakes or granules. Other forms include lozenges or tablets. In some embodiments, the composition is a paste. A paste, for example, may not readily flow at ambient or room temperature (e.g. 20-26°C), but it may have a soft and/or malleable consistency.

In some embodiments, the composition may comprise dry honey or dried honey. Dry honey or dried honey products are commercially available and are typically obtained by processes such as spray-drying, freeze-drying or vacuum-drying. Compositions of the invention with reduced water content may thus be manufactured using such processes. Compositions of the invention may be obtained, or obtainable, by adding an enzyme that is able to convert a substrate to release hydrogen peroxide to a substance that includes a substrate for the enzyme (e.g. an unrefined natural substance, such as honey), wherein the substance has been dried and comprises 12% or less, 10% or less, 5% or less or 3% or less (by weight) of water. Alternatively, the enzyme may be added to the substance, and the resulting composition is dried. The enzyme may be in a dry or dried form when it is added to the substance. For example, the enzyme may have been freeze-dried. The enzyme may contain 12% or less, 10% or less, 5% or less or 3% or less (by weight) of water. Preferably, if the enzyme is in a dry or dried form, it contains less 5% (by weight) of water.

Additives may be added to facilitate the drying process or to improve the properties of the dried composition. For example, dried honey products have a tendency to cake or agglutinate unless processing additives are included. Suitable additives include processing aids, drying aids, bulking agents or anticaking agents. Additives may include starch, milk powder, calcium stearate, bran dextrins, lecithin and soy flour. Dried honey formulations typically contain greater than or equal to 50%, 65%, 70% (by weight) of honey. The remainder of the formulation may thus include suitable additives. In preferred embodiments, there are no additives added to facilitate the drying process or to improve the properties of the composition.

Consequently, according to the disclosure there may be provided a composition comprising: an enzyme that is able to convert a substrate to release hydrogen peroxide; and a substance that includes a substrate for the enzyme, wherein the enzyme is additional to any enzyme activity able to convert the substrate to release hydrogen peroxide that may be present in the substance, and wherein the composition comprises 12% or less, 10% or less, 5% or less or 3% or less (by weight) of water. The composition may be further defined as described herein. For example, the enzyme may be glucose oxidase and the substance may be an unrefined natural substance (such as honey), or it may be a purified substrate (such as glucose). The composition may be obtained or obtainable by freeze-drying. The composition may then be used in the treatment of microbial infections, and other medical conditions, as described herein. For example, the composition may be used to treat a microbial infection that comprises a biofilm, or a microbe that is capable of forming a biofilm. To treat a microbial infection, or medical condition, the composition may first be added to water to form an aqueous solution. Addition of water may initiate hydrogen peroxide production.

According to the disclosure, there may be provided a method comprising: drying a composition, the composition comprising an enzyme that is able to convert a substrate to release hydrogen peroxide, and a substance that includes a substrate for the enzyme, wherein the enzyme is additional to any enzyme activity able to convert the substrate to release hydrogen peroxide that may be present in the substance; and. The composition may be dried by spray-drying, freeze-drying or vacuum-drying. The drying may reduce the water content in the composition to 12% or less, 10% or less, 5% or less or 3% or less (by weight) of water.

Alternatively, there may be provided a method, comprising: adding an enzyme to a dried substance that includes a substrate for the enzyme. Alternatively, the method may comprise i) drying a substance that includes a substrate for the enzyme, such that the drying results in the substance having 12% or less, 10% or less, 5% or less or 3% or less (by weight) of water; and ii) adding the enzyme to the dried substance, the enzyme being additional to any enzyme activity able to convert the substrate to release hydrogen peroxide that may be present in the substance. The substance may be dried by spray-drying, freeze-drying or vacuum-drying. The enzyme added to the substance may be in a dry or dried form.

Compositions or mixtures of the invention may comprise sufficient enzyme and substrate to provide for sustained release of hydrogen peroxide at a specific level or concentration. For example, compositions or mixtures for use in the invention, may provide for sustained release of hydrogen peroxide at a concentration of at least 2 ppm, at least 5 ppm, at least 10 ppm, at least 20 ppm or at least 50 ppm. In preferred embodiments, the level may be at least 2 ppm. In some embodiments, the concentration may be, at the most, 500 ppm, 200 ppm, 100 ppm, 50 ppm, 20 ppm or 10 ppm. In preferred embodiments, the level may be 50 ppm or less. In more preferred embodiments, the level may be 20 ppm or less. In even more preferred embodiments, the level may be 10 ppm or less. For example, the concentration may be 10 to 500 ppm, 20 to 200 ppm or 50 to 100 ppm, 2 to 50 ppm, 2 to 20 ppm or 5 to 10 ppm. If the composition does not comprise sufficient free water to allow the enzyme to convert the substrate (e.g. if the composition is a dry or dried composition), hydrogen peroxide production may only occur once it has been diluted by water and there is sufficient free water to allow the enzyme to convert the substrate. Addition of water may thus initiate hydrogen peroxide production. Compositions or mixtures may provide for sustained release of hydrogen peroxide for at least 1 hour, at least 12 hours, at least 24 hours, at least 2 days, or at least 4 days. Preferably, the level of hydrogen peroxide is sustained for at least 4 days. In preferred embodiments, the level of hydrogen peroxide is sustained at 10 to 500 ppm for at least 1 hour, at least 12 hours, at least 24 hours, at least 2 days, or at least 4 days. In other embodiments, the level of hydrogen peroxide is sustained at 50 to 100 ppm for at least 1 hour, at least 12 hours, at least 24 hours, at least 2 days, or at least 4 days. In other embodiments, the level of hydrogen peroxide is sustained at 2 to 50 ppm for at least 1 hour, at least 12 hours, at least 24 hours, at least 2 days, or at least 4 days. In other embodiments, In other embodiments, the level of hydrogen peroxide is sustained at 5 to 10 ppm for at least 1 hour, at least 12 hours, at least 24 hours, at least 2 days, or at least 4 days.

Compositions for use in the invention, may comprise a non-aqueous solvent. This may be particularly advantageous if the composition comprises an unrefined natural substance such as honey. Honey, for example, has a high viscosity and is sticky, which can make it difficult to handle and deliver with certain products. If a storage-stable composition is desired, it may not be possible to decrease viscosity by adding aqueous solvent. The viscosity of honey may be reduced by using a non-aqueous solvent.

By forming a solution in a non-aqueous solvent, the substance in the composition may become more readily processable and less sticky. Such solutions may thus be coated onto substrates, such as fabric substrates, and may thus provide a component of a wound dressing. Furthermore, a less viscous composition may be sprayable.

So, according to the disclosure, there is provided a composition for generating antimicrobial activity comprising an enzyme that is able to convert a substrate to release hydrogen peroxide, a substance that includes a substrate for the enzyme, and a non-aqueous solvent.

In compositions that comprise a non-aqueous solvent, the substance may be an unrefined natural substance, such as honey, or a substance that includes a purified substrate for the enzyme, as described herein.

According to the disclosure, there is provided a composition for generating anti-microbial activity, wherein the composition comprises an enzyme that is able to convert a substrate to release hydrogen peroxide, an unrefined natural substance that includes a substrate for the enzyme, and a non-aqueous solvent.

According to the disclosure, there is provided a composition for generating anti-microbial activity, wherein the composition comprises an enzyme that is able to convert a substrate to release hydrogen peroxide, a substance that includes a purified substrate for the enzyme, and a non-aqueous solvent.

According to the disclosure there is provided a composition for use in the treatment of a wound having a bacterial load of greater than 10⁵ organisms/gram of tissue, wherein the composition comprises an enzyme that is able to convert a substrate to release hydrogen peroxide, a substance that includes a substrate for the enzyme, and a non-aqueous solvent.

According to the disclosure, there is provided a composition for generating anti-microbial activity that comprises: an enzyme that is able to convert a substrate to release hydrogen peroxide; a substance that includes a substrate for the enzyme; a salt; and a non-aqueous solvent.

Compositions that comprise a non-aqueous solvent may comprise a polymer, as described herein.

According to the disclosure, there is provided a composition for generating anti-microbial activity, wherein the composition comprises: an enzyme that is able to convert a substrate to release hydrogen peroxide; an unrefined natural substance that includes a substrate for the enzyme, a polymer; and a non-aqueous solvent.

According to the disclosure, there is provided a composition for generating anti-microbial activity, wherein the composition comprises: an enzyme that is able to convert a substrate to release hydrogen peroxide; a substance that comprises a purified substrate for the enzyme; a polymer; and a non-aqueous solvent.

Compositions comprising a non-aqueous solvent may be storage-stable compositions which do not include sufficient free water to allow the enzyme to convert the substrate, as described herein.

According to the disclosure, there is provided a storage-stable composition for generating antimicrobial activity, which comprises: a purified enzyme that is able to convert a substrate to release hydrogen peroxide; a substance that includes a substrate for the enzyme; and a non-aqueous solvent, wherein the composition does not include sufficient free water to allow the enzyme to convert the substrate.

In compositions comprising a non-aqueous solvent the substance may lack catalase activity, as described herein.

According to the disclosure, there is provided a storage-stable composition for generating antimicrobial activity, which comprises: an enzyme that is able to convert a substrate to release hydrogen peroxide; a substance that lacks catalase activity and that includes a substrate for the enzyme; and a non-aqueous solvent wherein the composition does not include sufficient free water to allow the enzyme to convert the substrate.

Compositions comprising a non-aqueous solvent may provide for sustained release of hydrogen peroxide for a period of time, as described herein. For example, compositions may provide for sustained release of hydrogen peroxide for at least twenty four hours, more preferably at least forty eight hours, possibly following dilution of the composition. Suitably compositions provide for sustained release of hydrogen peroxide at a level of less than 2 mmol/litre for a period of at least twenty four hours, following dilution of the composition.

Compositions comprising non-aqueous solvents, may comprise sufficient enzyme and substrate to provide for sustained release of at least 0.1, 0.5, 1 or 1.5 mmol/litre hydrogen peroxide for a period of at least 24 hours, more preferably 48 hours.

According to the disclosure, there is provided a composition for generating anti-microbial activity that comprises: an enzyme that is able to convert a substrate to release hydrogen peroxide; a substance that includes a purified substrate for the enzyme; and a non-aqueous solvent, wherein the composition is a storage-stable composition that does not include sufficient free water to allow the enzyme to convert the substrate, and wherein the composition provides for sustained release of hydrogen peroxide at a level of less than 2 mmol/litre for a period of at least 24 hours following dilution of the composition.

According to the disclosure there is provided a composition comprising: an enzyme that is able to convert a substrate to release hydrogen peroxide; a substance that includes a substrate for the enzyme; and a non-aqueous solvent, wherein the enzyme is additional to any enzyme activity able to convert the substrate to release hydrogen peroxide that may be present in the substance, and wherein the composition comprises 12% or less, 10% or less, 5% or less or 3% or less (by weight) of water.

In compositions comprising a non-aqueous solvent, the enzyme is additional (i.e. added as a result of human intervention) to any enzyme activity able to convert the substrate to release hydrogen peroxide (referred to herein as "substrate conversion activity") that may be present in the substance. Compositions comprising non-aqueous solvent may comprise a purified enzyme, as described herein.

There is also provided according to the disclosure compositions comprising a non-aqueous solvent for use as a medicament. For example, such compositions may be used for treating, or used in methods of treating, any disease or condition described herein. In some examples, a composition comprising a non-aqueous solvent may be for use in preventing or treating a microbial infection, such as one that comprises a biofilm, or a microbe that is capable of forming a biofilm.

In compositions of the invention that comprise a non-aqueous solvent, the non-aqueous solvent may comprise ethanol, dimethyl sulphoxide, glycerol, ethylene glycol or propylene glycol. Preferably, the non-aqueous solvent is or comprises glycerol. Glycerol may act as a humectant and so compositions comprising glycerol may assist in softening or moisturising dry skin.

Solubility parameters and viscosity parameters for various non-aqueous solvents are shown in the following table.

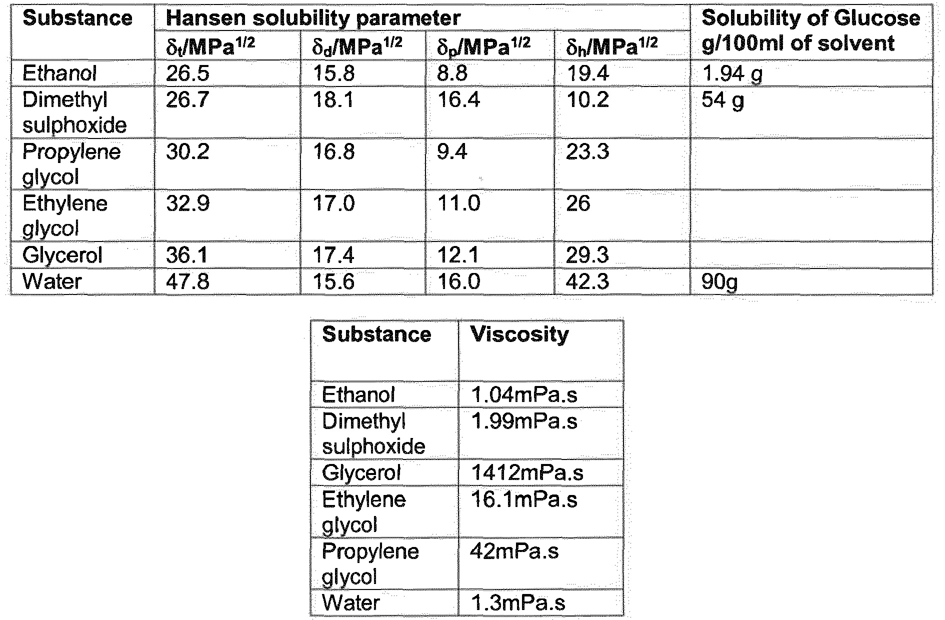

In preferred embodiments, non-aqueous solvents may be selected so that they have solubility parameters in the range of the non-aqueous solvents exemplified in the tables below. For example, δₜ/MPa^{1/2} may be from 26 to 50, such as 26.5 to 47.8. δ_{d}/MPa^{1/2} may be from 15 to 19, such as 15.6 to 18.1. δₚ/MPa^{1/2} may be from 8 to 16, such as 8.8 to 16. δₕ/MPa^{1/2} may be from 10 to 45, such as 10.2 to 42.3.

The non-aqueous solvent may be selected depending on the desired viscosity. For example, if a greater viscosity is desired, glycerol may be preferred.

In preferred embodiments of compositions comprising a non-aqueous solvent, the compositions may comprise at least 10% by weight of the non-aqueous solvent. In other embodiments, the composition may comprise at least 20% by weight of the non-aqueous solvent. In other embodiments, the composition may comprise at least 25% by weight of the non-aqueous solvent. In other embodiments, the composition may comprise at least 50% by weight of the non-aqueous solvent. In some embodiments, the composition may comprise at least 75% by weight of the non-aqueous solvent. The amount of aqueous solvent may be varied depending on the intended application of the composition. For example, sprayable compositions, or compositions for use with an antibacterial wipe may comprise higher levels of the non-aqueous solvent, such that the compositions have a lower viscosity. In some embodiments, the amount of non-aqueous solvent in the composition may be 50-90%, by weight.

For some applications, it may be desirable to have compositions that comprise lower amounts of non-aqueous solvent, such as compositions for use in forming wound dressings. Consequently, some compositions may comprise a maximum amount of non-aqueous solvent. The maximum amount of non-aqueous solvent in the composition may be 50% by weight or less. In some embodiments, the amount of non-aqueous solvent in the composition may be 1-50, 5-50 or 10-50%, by weight.

In compositions of the invention that comprise non-aqueous solvents, the compositions may comprise honey. If the compositions contain honey, the amount of honey may be at least 20% by weight. In some embodiments, honey may be present in an amount of at least 25% by weight. In some embodiments, the honey may be present in an amount of at least 50% by weight. In some embodiments, the honey may be present in an amount of at least 75% by weight. In some embodiments, the amount of honey in the composition may be 50% or less, by weight. In some embodiments, the amount of honey in the composition may be 25% or less by weight. In some embodiments, honey may be present in the composition in an amount of 50-90%. In some embodiments, the honey in the composition may be present in an anmount of 1-50, 5-50 or 10-50 % by weight.

If compositions are to be used to coat a substrate, such as a fabric, the weight of the composition is preferably at least 100g per square metre of the substrate. In other embodiments, the weight of the composition may be at least 200g per square metre of the substrate. In other embodiments, the weight of the composition may be at least 300g per square mere of the substrate.

In some embodiments, compositions that comprise a non-aqueous solvent comprise substantially no polymer, for example less than 1%, 0.5%, 0.1% or 0.01% by weight of the polymer.

In preferred embodiments, compositions of the invention that comprise a non-aqueous solvent are not, or do not comprise, the following:

### i) Alcoholic Gel

| | |
|---|---|
| Carbopol 940 | 0.3 % |
| Triethanolamine | 0.4 % (needed for pH and stability control) |
| Active honey | 65% |
| Ethanol | 25.0 % |
| water qs | |

wherein "Active honey" is honey with added glucose oxidase

In preferred embodiments, compositions of the invention that comprise a non-aqueous solvent are not, or do not comprise, the following:

### ii) Non-Aqueous spray

| | |
|---|---|
| Active honey | 70% |
| Proplyene Glycol | 30% |

In preferred embodiments, compositions of the invention that comprise a non-aqueous solvent are not, or do not comprise, the following:

### iii) Fungal Nail Treatment

Plaster with Active Honey in a foam well
Hydroxypropylcellulose
Glycerol
Isopropyl Alcohol
Citric Acid
Monohydrate

In preferred embodiments, compositions of the invention that comprise a non-aqueous solvent are not, or do not comprise, the following:

### iv) Throat spray

Honey and glycerol, preferably comprising 5-20% honey

In preferred embodiments, compositions of the invention that comprise a non-aqueous solvent are not, or do not comprise, the following:

### v) Cream

| | |
|---|---|
| Honey | 15% |
| Carbomer | 2.63% |
| Dimethicone | 0.13% |
| Disodium Lauryl Sulphosuccinate | 0.05% |
| Disodium Edetate | 0.13% |
| Glycerol | 5.26% |
| Silica Colloidal Hydrated | 0.33% |
| Poloxamer | 0.26% |
| Sodium Hydroxide | 0.41% |
| Purified Water | 85.03% |

### vi) Powder

Active honey
Maltodextrin
Ibuprofen
Microcrystalline Cellulose (CMC)
Polyvinyl Pyrrolidone (PVP)

In certain embodiments, a composition of the invention may exclude any one, or any combination, of compositions (i) to (vi) above. In particular, a composition of the invention may exclude a composition of (i); or (ii); or (iii); or (iv); (v); or (vi) above; or a composition of the invention may exclude any of the following combinations of the above compositions:
(i) and (ii); (i) and (iii); (i) and (iv); (i) and (v); (i) and (vi); (ii) and (iii); (ii) and (iv); (ii) and (v); (ii) and (vi); (iii) and (iv); (iii) and (v); (iii) and (vi); (iv) and (v); (iv) and (vi); or (v) and (vi); or
(i), (ii), and (iii); (i), (ii), and (iv); (i), (ii), and (v); (i), (ii), and (vi); (i), (iii), and (iv); (i), (iii), and (v); (i), (iii), and (vi); (i), (iv), and (v); (i), (iv), and (vi); (i), (v), and (vi); (ii), (iii), and (iv); (ii), (iii), and (v); (ii), (iii), and (vi); (ii), (iv), and (v); (ii), (iv), and (vi); (ii), (v), and (vi); (iii), (iv), and (v); (iii), (iv), and (vi); or (iii), (v), and (vi); or
(i), (ii), (iii), and (iv); (i), (ii), (iii), and (v); (i), (ii), (iii), and (vi); (i), (iii), (iv), and (v); (i), (iii), (iv), and (vi); (i), (iii), (v), and (vi); (i), (iv), (v), and (vi); (ii), (iii), (iv), and (v); (ii), (iii), (iv), and (vi); (ii), (iii), (v), and (vi); (ii), (iv), (v), and (vi); or (iii), (iv), (v), and (vi); or
(i), (ii), (iii), (iv), and (v); (i), (ii), (iii), (iv), and (vi); (i), (ii), (iv), (v), and (vi); or (i), (iii), (iv), (v), and (vi); or
(i), (ii), (iii), (iv), (v), and (vi).

Specific examples of compositions comprising non-aqueous solvent are described in Example 23.

Examples are described below, with reference to the accompanying drawings in which:
Figure 1 shows the effect of: (A) neat SH1 Surgihoney; or (B) serial dilutions of SH1 Surgihoney on formation of biofilms by *Pseudomonas aeruginosa* strain PA01, and clinical burn wound isolate 1054; and (C) neat SH1 Surgihoney; or (D) serial dilutions of SH1 Surgihoney on formation of biofilms by *Acinetobacter baumannii* control strain AYE, and ACI clone NCTC_13420 (C59);
Figure 2 shows the effect of neat Manuka honey on formation of biofilms by: (A) *Pseudomonas aeruginosa* strain PA01, and clinical burn wound isolate 1054; or (B) *Acinetobacter baumannii* control strain AYE, and ACI clone NCTC_13420 (C59); and (C) serial dilutions of Manuka honey on formation of biofilms by *Pseudomonas aeruginosa* strain PA01, and clinical burn wound isolate 1054 (upper part of Figure 2(C)), or *Acinetobacter baumannii* control strain AYE, and ACI clone NCTC_13420 (C59) (lower part of Figure 2(C));
Figure 3 shows the effect of neat and serial dilutions of SH1, SH2, and SH3 Surgihoney on formation of biofilms by: (A) *Pseudomonas aeruginosa* strain PA01, and clinical burn wound isolate 1054; and (B) *Acinetobacter baumannii* control strain AYE, and ACI clone NCTC_13420 (C59);
Figure 4 shows the effect of Surgihoney preparations SH1, SH2, and SH3, and Manuka honey (MH), on biofilm formation by a biofilm-producing isolate of *Pseudomonas aeruginosa* (PS_1586);
Figure 5 shows the effect of Surgihoney preparations SH1, SH2, and SH3, and Manuka honey (MH), on biofilm formation by a biofilm-producing isolate of *Pseudomonas aeruginosa* (PA_6749);
Figure 6 shows the effect of Surgihoney preparations SH1, SH2, and SH3, and Manuka honey (MH), on biofilm formation by a biofilm-producing isolate of *Acinetobacter baumannii* (ACI_19606);
Figure 7 shows the effect of Surgihoney preparations SH1, SH2, and SH3, and Manuka honey (MH), on biofilm formation by a biofilm-producing isolate of *Acinetobacter baumannii* (ACI_C60);
Figure 8 shows the effect of Surgihoney SH1, SH2, and SH3 preparations, and Manuka honey (MH), on the prevention or reduction of the seeding of pre-formed biofilms produced by *Acinetobacter baumannii* ACI_C59;
Figure 9 shows the effect of Surgihoney SH1, SH2, and SH3 preparations, and Manuka honey (MH), on the prevention or reduction of the seeding of pre-formed biofilms produced by *Acinetobacter baumannii* ACI_AYE;
Figure 10 shows the effect of Surgihoney SH1, SH2, and SH3 preparations on prevention of biofilm formation by *Pseudomonas aeruginosa* (control strain PA01) in comparison with deactivated Surgihoney (DE), Manuka honey (MH: 'Comvita Manukacare 18+'), acetic acid (AA), and several commercially available wound dressings, and wound creams;
Figure 11 shows the effect of Surgihoney SH1, SH2, and SH3 preparations on prevention of biofilm formation by *Acinetobacter baumannii* (control strain AYE) in comparison with deactivated Surgihoney (DE), Manuka honey (MH: 'Comvita Manukacare 18+'), acetic acid (AA), and several commercially available wound dressings, and wound creams;
Figure 12 shows the effect of Surgihoney preparation SH1 and Manuka honey (MH) on biofilm formation by multi-drug resistant (MDR) biofilm-producing isolates of *Pseudomonas aeruginosa* (VIM positive) and CRE *Klebsiella pneumoniae,* and two biofilm-producing isolates of *Acinetobacter baumanii* (ACI_C60 (1), ACI_C60 (2));
Figure 13 shows the effect of Surgihoney treatment of chronic rhinosinusitis (CRS)-associated S. *aureus* planktonic populations at different concentrations of Surgihoney to determine the minimum inhibitory concentration (MIC) of Surgihoney. The first column for each treatment condition represents the result for a Methicillin-resistant *Staphylococcus aureus* (MRSA) isolate, the next four columns represent the results for different Methicillin-sensitive *Staphylococcus aureus* (MSSA) isolates;
Figure 14 shows photographs of chocolate blood agar plates onto which planktonic Methicillin-resistant *Staphylococcus aureus* (MRSA) or Methicillin-sensitive *Staphylococcus aureus* (MSSA) have been subcultured with effective concentrations of Surgihoney to determine the minimum bactericidal concentration (MBC) of Surgihoney;
Figure 15 shows the effect of Surgihoney treatment of CRS-associated S. *aureus* biofilms at different concentrations of Surgihoney. **p=0.002;
Figure 16A shows different hydrogen peroxide production rates for Surgihoney SH1, SH2, and SH3. Figure 16B shows the relationship between phenol activity and maximum hydrogen peroxide activity in Surgihoney SH1, SH2, and SH3;
Figure 17 shows a photograph of a Salter respiratory nebuliser with a nebuliser face mask tub attached. The reservoir contains a liquefied mixture of Surgihoney and saline;
Figure 18 shows photographs of blood agar plates that have been cultured with paper discs inoculated with *Staphylococcus aureus* and contacted with nebulised Surgihoney for 5, 15, 20, or 30 minutes;
Figure 19 shows the effect of Surgihoney treatment of CRS-associated S. *aureus* biofilms (MSSA) at different concentrations of Surgihoney;
Figure 20 shows: (A) a photograph of a long-standing ischaemic ulcer chronically infected with *Pseudomonas aeruginosa;* (B) a photograph of the same ulcer after a 7 day treatment with Surgihoney;
Figure 21 shows: (A) a photograph of a paediatric MRSA wound infection; (B) a photograph of the same wound infection after a 10 day treatment with Surgihoney;
Figure 22 shows: (A) a photograph of a CA MRSA superficial infection; (B) a photograph of the same infection after a 5 day treatment with Surgihoney; and (C) a photograph of the same infection after a 10 day treatment with Surgihoney;
Figure 23 shows the results of an assay for the cytotoxic activity of Surgihoney;
Figure 24 shows hydrogen peroxide production by dried Surgihoney granules, and dried Surgihoney that has been dissolved in water to form a solution; and
Figure 25 shows hydrogen peroxide production by the solution of Surgihoney of Figure 24 after various time periods, up to four days.

The Examples below describe determination of the *in vitro* antibacterial activity of a composition for use according to the invention (Surgihoney) against important biofilm-forming burn wound pathogens.

Surgihoney (SH) has previously demonstrated highly potent inhibitory and cidal activity against a wide range of planktonic Gram-positive and Gram-negative bacteria in both laboratory tests and clinical practice. Prophylactic application to caesarean wounds has demonstrated eradication of resistant organisms and reduced rates of colonisation and infection.

Given the global concerns surrounding antimicrobial resistance, and the challenges posed by biofilms, the *in vitro* antibacterial activity of three different formulations of SH (SH1, SH2 and SH3) against biofilm-forming isolates of *Pseudomonas aeruginosa* and *Acinetobacter baumannii* was investigated to assess whether SH can i) prevent the formation of a biofilm and ii) eradicate or prevent seeding of a pre-formed biofilm.

The anti-biofilming properties of Surgihoney formulations SH1, SH2 and SH3 were investigated and compared against standard Manuka honey using two biofilm assays. These enabled *in vitro* measurement of the 'Minimum Biofilm Inhibition Concentration' (MBIC), and the 'Minimum Biofilm Eradication Concentration' (MBEC) of SH. Further experiments were performed to compare the anti-biofilming activity of SH to a range of commercial dressings, including one that contains 20% honey (L-Mesitran net).

### Example 1

### Prevention of biofilm formation by Surgihoney compared with Manuka honey

This example describes a first experiment (Experiment 1), which compares the effect of Surgihoney (SH1), and Manuka honey (MH), and a second experiment (Experiment 2), which compares the effect of three different strength preparations of Surgihoney (SH1, SH2, and SH3) on biofilm formation by biofilm-producing isolates of *Pseudomonas aeruginosa* (control strain PA01, and clinical burn wound isolate 1054) and *Acinetobacter baumannii* (control strain AYE, and UK ACI clone NCTC_13420(C59)).

### Methods

For each neat honey sample, one loopful of honey was placed into 100µl water in a well of a 96-well microtitre plate. For the diluted honey samples, approximately 2.5ml honey was placed into a universal with 6ml of water. 3ml of this mixture was then serially diluted down to 1:2048. 100µl of each serial dilution was added to a different well of the 96-well plate.

Overnight cultures of the isolates were diluted in Muller-hinton broth to an OD600 of 0.1. 100µl of the diluted overnight culture was added to each neat or diluted honey sample well. Each positive control well contained 100µl diluted overnight culture, and 100µl water. Each negative control well contained 200µl broth or 200µl neat honey sample.

The plate was incubated for 72 hours at 33°C (wound temperature) to encourage biofilm formation. The plate was then developed using crystal violet dye (which binds to dead and living biofilms), and visualised following solubilisation of the dye using 70% ethanol.

A spectrophotometer was then used to assess the optical density (OD) of the solubilised dye. The OD reading corresponds to the amount of incorporated crystal violet. Higher OD readings represent dark wells, and greater mass of biofilm. The OD readings were then plotted to make a graph.

### Results

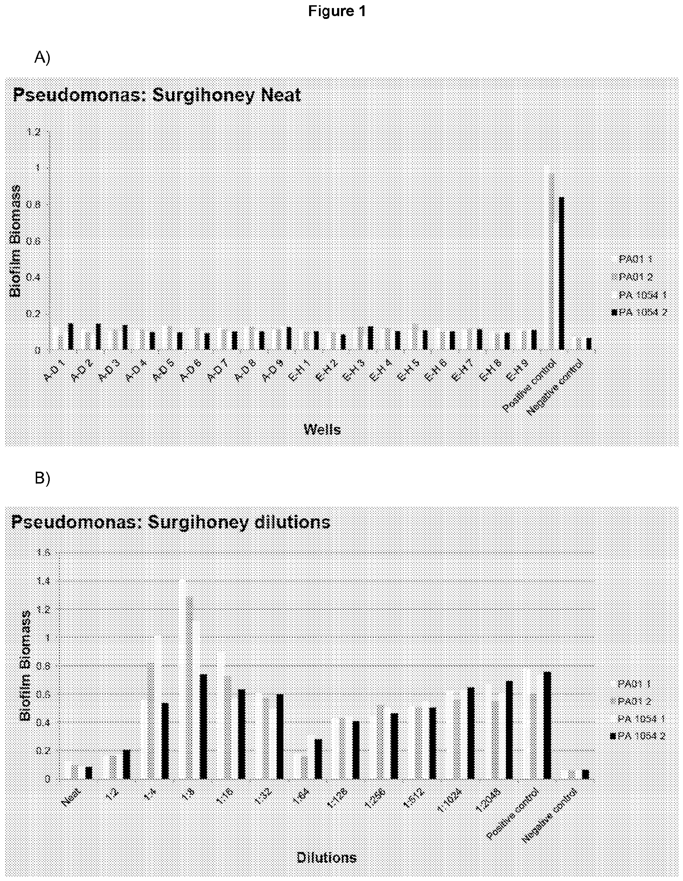

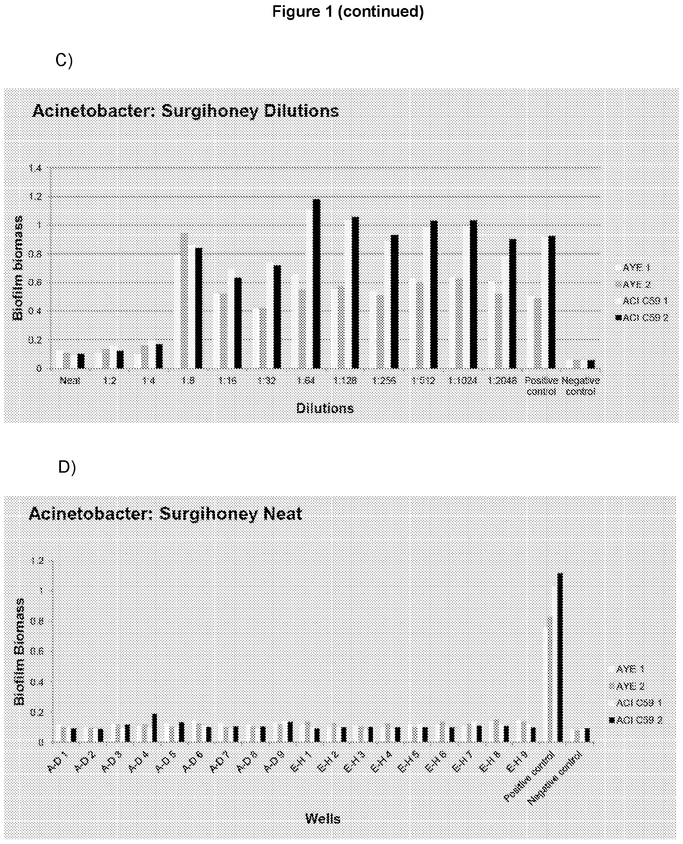

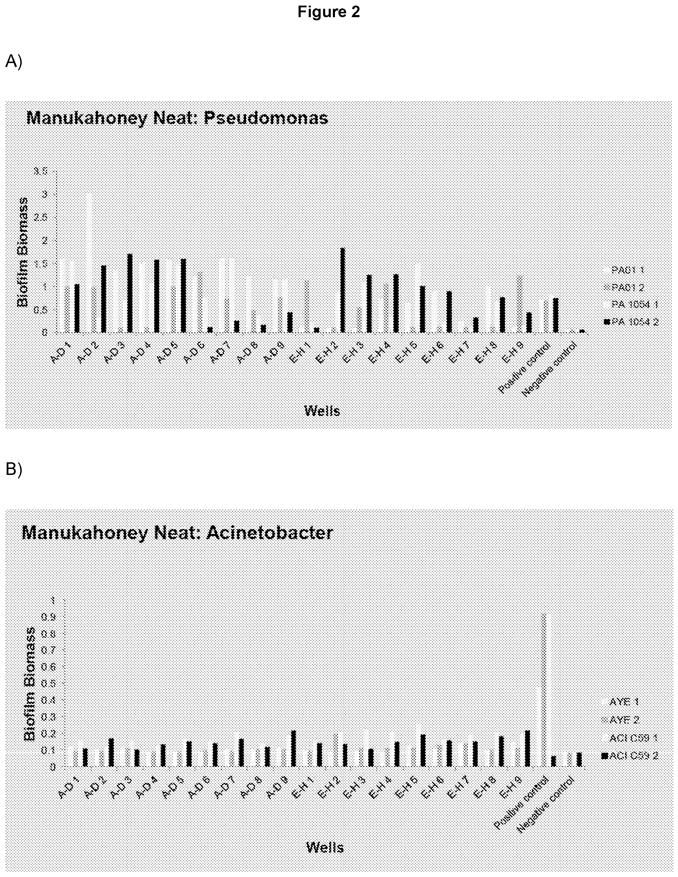

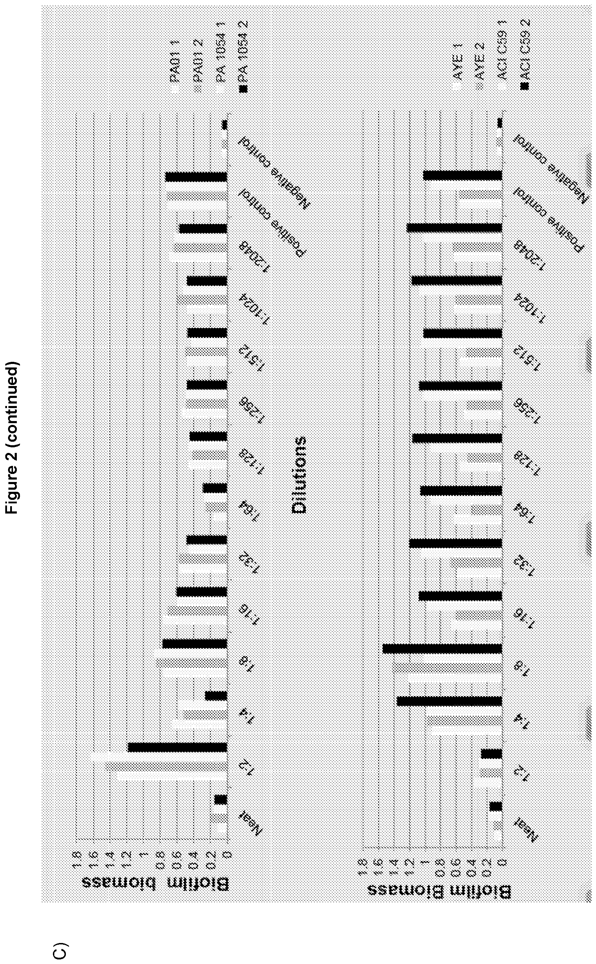

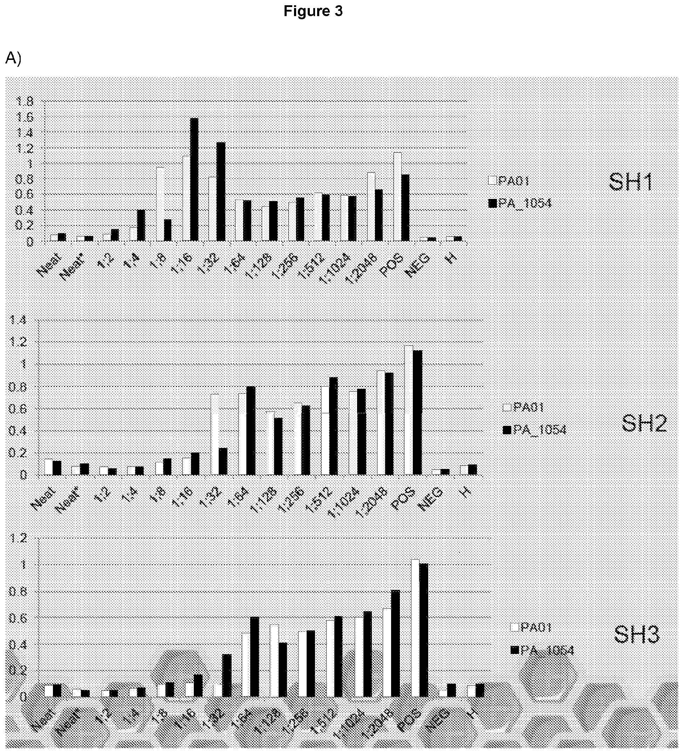

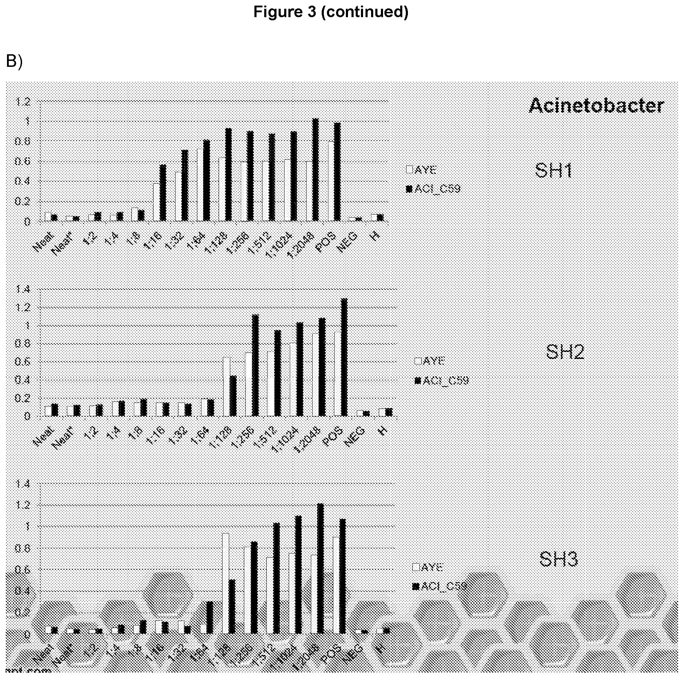

The results are shown in Figures 1-3, and summarised in the table below, which records the Minimum Biofilm Inhibitory Concentration (MBIC) for each strain/isolate (using an OD of 0.3 as a cut-off):

| **Strain/Isolate** | **MBIC** | | | | |
|---|---|---|---|---|---|
| | **Experiment 1** | | **Experiment 2** | | |
| | **SH1** | **MH** | **SH1** | **SH2** | **SH3** |
| PA01 | 1:2 | Neat¹ | 1:4* | 1:16* | 1:32* |
| 1054 | 1:2 | Neat¹ | 1:8* | 1:32* | 1:16* |
| AYE | 1:4 | 1:2 | 1:8* | 1:64* | 1:64* |
| C59 | 1:4 | 1:2 | 1:8* | 1:64* | 1:32* |

| | | | | | |
|---|---|---|---|---|---|
| ¹ The activity of Manuka honey against *Pseudomonas* was sporadic * = Statistically significant (P<0.005) when student t-test performed to compare growth with honey and positive control. | | | | | |

The results show that Surgihoney SH1 prevented *Pseudomonas* biofilm formation when used neat or at 1:2 dilution, and prevented *Acinetobacter* biofilm formation when used neat or at 1:2 or 1:4 dilution.

Manuka honey had a sporadic effect on *Pseudomonas* biofilm production when used neat, but prevented *Acinetobacter* biofilm formation when used neat or at 1:2 dilution.

Surgihoney SH2 and SH3 also prevented *Pseudomonas* and *Acinetobacter* biofilm formation, but were able to do so at lower concentrations than SH1 Surgihoney.

There appeared to be little significant difference in the ability of Surgihoney SH2 and SH3 to inhibit *Pseudomonas* and *Acinetobacter* biofilm formation.

Biofilm formation by *Acinetobacter* appeared to be more sensitive to Surgihoney and Manuka honey treatment than *Pseudomonas* biofilm formation.

### Conclusions

It was concluded from these results that Surgihoney is able to prevent biofilm formation of biofilms, and that Surgihoney SH2 and SH3 were able to prevent biofilm formation at lower concentrations than Surgihoney SH1. Each Surgihoney preparation was able to prevent biofilm formation at a lower concentration than Manuka honey.

### Example 2

### Prevention of biofilm formation by different strength preparations of Surgihoney compared with Manuka honey

This example describes the effect of Surgihoney1 (SH1), Surgihoney 2 (SH2), Surgihoney 3 (SH3) and Manuka honey (MH), on biofilm formation by biofilm-producing isolates of *Pseudomonas aeruginosa* (PS_1586] and PS_6749) and *Acinetobacter baumannii* (ACI_C60 and ACI_19606).

### Methods

Each honey was placed in a 37°C incubator for 30 minutes. Approximately 3ml each honey was then placed into a universal, and 3ml sterile water was added. This mixture was then vortexed vigorously and serially diluted down to 1:4096.

100µl of a diluted overnight culture of each isolate was added to 100µl of diluted honey in a 96-well microtitre plate, and incubated for 72 hours at 33°C (wound temperature) to encourage biofilm formation.

The plate was then developed using crystal violet dye, and visualised following solubilisation of the dye using 70% ethanol.

A spectrophotometer was then used to assess the optical density (OD) of the solubilised dye. The OD reading corresponds to the amount of incorporated crystal violet. Higher OD readings represent dark wells, and greater mass of biofilm. The OD readings were then plotted to make a graph.

### Results

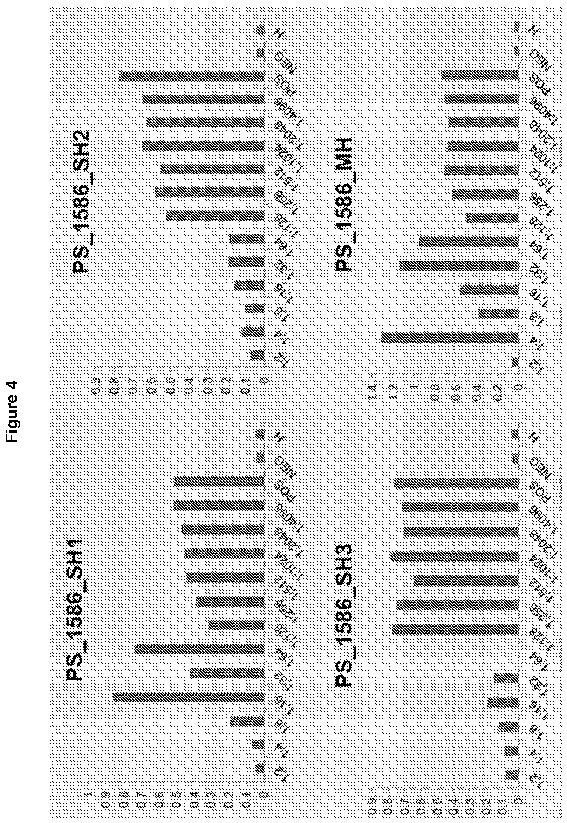

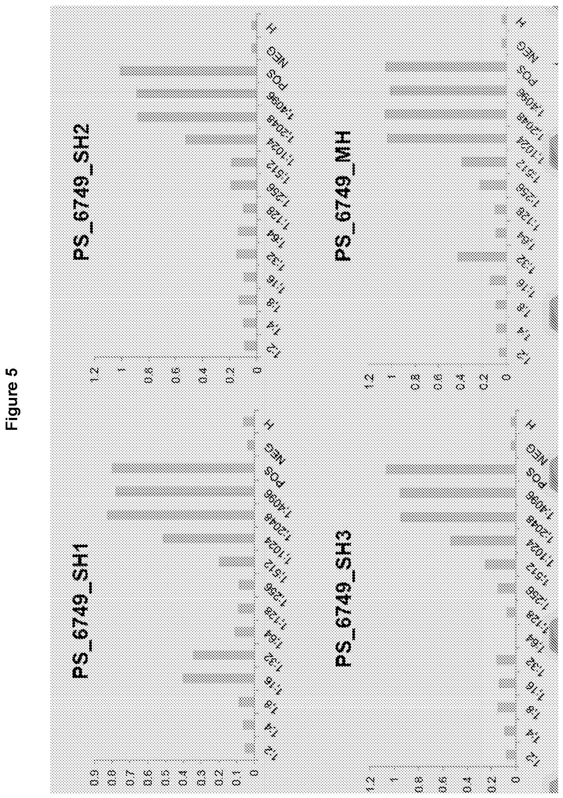

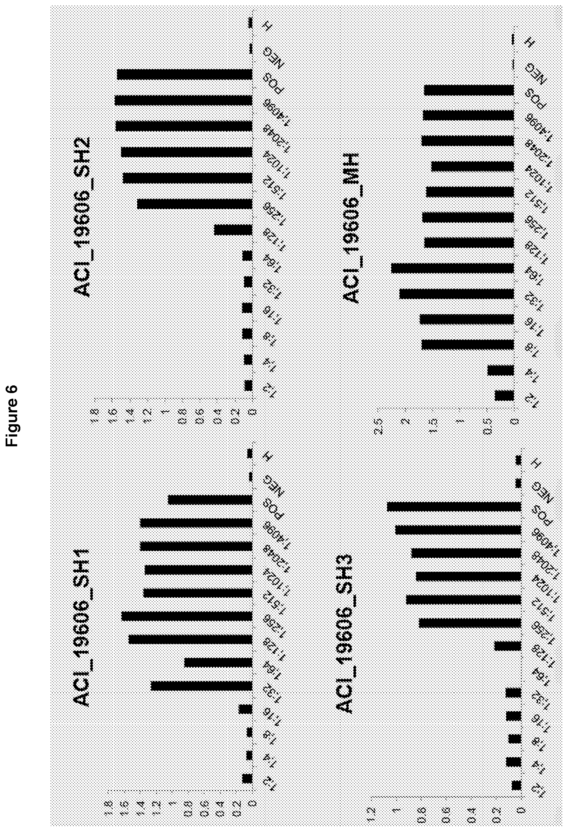

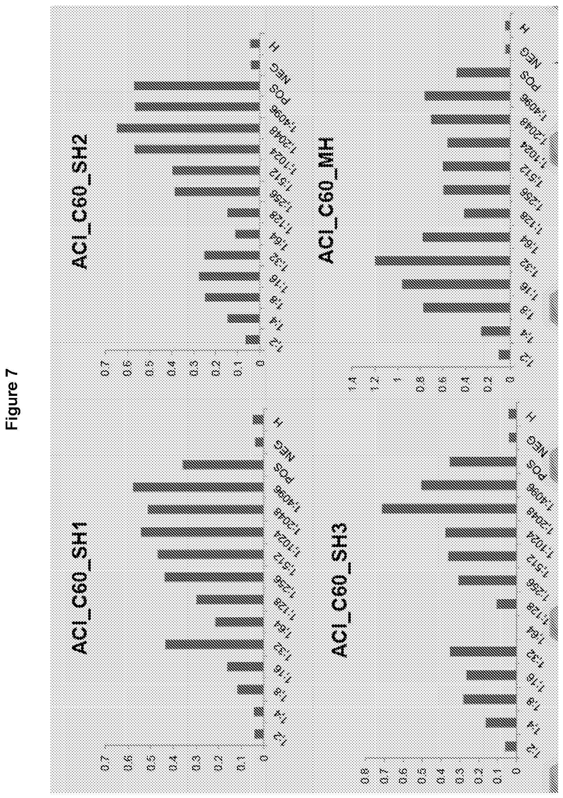

The results are shown in Figures 4-7, and summarised in the table below, which records the Minimum Biofilm Inhibitory Concentration (MBIC) for each isolate (using an OD of 0.3 as a cut-off):

| **Isolate** | **MBIC** | | | |
|---|---|---|---|---|
| | **SH1** | **SH2** | **SH3** | **MH** |
| PS_1586 | 1:8 | 1:64 | 1:32¹ | 1:2² |
| PS_6749 | 1:8 | 1:512 | 1:512 | 1:16³ |
| ACI_C60 | 1:16⁴ | 1:128⁴ | 1:16¹ | 1:4 |
| ACI_19606 | 1:16 | 1:64 | 1:128 | 1:2 |

| | | | | |
|---|---|---|---|---|
| ¹ 1:64 dilution not tested ² Enhanced biofilming observed at 1:4 dilution compared to lower dilutions (see below) ³ Enhanced biofilming observed at 1:32 dilution compared to lower dilutions (see below) ⁴ Enhanced biofilming observed at 1:8 to 1:32 dilutions compared to lower dilutions (see below) The positive controls (POS) show that all the isolates were able to form a biofilm, and the negative controls (NEG) show that there was no contamination. | | | | |

For some of the isolates, enhanced biofilming is observed at certain concentrations, compared with biofilming at higher and lower concentrations, of the Surgihoney or Manuka honey. This effect has been observed occasionally with other biocides. It is believed to be due to the 'stress' of the biocide causing enhanced biofilming.

### Conclusions

It was concluded from these results that each Surgihoney preparation (SH1, SH2, and SH3) was able to prevent each of the isolates tested from forming a biofilm. The SH2 and SH3 Surgihoney preparations were able to do so at lower concentrations than S1 Surgihoney.

The optimum Surgihoney preparation for preventing biofilm formation of the isolates tested was SH2. A dilution of 1:64 SH2 was able to prevent biofilm formation of each isolate tested.

Each strength preparation of Surgihoney tested (SH1, SH2, and SH3) was generally able to prevent biofilm formation at lower concentrations than Manuka honey.

### Example 3

### Prevention of pre-formed biofilm seeding by Surgihoney

This example describes the effect of Surgihoney SH1, SH2, and SH3 preparations, and Manuka honey (MH), on the prevention or reduction of the seeding of pre-formed biofilms produced by two isolates of *Acinetobacter baumannii* (ACI_AYE and ACI_C59).

### Methods

200µl of a serially diluted overnight culture of *Acinetobacter baumannii* (ACI_AYE or ACI_C59) was added to each well of a 96-well microtitre plate. A PCR peg plate was placed on top of the microtitre plate so that each well contains a 'peg' on which a biofilm can form. The 96-well plate was then incubated for 72 hours at 33°C to encourage biofilms to grow.

After 72 hours, the pegs were washed and then placed into a further 96-well plate with wells containing the test agent (Surgihoney or Manuka honey), or broth alone (for the controls). After 24 hours, the pegs were washed and then placed into another 96-well plate containing sterile broth for overnight incubation. The OD of the broth was assessed the following day using the spectrophotometer (as described in Examples 1 and 2 above). Turbid broth has a high OD and represents successful seeding of the biofilm.

Finally, the pegs were subjected to a crystal violet assay (as described in Examples 1 and 2) to prove that biofilms were present on the pegs in the first place.

### Results

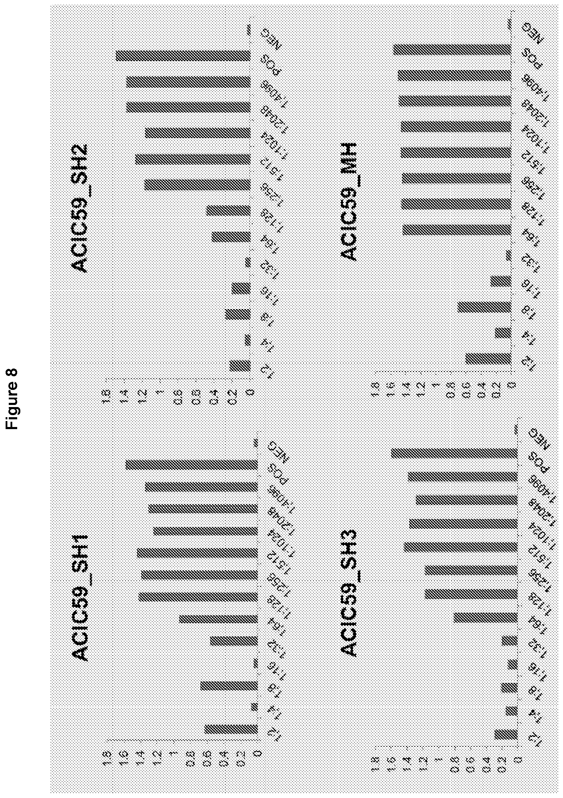

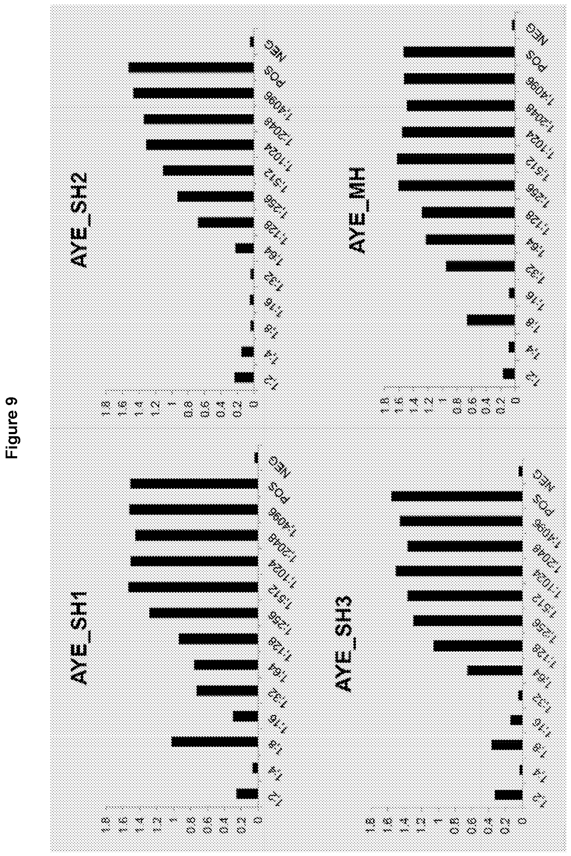

The results are shown in Figures 8 and 9.

In the Figures, the y-axis represents the OD of the broth and, therefore, the amount of seeding. As expected, a large amount of biofilm seeding was observed with each positive control, and minimal background turbidity was observed with each negative control. Biofilms were present on all of the pegs. The results allow determination of the Minimum Biofilm Eradication Concentration (MBEC) of Surgihoney.

**SH1:** Reduced seeding was observed as low as the 1:16 dilution of SH1 for both isolates. Some turbidity was observed at the 1:2 dilution. It is believed that this may have been an artefact due to some of these test wells being in the corner of the plate and, therefore, being more difficult to wash. Some turbidity was also observed at the 1:8 dilution. Again, this is believed to have been an artefact, possibly because these test wells were next to the positive control on the plate.

**SH2:** Reduced seeding was observed for the 1:2 to 1:32/1:64 dilutions for both isolates. As for SH1, some turbidity was observed at the 1:2 dilution. This may have been due to some of these test wells being in the corner of the plate and, therefore, being more difficult to wash.

**SH3:** Reduced seeding was observed for the 1:2 to 1:32/1:64 dilutions for both isolates. As for SH1 and SH2, some turbidity was observed at the 1:2 dilution. This may have been due to some of these test wells being in the corner of the plate and, therefore, being more difficult to wash.

**MH:** Reduced seeding was observed for the 1:2 to 1:16/1:32 dilutions.

### Conclusions

The results confirm that Surgihoney prevented or reduced the seeding of pre-formed biofilms. The SH2 and SH3 preparations of Surgihoney were able to prevent or reduce biofilm seeding at lower concentrations than the SH1 preparation, although there appeared to be little difference in potency between the SH2 and SH3 preparations. The SH2 and SH3 preparations appeared to be slightly more potent than Manuka honey in reducing biofilm seeding.

### Example 4

### Prevention of biofilm formation by Surgihoney compared with commercially available wound dressings

This example describes the effect of Surgihoney SH1, SH2, and SH3 preparations on prevention of biofilm formation by *Pseudomonas aeruginosa* (control strain PA01), and *Acinetobacter baumannii* (control strain AYE), in comparison with deactivated Surgihoney (DE), Manuka honey (MH: 'Comvita Manukacare 18+'), acetic acid (AA), and several commercially available wound dressings, and wound creams.

### Methods

Overnight cultures of the isolates were diluted in Muller-hinton broth to an OD600 of 0.1.

Surgihoney SH1, SH2, and SH3, deactivated Surgihoney (DE), and Manuka honey (MH: 'Comvita Manukacare 18+'), were tested at serial dilutions of 1:2 - 1:256 (using water as diluent). 1 ml honey was placed in each well with 1ml diluted isolate culture.

1cm² of each of the following commercially available dressings was added to 1ml water and 1ml diluted isolate culture: Mepilex Ag; Urgotul Ag; Acticoat (Ag); Urgotul (no AM agent); Mesitran Net (honey-based dressing); Polymem (no AM agent).

Commercially available creams Trimovate and Flamazine (used for treatment of skin infections) were also tested. Acetic acid was also tested from 5% down to 0.04%.

The plate was incubated for 72 hours at 33°C (wound temperature) to encourage biofilm formation. The plate was then developed using crystal violet dye, and visualised following solubilisation of the dye using 70% ethanol.

A spectrophotometer was then used to assess the optical density (OD) of the solubilised dye. The OD reading corresponds to the amount of incorporated crystal violet. Higher OD readings represent dark wells, and greater mass of biofilm. The OD readings were then plotted to make a graph.

### Results

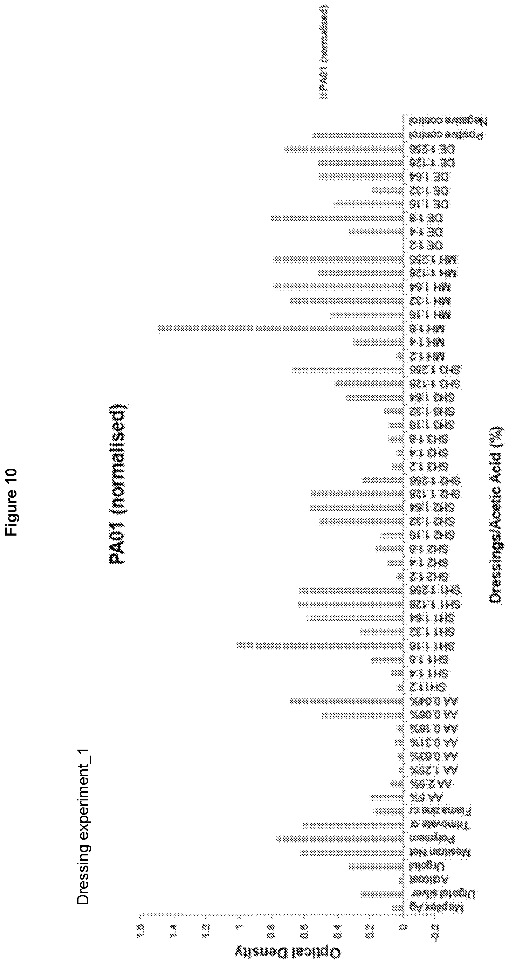

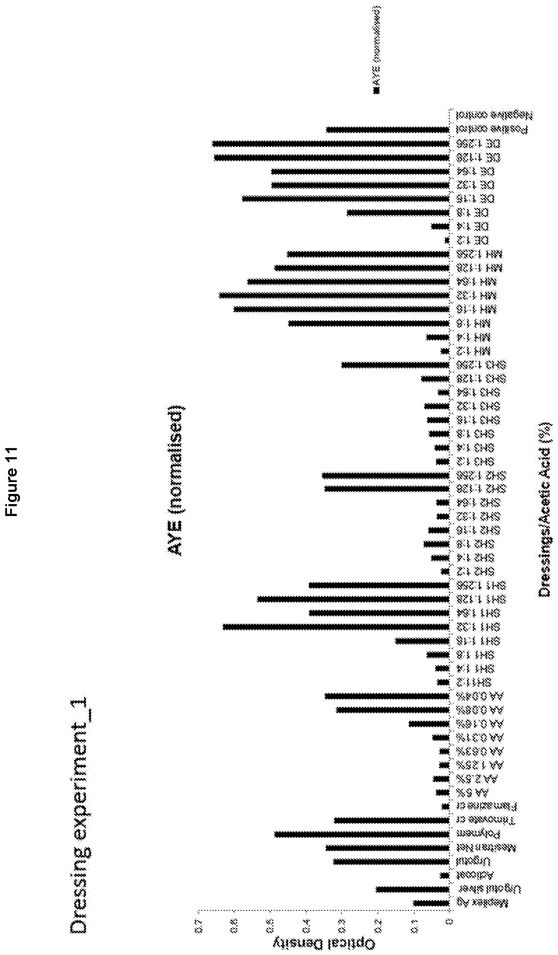

The results are shown in Figures 10 and 11.

The results show that Acticoat and Mepilex Ag dressings (and to a lesser extent, Urgotel silver) were effective at preventing biofilm formation of both strains, as was the Flamazine cream. The Urgotul and Polymem dressings, and Mesitran net (a commercial honey-based dressing), and the Trimovate cream did not appear to be effective in preventing biofilm formation.

Acetic acid was effective at preventing biofilm formation down to 0.31% (AYE) and 0.1% (PA01).

All of the Surgihoneys tested were effective at preventing biofilm formation of both strains at 1:2 and 1:4 dilution, at least, and some at lower concentrations (for example, SH2 was effective against AYE at 1:64 dilution).

The SH2 and SH3 Surgihoneys were more effective in preventing biofilm formation at lower concentrations than SH1 Surgihoney, and each Surgihoney preparation was more effective at lower concentrations than Manuka honey (see, for example, the effect of the 1:8 dilution for each). Manuka honey was effective in preventing biofilm formation down to 1:2 to 1:4 dilution.

The results also show that the deactivated Surgihoney (DE) was effective in preventing biofilm formation. However, when the DE honey was tested for hydrogen peroxide activity, it was found to retain some hydrogen peroxide activity, and so did not appear to have been fully inactivated.

### Conclusions

It was concluded from these results that Surgihoney was comparable in preventing biofilm formation to several commercially available wound dressings, and more effective than Mesitran net (a commercial honey-based dressing).

In the above examples, four isolates of *A. baumanii,* and three of *P. aeruginosa* were tested and Surgihoney was able to prevent biofilm formation of all isolates in a dose-dependent manner. Pre-formed biofilms of *A. baumannii* were additionally exposed to all SH formulations for 24 hours. Reduced seeding of the biofilms was observed for both strains tested and was again dose-dependent.

The dressings experiment (Example 4) revealed SH to be equally or more effective in biofilm prevention than three of the eight commercial creams and dressings tested. Furthermore, in this *in vitro* test, SH was more effective than L-Mesitran net at preventing biofilm formation of single isolates of *A*. *baumanii* and *P. aeruginosa.*

These results show that Surgihoney has potent anti-biofilming activity against key Gram-negative pathogens of burn wounds, and superior activity to the majority of commercial dressings tested. This composition can be used in place of antiboiotics, and in an era of increasing antibiotic resistance, help to reduce inappropriate antibiotic use.

### Example 5

### Inhibition of biofilm formation by Surgihoney compared with Manuka Honey

This example describes an experiment to test the effect of Surgihoney1 (SH1) and Manuka Honey (MH) on biofilm formation by multi-drug resistant (MDR) biofilm-producing isolates of *Pseudomonas aeruginosa* (VIM positive) and CRE *Klebsiella pneumoniae,* and two different biofilm-producing isolates of *Acinetobacter baumanii.*

### Methods

The isolates were cultured with different concentrations of SH1 or MH, and MBIC calculations were made, by similar methods to those described in Example 2.

### Results

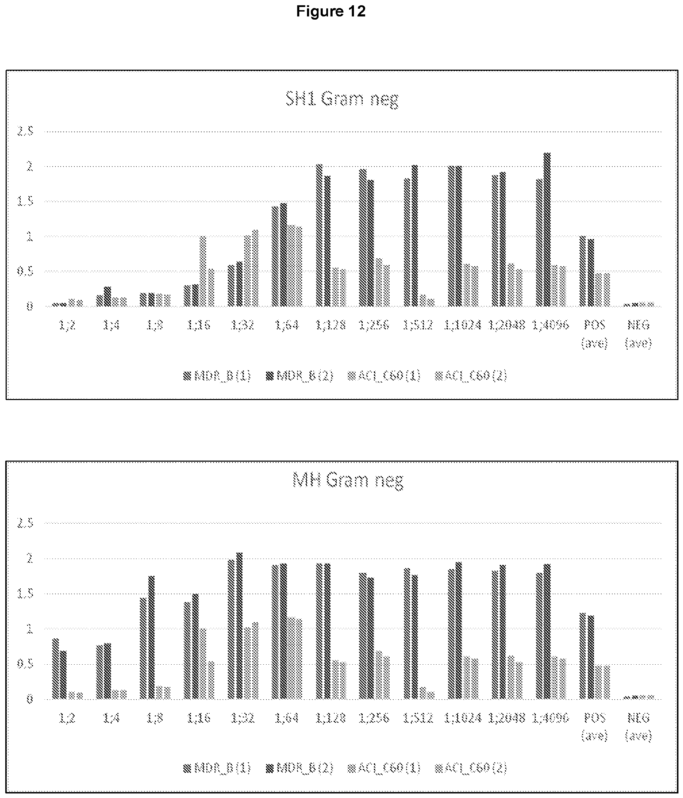

The results are shown in Figure 12. The results show that SH1 was able to prevent each of the isolates from forming a biofilm. SH1 was able to prevent biofilm formation of MDR biofilm-producing isolates of *Pseudomonas aeruginosa* (VIM positive) and CRE *Klebsiella pneumonia* at lower concentrations than MH.

### Example 6

### Surgihoney treatment of CRS-associated S. aureus planktonic populations

The minimum inhibitory concentration (MIC) of Surgihoney for different isolates of CRS-associated *S. aureus* planktonic populations was determined using a 96-well plate-based absorbance assay. The isolates tested were one Methicillin-resistant *Staphylococcus aureus* (MRSA) isolate, and four Methicillin-sensitive *Staphylococcus aureus* (MSSA) isolates - 3 polyp isolates, and 1 mucosal isolate. The Surgihoney concentrations tested were 180 g/L, 225, 270, 315, 360, 405, 450, 495, 540, 720, and 900 g/L Surgihoney. Absorbance at OD₅₉₅ was measured after 18 hours.

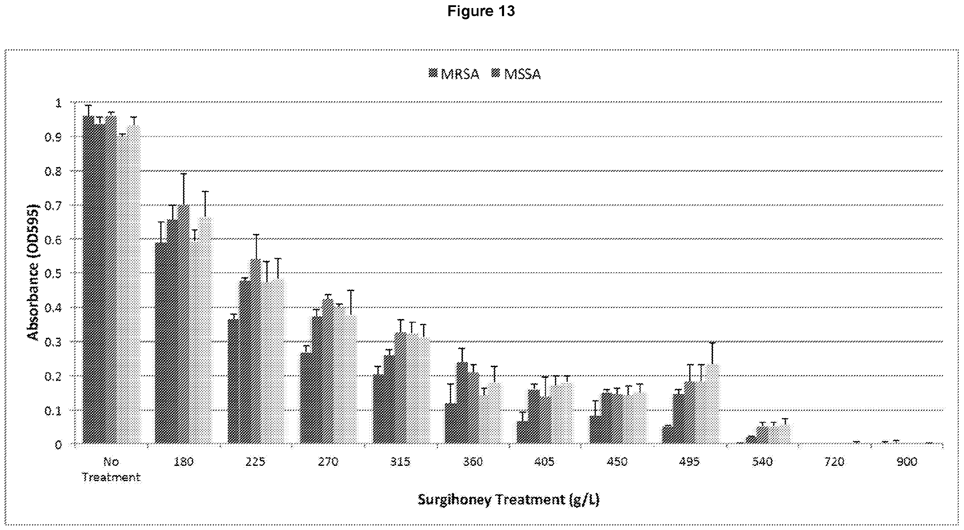

The results are shown in Figure 13. The results show that the MIC of Surgihoney for planktonic MRSA was 540 g/L, and the MIC of Surgihoney for planktonic MSSA was 720 g/L.

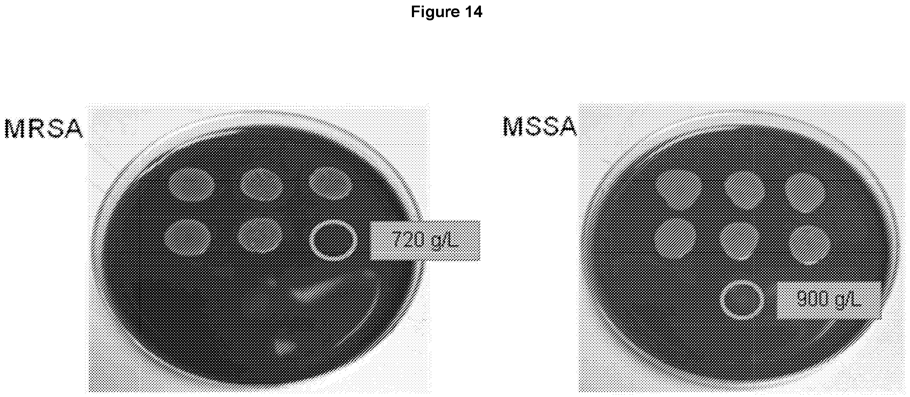

MRSA and MSSA isolates were subcultured onto chocolate blood agar plates with effective concentrations of Surgihoney (360, 405, 450, 495, 540, 720, 900 g/L) to determine the minimum bactericidal concentration (MBC) of Surgihoney for MRSA and MSSA. Photographs of the plates are shown in Figure 14. The photographs show that the MBC of Surgihoney for planktonic MRSA was 720 g/L, and the MBC of Surgihoney for planktonic MSSA was 900 g/L.

### Example 7

### Surgihoney treatment of CRS-associated S. aureus biofilms

An MRSA isolate was grown to mid-exponential phase by static culture in a polystyrene 6-well plate. The 6-well plate was inoculated and supplemented with nutritionally weaker media, and cultured for 48 hours (with fresh media exchange after 24 hours). Biofilms were washed to remove planktonic population, and treated with 500 g/L and 1,000 g/L Surgihoney for 18 hours. The treatment and planktonic population was removed, and the biofilms were re-suspended. The absorbance at OD₆₀₀ was measured to determine the overall biomass (comprising cellular and extracellular material). The biofilms were cultured onto chocolate blood agar plates for colony forming unit enumeration to determine the percentage of viable cells remaining in the biofilm after treatment.

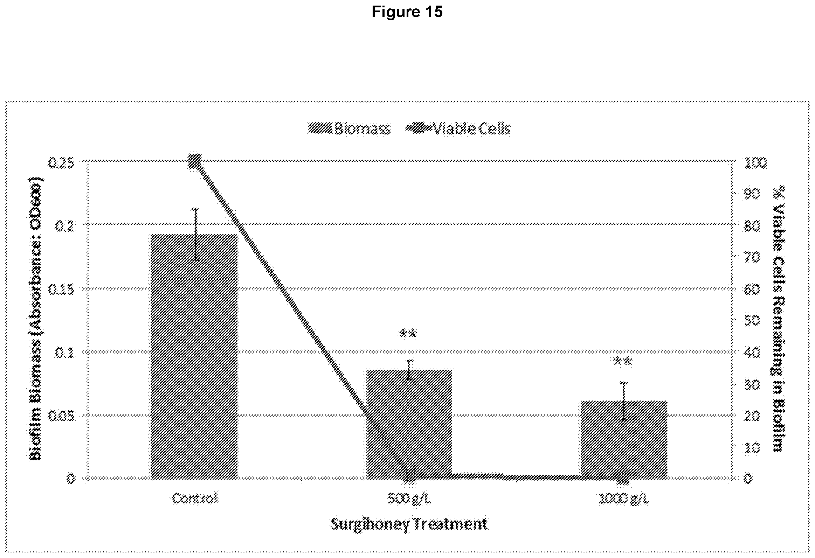

The results are shown in Figure 15. The results show that treatment of a CRS-associated MRSA biofilm with Surgihoney reduced biofilm biomass in a dose-dependent manner, with 500 g/L Surgihoney reducing biomass by approximately one-half, and 100 g/L Surgihoney reducing biomass by approximately two-thirds, compared with the biofilm biomass without treatment. The number of viable cells remaining in the biofilm was also dramatically reduced, with 500 g/L Surgihoney reducing the percentage of viable cells to 0.5%, and 100 g/L Surgihoney reducing the percentage of viable cells to 0.13%.

### Example 8

### Sprayable compositions

A composition containing 1% by weight polyethylene oxide (PEO), 79% by weight deionised water and 20% by weight Surgihoney was added to a pump action spray device.

The spray device was used to spray the composition on to a hydrogen peroxide test strip. The test strip indicated the presence of hydrogen peroxide in the composition.

After five months, the spray was re-tested for its ability to generate hydrogen peroxide, by spraying on to a hydrogen peroxide test strip. The test strip indicated the presence of hydrogen peroxide in the composition.

### Example 9

### Antimicrobial activity of Surgihoney

The antimicrobial activity of Surgihoney (SH) and two prototype modified honeys made by *Apis mellifera* (honeybee) against *Staphylococcus aureus* (NCIMB 9518) was tested. We also examined a number of modified types of Surgihoney for the ability to change the level of production of hydrogen peroxide from the samples.

Methods: Surgihoney (SH) was compared with two modified honeys, Prototype 1 (PT1) and Prototype 2 (PT2) using a bioassay method against a standard strain of *Staphylococcus aureus.* Further work studied the rate of generation of hydrogen peroxide from these preparations.

Results: Surgihoney antimicrobial activity was shown to be largely due to hydrogen peroxide production. By modification of Surgihoney, two more potent honey prototypes were shown to generate between a two- and three-fold greater antibacterial activity and up to ten times greater peroxide activity.

Conclusions: Surgihoney is a wound antiseptic dressing that shows good antimicrobial activity. Two further honey prototypes have been shown to have antimicrobial activity that is possible to be enhanced due to demonstrated increases in peroxide activity.

### Methods

### 1. Determination of Honey Activity by Bioassay Method

The antibacterial activity of Surgihoney (S) and two modified honeys, Prototype 1 (PT1) and Prototype 2 (PT2) was measured using Staphylococcus aureus (NCIMB 9518) and expressed as the equivalent % phenol. Values were calculated of the mean from three sample replicates tested, repeated on three days.

**Assay Method.** The agar well diffusion method used was adapted from the punch plate assay for inhibitory substances described in the Microbiology Standard Methods Manual for the New Zealand Dairy Industry (1982) [Bee Products Standards Council: Proposed standard for measuring the non peroxide activity of honey. In. New Zealand: Bee Products Standards Council; 1982.].

**Inoculum Preparation.** Overnight culture was adjusted to an absorbance of 0.5 measured at 540 nm using sterile nutrient broth as a blank and a diluents and a cuvette with a 1 cm pathway.

**Assay Plate preparation.** A volume of 100 µl of the culture adjusted to 0.5 absorbance was used to seed 150 ml nutrient agar to make the assay plates. The agar was swirled to mix thoroughly and poured into large petri dishes which had been placed on a level surface. As soon as the agar was set the plates were placed upside down overnight before using the next day. For assay these seeded plates were removed from 4°C and allowed to stand at room temperature for 15 min before cutting 7.0 mm diameter wells into the surface of the agar. 250 µl of test material (sample or standard) was placed into each well.

**Catalase solution.** A 200 mg/ml solution of catalase from bovine liver (Sigma C9322, 2900 units/mg) in distilled water was prepared fresh each day.

**Sample preparation.** Primary sample solutions were prepared by adding 4 g of sample to 4 ml of distilled water in universals and placed at 37°C for 30 minutes to aid mixing. To prepare secondary solutions, 2 ml of the primary sample solution was added to 2 ml of distilled water in universals and mixed for total activity testing and 2 ml of the primary sample solution was added to 2 ml of catalase solution and mixed for non-peroxide activity.

**Preparation of phenol standards.** Standards (w/v) 10%, 30%, 50% phenol were prepared by dissolving phenol in water. Phenol standards were brought to room temperature in the dark before use and were mixed thoroughly before addition to test wells. Each standard was placed in three wells to test in triplicate. Standards were kept at 4°C with an expiry date of one month.

**Sample and standard application.** All samples and standards were tested in triplicate by adding 250 µl to each of 3 wells.

**Plate incubation.** After application of samples the plates were incubated for approximately 18 hours at 37 °C. The diameter of inhibition zones, including the diameter of the well (7.0 mm), was recorded.

**Calculation of antibacterial activity of samples.** The mean diameter of the clear zone around each phenol standard was calculated and squared. A standard graph was plotted of % phenol against the square of the mean diameter of the clear zone. A best-fit straight line was obtained using linear regression and the equation of this line was used to calculate the activity of each diluted honey sample from the square of the mean measurement of the diameter of the clear zone. To allow for the dilution (assuming the density of the Surgihoney to be 1.35 g/ml) this figure was multiplied by a factor of 4.69 and the activity of the samples was then expressed as the equivalent phenol concentration (% w/v).

Total Activity: all the activity, including activity due to hydrogen peroxide (H₂O₂).

Non-Peroxide Activity: H₂O₂ is removed by treating samples with catalase enzyme.

### 2. Determination of Honey Activity by H₂O₂ Method

The activity was measured using the *Merckoquant*® *1.10011. & 1.10081.*

**Peroxide Test Kits.** Concentrations expressed as the equivalent mg/L H₂O₂.

Samples were diluted 1:10 with purified water. Following 5 min incubation, all samples were measured for H₂O₂ production each hour over a 12 hour period followed by 24 and 48 hour time points.

**Method of Determination.** Peroxidase transfers oxygen from the peroxide to an organic redox indicator, which is then converted to a blue coloured oxidation product. The peroxide concentration is measured semi-quantitatively by visual comparison of the reaction zone of the test strip with the fields of a colour scale. The reaction zone of the test strip is immersed into the Surgihoney sample for 1 sec, allowing excess liquid to run off the strip onto an absorbent paper towel and after 15 seconds (Cat. No. 110011), 5 seconds (Cat. No. 110081), after which a determination of the colour formed in the reaction zone more precisely coincided with the colour fields scale.

### Results

### 1. Activity Rating

The antimicrobial activity produced by the modification of the honey samples resulted in a twofold and almost three-fold respectively increase in phenol activity with PT1 and PT2 compared with Surgihoney alone. The results for the three samples of Surgihoney (SH) and two modified prototypes, PT1 and PT2 are shown in the Table below.

Table showing the peroxide and non-peroxide antibacterial activities of Surgihoney (SH) and two modified prototypes, PT1 and PT2 against Staphylococcus aureus (NCIMB 9518).

| **Sample Name** | **Batch No.** | **Total Activity (% phenol)** | **Non-Peroxide Activity (% phenol)** |
|---|---|---|---|
| **Surgihoney** | 2015-06-018B | 32 | 0 |
| **Surgihoney PT1** | HHI4110311 | 65 | 7 |
| **Surgihoney PT2** | HHI14110312 | 83 | 10 |

### 2. Determination of Honey Activity by H₂O₂ Method

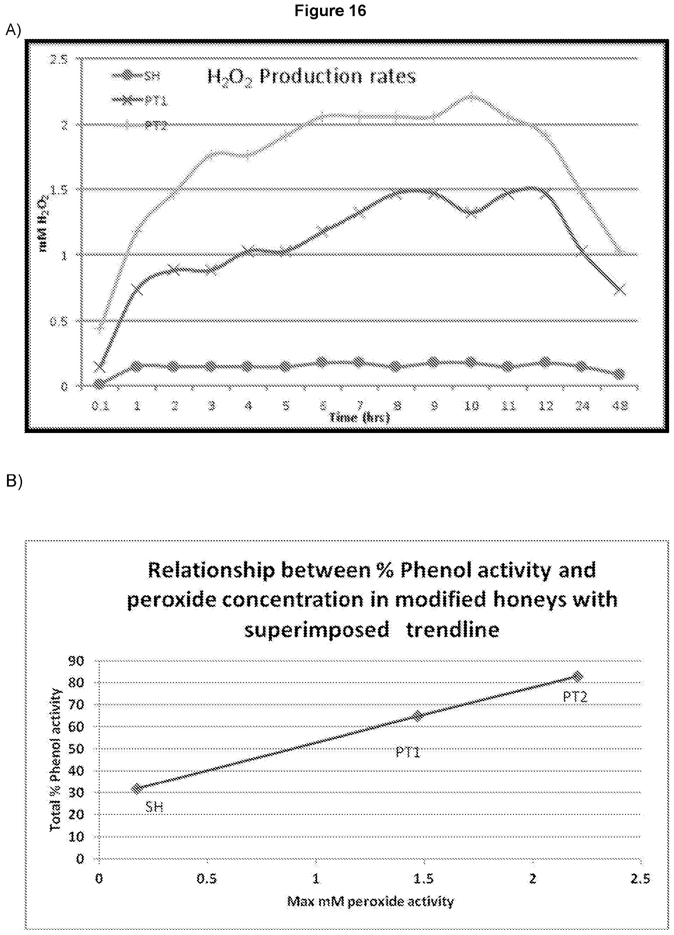

The prototype modifications are observed to generate up to seven and ten times the hydrogen peroxide activity of Surgihoney. The results for the three samples are shown in Figure 16A. By taking the maximum level of hydrogen peroxide output for each of the three honey prototypes and plotting this against the total phenol activity a linear relationship is observed (Figure 16B).

### Discussion

The results from this work show that the main antimicrobial activity of Surgihoney and two modified prototypes, PT1 and PT2 are due to hydrogen peroxide. This is a similar finding to certain other honeys from a variety of floral sources. However, unlike previous work the availability of hydrogen peroxide from the samples is able to be enhanced and at 12 hours is seven and ten times respectively the value for Surgihoney alone. There is a striking linear relationship between the antimicrobial activity and the maximum output of hydrogen peroxide from the three honey prototypes.

This peroxide activity offers potent antimicrobial activity that is ideally suited for a wound dressing that is applied to acute or chronic wounds to treat or prevent wound infections. Whilst a small amount of catalase is present in wounds and serum level of catalase in males has been reported as 50 kU/l it has been shown that catalase activity in healing wounds actually decrease during the first week post-wounding and activity levels of catalase recover to its original level at two weeks post-wounding. Such concentrations of catalase are thus extremely unlikely to influence the antimicrobial activity observed with exogenously applied Surgihoney or the two modified prototypes, PT1 and PT2.

The ideal characteristics for an antimicrobial wound dressing are: effectiveness, lack of toxicity, ease of use, patient and clinician acceptability and value for money. Hydrogen peroxide is an effective antimicrobial and is already used as a biocide for its potent activity against vegetative bacteria, yeasts and spores. It produces its antimicrobial effect through chemical oxidation of cellular components.

The human toxicity of hydrogen peroxide is concentration dependent and one study has claimed that the differential concentrations for antimicrobial and human toxicity might overlap. By contrast, certain preparations of honey have been shown to be an effective antimicrobial agent by supplying low concentrations of hydrogen peroxide to wounds continuously over time rather than as a large amount at the time of dressing and without such toxicity. Indeed there is compelling evidence that where physiological levels of hydrogen peroxide are applied to mammalian cells there is a stimulation of biological responses and activation of specific biochemical pathways in these cells.

Clearly Surgihoney and the two modified prototypes, PT1 and PT2 are antimicrobial dressings that offer effective hydrogen peroxide release over at least 24 hours.

### Conclusions

Surgihoney and the two modified prototypes, PT1 and PT2 have been shown to have potent antimicrobial activity against a standard strain of *Staphylococcus aureus.* These antimicrobial activities have been shown to be due to hydrogen peroxide. The activity is scalable and can be described in terms of hydrogen peroxide activity. These modified honeys offer a dressing that is effective, non-toxic and easy to administer.

### Example 10

### Nebulised Surgihoney

This example describes nebulisation of Surgihoney, and the antimicrobial effect of nebulised Surgihoney.

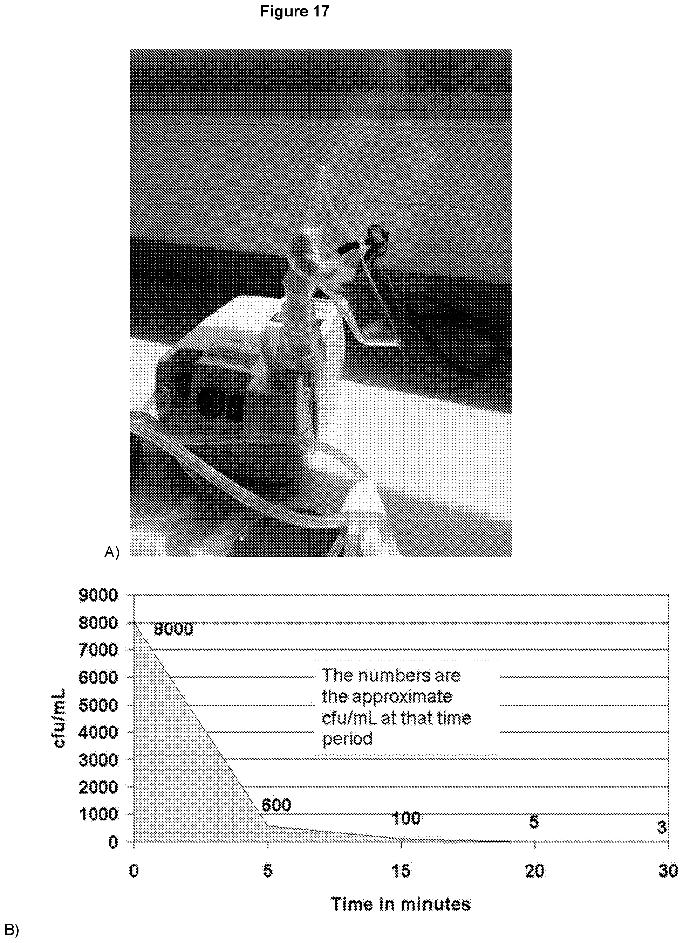

One 10g sachet of SH1 Surgihoney was dissolved in 4ml sterile 0.9% saline solution and warmed in a water bath to liquefy the honey. Thus, the liquefied solution contained 250% SH1 Surgihoney. The liquefied honey was placed in the medicine reservoir of a nebuliser face mask tub. This was connected to a standard Salter respiratory nebuliser and switched on. Nebulised vapour smelling and tasting of honey was produced by the nebuliser (see Figure 17A). A volunteer inhaled the nebulised Surgihoney, with no ill effects.

The antimicrobial effect of the nebulised Surgihoney was assessed against a strain of *Staphylococcus aureus.* Absorbent paper discs were inoculated with *S. aureus* prepared to a concentration of 10³-10⁴ cfu/ml. The discs were placed in a tube (simulating the bronchus). Nebulised Surgihoney was passed over the paper discs for 5, 15, 20, or 30 minutes. The discs were then cultured on blood agar plates to determine the number of surviving colony forming units. A control disc inoculated with *S. aureus* that was not contacted with nebulised Surgihoney was also cultured.

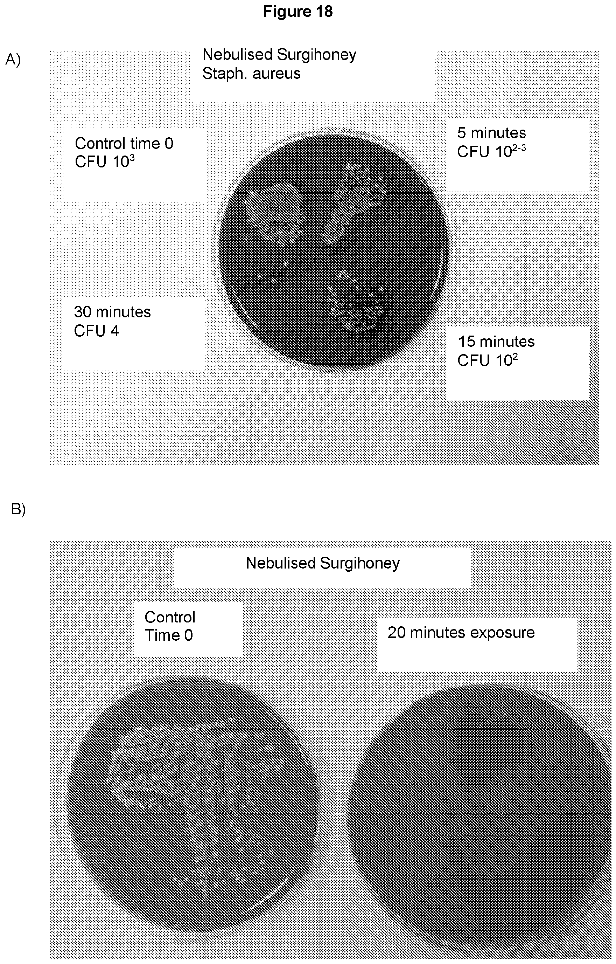

Photographs of the blood agar plates are shown in Figure 18. The number of colony forming units (cfu/ml) for each plate is shown in the plot in Figure 17B, and the approximate number of cfu/ml after each exposure time is recorded in the Table below.

| **Time (minutes)** | **Approximate number of cfu/ml** |
|---|---|
| 0 | 8000 |
| 5 | 600 |
| 15 | 100 |
| 20 | 5 |
| 30 | 3 |

It was concluded that Surgihoney can readily be nebulised. Nebulised Surgihoney appears to be safe to inhale, with no observed adverse effects in one volunteer. The nebulised Surgihoney has significant antimicrobial activity, reducing the bacterial load by 1000 fold in a simulated respiratory tract model.

### Effectiveness of nebulised Surgihoney in vivo, for the treatment of respiratory disease

A patient with severe bronchiectasis was colonised in the respiratory tract with *Mycobacterium avium* and *Mycobacterium abscessus.* The patient was treated by administering nebulised SH1 Surgihoney (prepared as described above), daily, for two months. The treatment cleared the *Mycobacterium* in the patient's respiratory tract as indicated by analysing the patient's sputum samples.

### Example 11

### Surgihoney treatment of CRS-associated S. aureus biofilms

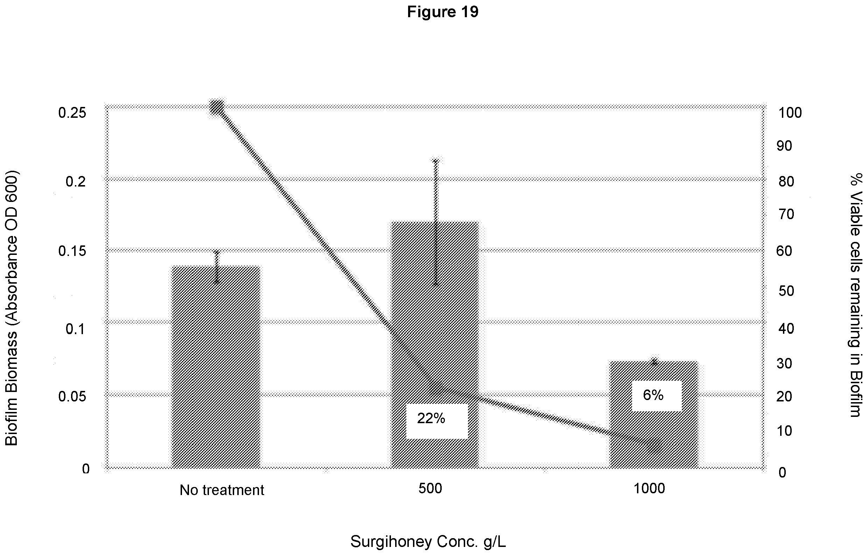

MSSA static biofilms were grown *in vitro,* and then treated with Surgihoney, as described in Example 7 above. The results are shown in Figure 19. The results show that, although treatment of a CRS-associated MSSA biofilm with 500 g/L Surgihoney did not appear to reduce biofilm biomass, treatment with 1000 g/L Surgihoney reduced biofilm biomass by approximately one-half, compared with the biofilm biomass without treatment. The number of viable cells remaining in the biofilm was also dramatically reduced, with 500 g/L Surgihoney reducing the percentage of viable cells by 78% to 22%, and 100 g/L Surgihoney reducing the percentage of viable cells by 94% to 6%.

The results of Examples 6, 7, and 11 demonstrate that Surgihoney has potent bactericidal and inhibitory effects on MRSA and MSSA in both planktonic and biofilm forms.

### Example 12

### Surgihoney treatment of ischaemic ulcer chronically infected with Pseudomonas aeruginosa

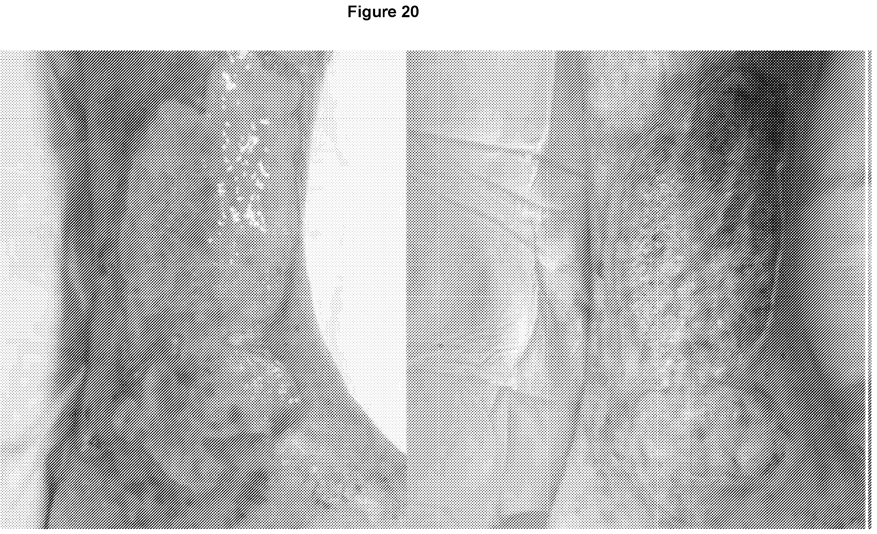

A 77 year-old male with peripheral vascular disease had developed long-standing ischaemic ulcers chronically infected with *Pseudomonas aeruginosa* (see Figure 20(A)). After a 7 day treatment with Surgihoney, a significant clinical improvement was seen (Figure 20 (B)).

### Example 13

### Use of Surgihoney as a topical anti-MRSA treatment

The World Health Organisation's (WHO) 2014 report on global surveillance of antimicrobial resistance has revealed that the world has reached a critical point. 1 in 5 of hospital-acquired infections are now attributed to Methicillin-resistant *Staphylococcus aureus* (MRSA). The UK Department of Health's annual report (2014 to 2015) of MRSA bacteraemia reported 874 community-acquired cases, and 349 hospital-acquired cases. The effect on the national health service (NHS) is significant because it causes a delay of elective surgery, prolonged hospital stay, and long-term antibiotic treatment.

Mupirocin (Bactroban) is a topical antibacterial treatment effective against Gram-positive bacteria, including MRSA, currently used as part of the decolonisation regime. However, recent evidence indicates increasing resistance of MRSA to Mupirocin.

### AIMS

1. To compare the efficacy of Surgihoney versus Mupirocin on MRSA isolates in the *in vitro* setting.
2. To conduct a small scale proof-of-principle clinical study examining the feasibility of using Surgihoney as a novel MRSA decolonisation therapy in MRSA-positive clinical subjects.

### METHODOLOGY

### Recruitment and phenotyping of clinical subjects:

Patients identified as MRSA carriers as a result of pre-assessment screening will be recruited into the study.

### Specimen acquisition, isolation of MRSA and in vitro bacterial analysis:

The antimicrobial activity of Surgihoney will be assessed *in vitro* on both planktonic and biofilm MRSA phenotypes. The bactericidal and inhibitory function of Surgihoney will be compared to current standard therapy (Mupirocin).

### Proof of principle clinical study:

A small scale proof of principle clinical study will be conducted to assess the feasibility of using Surgihoney as a novel MRSA decolonisation therapy in the nasal cavity.

The results from this study will allow use of Surgihoney as a topical therapy in MRSA nasal carriers and surgical patients with MRSA-infected wounds.

### Example 14

### Clinical response of 114 chronic wounds to treatment with Surgihoney

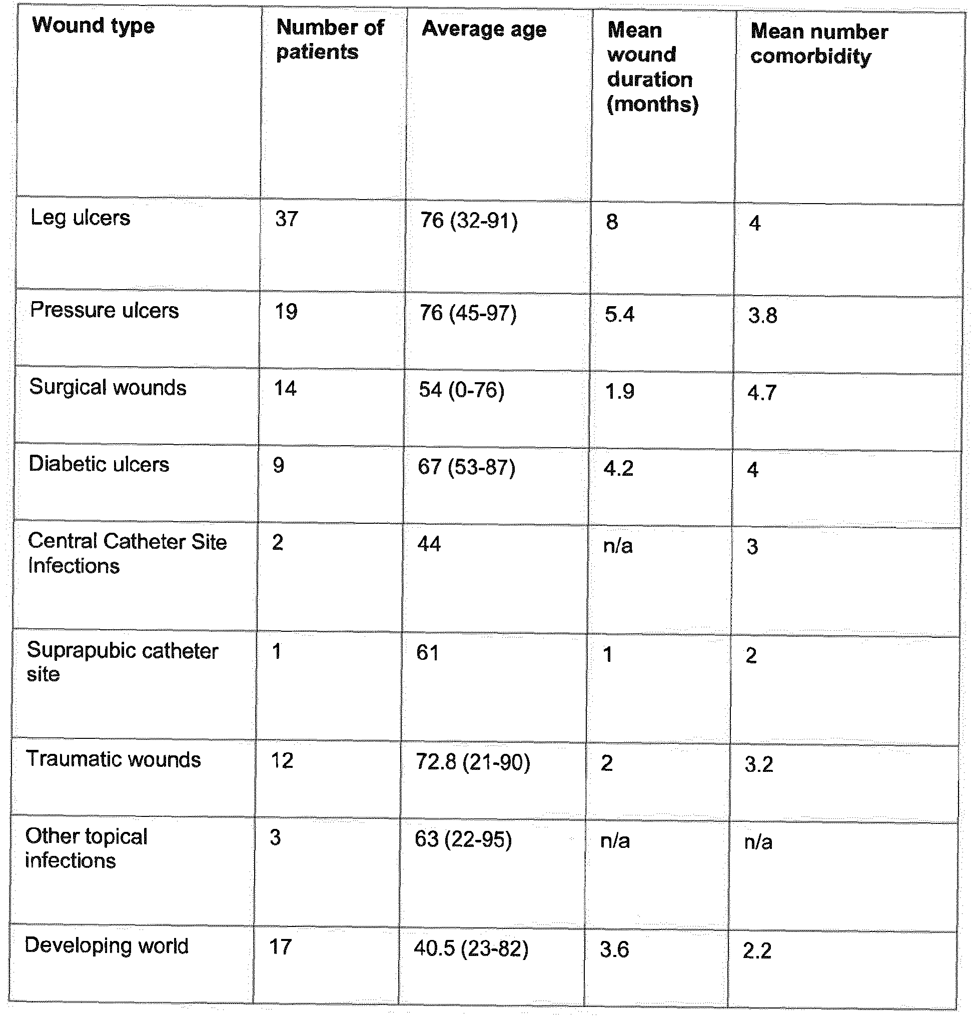

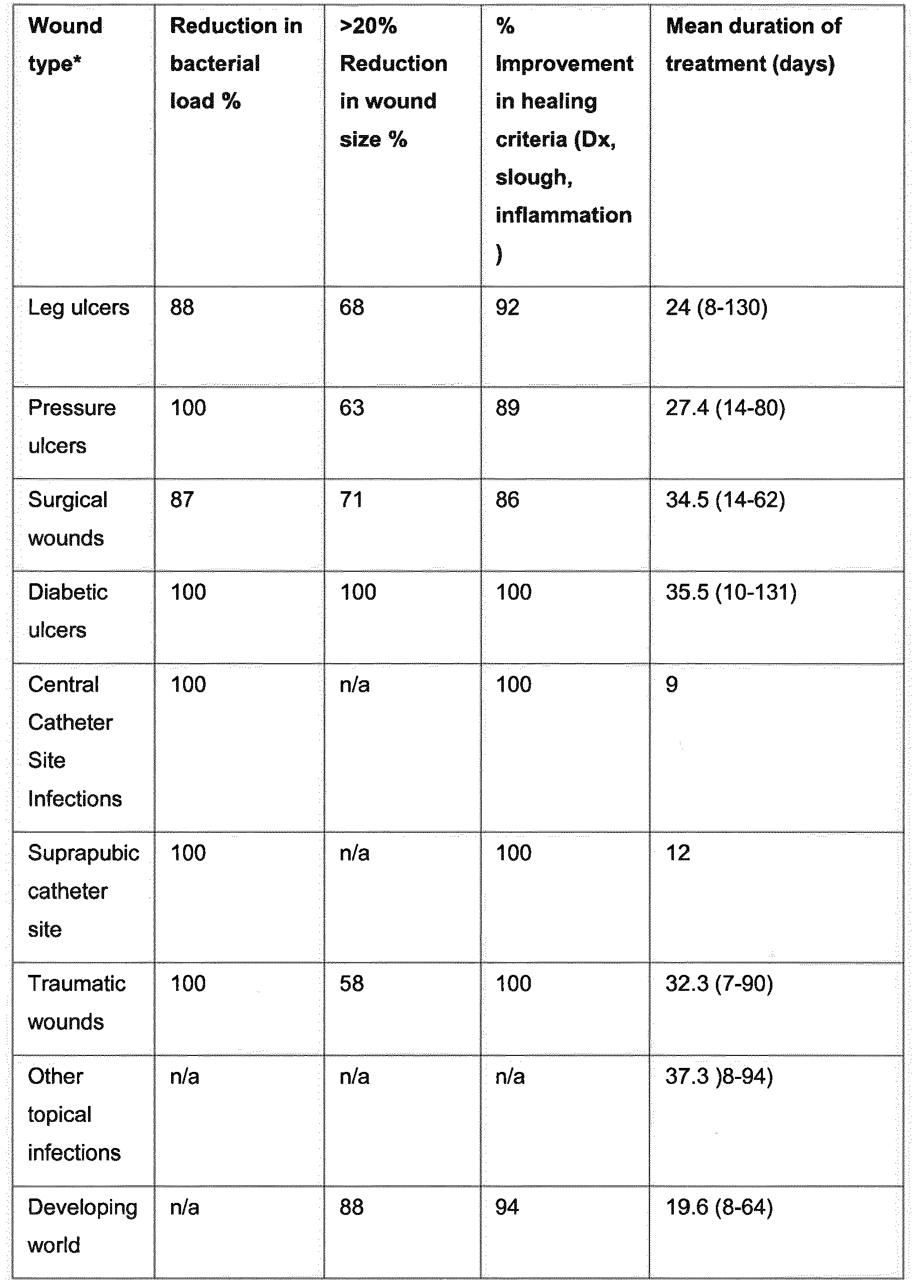

### Example 15

### Paediatric MRSA wound infection

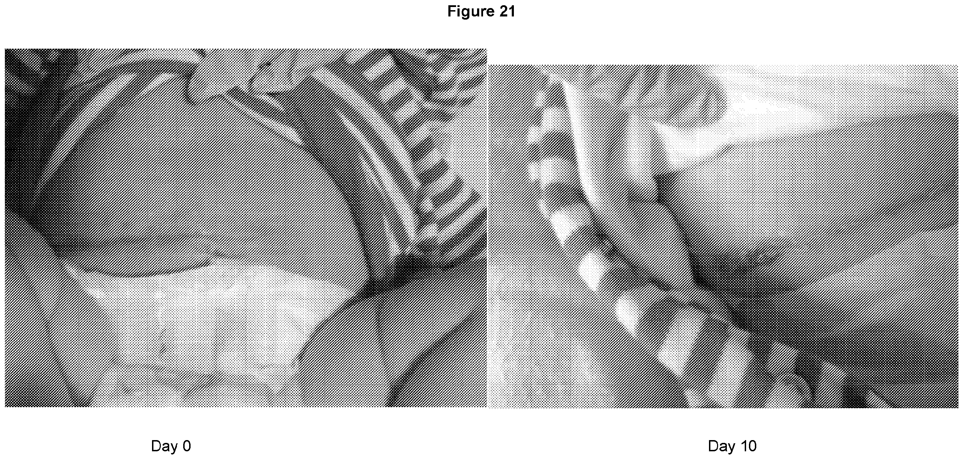

Figure 21 shows the effects of treating a MRSA wound infection with Surgihoney over 10 days.

### Example 16

### CA MRSA superficial infection

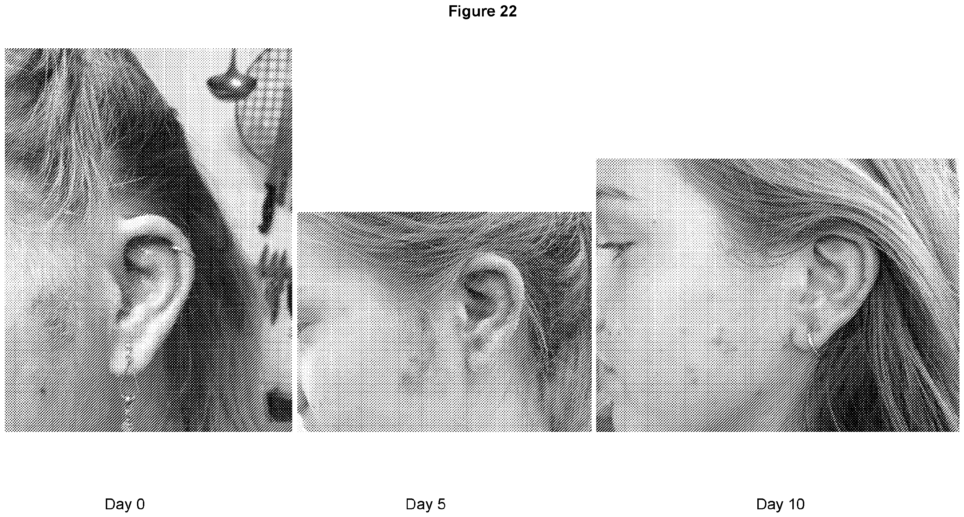

Figure 22 shows the effect of treating a CA MRSA infection with Surgihoney, over 5 days and over 10 days.

### Example 17

### Anti-viral activity of Surgihoney

SH1 or SH2 Surgihoney was mixed with Herpes Simplex Virus (HSV) (50□g honey and 50□l virus) and incubated for 1 hour at 37°C. A dilution series (10⁻², 10⁻³, 10⁻⁴, 10⁻⁵) was then made from the mixture, and the dilutions were used in a plaque reduction assay. Controls with no honey, or with control honey were also performed. The number of viral plaques formed for each dilution was recorded. The results are shown in the Table below.

| | | **Experiment 1** | | | **Experiment 2** | | |
|---|---|---|---|---|---|---|---|
| **Honey** | **Dilution** | **well 1** | **well 2** | **well 3** | **well 1** | **well 2** | **well 3** |
| **SH1** | -2 | * | * | * | * | * | * |
| | -3 | 1 | 1 | 5 | 0 | 0 | 0 |
| | -4 | 0 | 1 | 1 | 0 | 0 | 0 |
| | -5 | 0 | 0 | 0 | 0 | 0 | 0 |
| **SH2** | -2 | * | * | * | * | * | * |
| | -3 | 0 | 0 | 0 | 0 | 0 | 0 |
| | -4 | 0 | 0 | 0 | 0 | 0 | 0 |
| | -5 | 0 | 0 | 0 | 0 | 0 | 0 |
| **Control Honey** | -2 | 100 | 95 | 88 | 108 | 128 | 106 |
| | -3 | 13 | 15 | 11 | 14 | 12 | 15 |
| | -4 | 2 | 1 | 2 | 3 | 2 | 2 |
| | -5 | 0 | 0 | 0 | 0 | 0 | 1 |
| **No Honey** | -2 | | | | 160 | 158 | 164 |
| | -3 | | | | 28 | 22 | 18 |
| | -4 | | | | 6 | 4 | 1 |
| | -5 | | | | 1 | 0 | 1 |

The results show that SH1 and SH2 Surgihoney was strongly virucidal against HSV in both experiments.

### Example 18

### Cytotoxic activity of Surgihoney

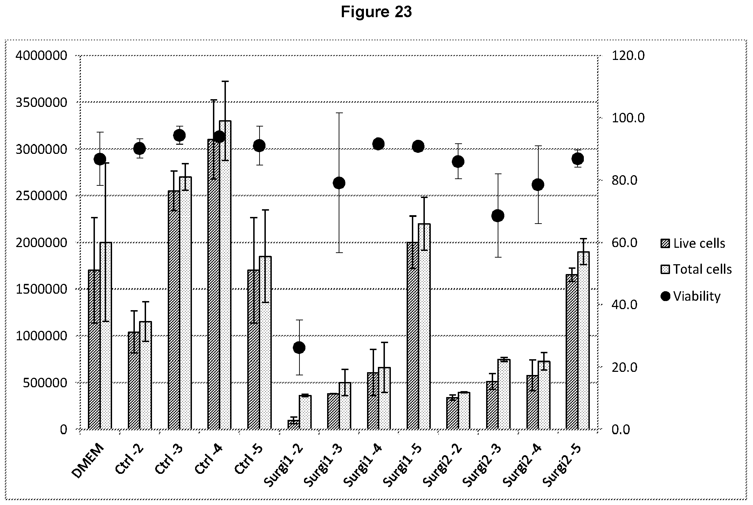

SH1 or SH2 Surgihoney (50µg honey diluted 10⁻², 10⁻³, 10⁻⁴, 10⁻⁵) was incubated on cells for 2 days. The number of live cells, and the total number of cells was counted (percentage viability = live/total x 100). The results are shown in the table below, and in Figure 23.

The results show that SH1 Surgihoney was cytotoxic at the 10⁻² dilution, and cytostatic at the 10⁻³ and 10⁻⁴ dilutions, and that SH2 Surgihoney was cytostatic at the 10⁻², 10⁻³ and 10⁻⁴ dilutions. SH1 and SH2 Surgihoney were not cytotoxic or cytostatic at the 10⁻⁵ dilution.

It is concluded from the results in Examples 17 and 18 that Surgihoney can be administered at doses which are virucidal but not cytotoxic or cytostatic.

| | | **Number of live cells** | | | | **Total number of cells** | | | | **Percentage viability** | | | |
|---|---|---|---|---|---|---|---|---|---|---|---|---|---|
| **Condition** | **Dilution** | **rep1** | **rep2** | **Ave** | **standard deviation** | **rep1** | **rep2** | **Ave** | **standard deviation** | **rep1** | **rep2** | **Ave** | **standard deviation** |
| **DMEM** | - | 1300000 | 2100000 | 1700000 | 565685.4 | 1400000 | 2600000 | 2000000 | 848528.1 | 92.9 | 80.8 | 86.8 | 8.5 |
| **Control honey** | **-2** | 1200000 | 880000 | 1040000 | 226274.2 | 1300000 | 1000000 | 1150000 | 212132 | 92.3 | 88.0 | 90.2 | 3.0 |
| | **-3** | 2700000 | 2400000 | 2550000 | 212132 | 2800000 | 2600000 | 2700000 | 141421.4 | 96.4 | 92.3 | 94.4 | 2.9 |
| | **-4** | 3400000 | 2800000 | 3100000 | 424264.1 | 3600000 | 3000000 | 3300000 | 424264.1 | 94.4 | 93.3 | 93.9 | 0.8 |
| | **-5** | 2100000 | 1300000 | 1700000 | 565685.4 | 2200000 | 1500000 | 1850000 | 494974.7 | 95.5 | 86.7 | 91.1 | 6.2 |
| **SH1** | **-2** | 120000 | 70000 | 95000 | 35355.34 | 370000 | 350000 | 360000 | 14142.14 | 32.4 | 20.0 | 26.2 | 8.8 |
| | **-3** | 380000 | 380000 | 380000 | 0 | 400000 | 600000 | 500000 | 141421.4 | 95.0 | 63.3 | 79.2 | 22.4 |
| | **-4** | 430000 | 780000 | 605000 | 247487.4 | 470000 | 850000 | 660000 | 268700.6 | 91.5 | 91.8 | 91.6 | 0.2 |
| | **-5** | 1800000 | 2200000 | 2000000 | 282842.7 | 2000000 | 2400000 | 2200000 | 282842.7 | 90.0 | 91.7 | 90.8 | 1.2 |
| **SH2** | **-2** | 320000 | 360000 | 340000 | 28284.27 | 390000 | 400000 | 395000 | 7071.068 | 82.1 | 90.0 | 86.0 | 5.6 |
| | **-3** | 450000 | 570000 | 510000 | 84852.81 | 760000 | 730000 | 745000 | 21213.2 | 59.2 | 78.1 | 68.6 | 13.3 |
| | **-4** | 460000 | 690000 | 575000 | 162634.6 | 660000 | 790000 | 725000 | 91923.88 | 69.7 | 87.3 | 78.5 | 12.5 |
| | **-5** | 1600000 | 1700000 | 1650000 | 70710.68 | 1800000 | 2000000 | 1900000 | 141421.4 | 88.9 | 85.0 | 86.9 | 2.7 |

### Example 19

### Dried Surgihoney

Dried honey granules (K24289) were supplied by Kanegrade Limited (Stevenage, UK). The dried honey granules were produced by vacuum drying and contained honey solids with skimmed milk powder. The water content was less than 3%.

The dried honey granules were activated by adding glucose oxidase at SH1 and SH2 levels (see above).

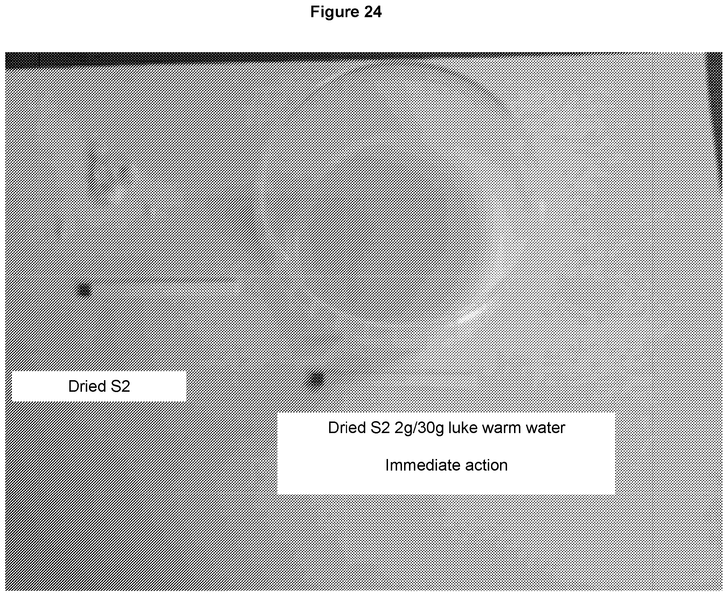

Figure 24 (left) demonstrates that adding water to the activated dried honey granules led to the immediate production of hydrogen peroxide at between 50 and 100 ppm, as detected by a hydrogen peroxide indicator strip.

Figure 24 (right) demonstrates the result of adding 2g of the activated dried honey granules to 30g of sterile warm water (at approximately 35°C). The activated honey granules dissolved easily with no scum formation. Even after four days, there was no settling out, as the granules remained fully dissolved.

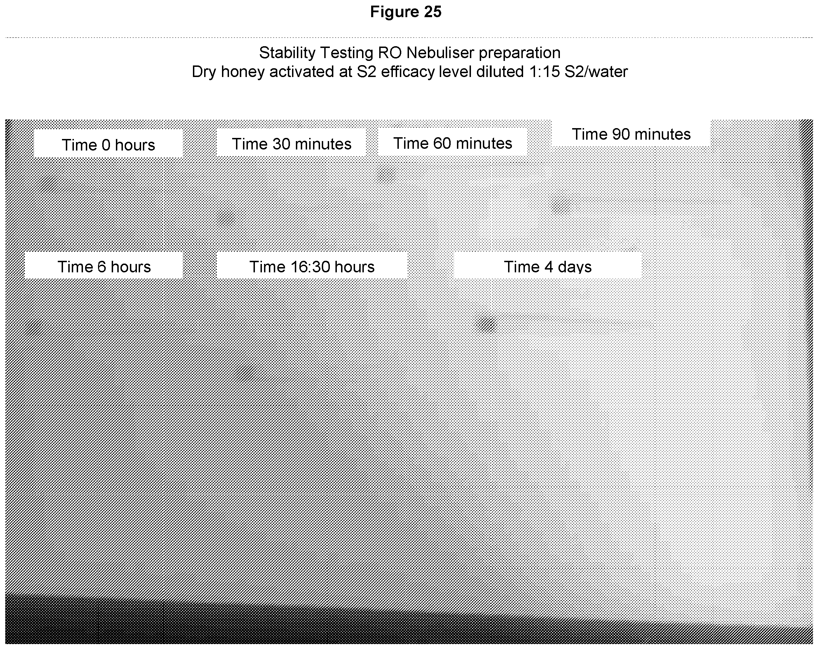

Figure 25 demonstrates that the activity of the dissolved active honey was retained over an extended period of time. Top row (left to right): 0 hours; 30 mins; 60 mins; 90 mins. Bottom row (left to right): 6 hours, 16.5 hours; 4 days. After four days, the level of hydrogen peroxide remained constant at between 50 and 100 ppm. This shows that the glucose oxidase remains active and there was sufficient glucose present to produce hydrogen peroxide over an extended period.

### Example 20

### Stability of aqueous Surgihoney compositions

A sample was prepared with the following composition, on 9 November 2014:
SH2 Surgihoney: 20 % by weight
PVA: 5 % by weight
Water: 75 % by weight

The sample was tested on 16 October 2015 using a hydrogen peroxide test strip and was found to still be producing hydrogen peroxide at a level of between 30-50 ppm.

A sample was prepared by mixing SH2 Surgihoney with water in a ratio of 1:15 (Surgihoney:water, by weight), on 14 September 2015. The sample was tested on 16 October 2015 using a hydrogen peroxide test strip and was found to still be producing hydrogen peroxide at a level of 30-50 ppm.

### Example 21

### Use of Surgihoney for the topical treatment of lower genital tract infections

Surgihoney has been used to treat persistent vaginal discharge that had not been responsive to standard therapy. The indications were vaginal discharge with various aetiologies e.g. bacterial vaginosis and general bacterial vaginal discharge.

Tampons coated with Surgihoney were inserted into the vagina and were replaced every 24 hours. The therapeutic outcome was good and there were no reported adverse effects.

### Example 22

### Use of Surgihoney to treat CPE

A patient developed a soft tissue infection of the foot whilst in India and was diagnosed with necrotising fasciitis. This required extensive debridement of dead tissue as well as antibiotics. Post-operatively, the patient was found to be colonised in the debrided wounds with CPE (Carbapenemase-producing Enterobacteriaceae). The patient required isolation and was treated with Surgihoney, which cleared the organisms.

### Example 23

### Compositions comprising non-aqueous solvent

### Sample 1

Lightweight hydro entangled fabric (40 g per square meter)
Coated with 25% ^{w}/_{w} honey/glycerol solution
Weight of coating on fabric 100 g per square meter
Suggested application: Absorbent fabric in a wound dressing.

### Sample 2

Lightweight hydro entangled fabric (40 g per square meter)
Coated with 75% ^{w}/_{w} honey/glycerol solution
Weight of coating on fabric 100 g per square meter
Suggested application: Absorbent fabric in a wound dressing.

### Sample 3

Lightweight hydro entangled fabric (40 g per square meter)
Coated with 25% ^{w}/_{w} honey/glycerol solution
Weight of coating on fabric 300 g per square meter
Suggested application: Anti bacterial wipe

### Sample 4

Lightweight hydro entangled fabric (40 g per square meter)
Coated with 75% ^{w}/_{w} honey/glycerol solution
Weight of coating on fabric 300 g per square meter
Suggested application: Anti bacterial wipe

### Sample 5

Paper like fabric (42 g per square meter)
Coated with 25% ^{w}/_{w} honey/glycerol solution
Weight of coating on fabric 100 g per square meter
Suggested application: Anti bacterial wipe

### Sample 6

Boots Wound Dressing coated with 25% ^{w}/_{w} honey/glycerol solution
Weight of coating on fabric 400 g per square meter

### Sample 7

Boots Adhesive Wound Dressing with absorbent pad coated with 25% ^{w}/_{w} honey/glycerol solution
Weight of coating on fabric 150 g per square meter

### Sample 8

Lightweight hydro entangled fabric (40 g per square meter)
Coated with 25% ^{w}/_{w} Sugihoney/glycerol solution
Weight of coating on fabric 100 g per square meter
Suggested application: Absorbent fabric in a wound dressing.

### Sample 9

Light weight hydro entangled fabric (40 g per square meter)
Coated with 25% ^{w}/_{w} Sugihoney/glycerol solution
Weight of coating on fabric 300 g per square meter
Suggested application: Absorbent fabric in a wound dressing.

## Claims

1. A composition for generating anti-microbial activity for use in the treatment of a microbial infection that comprises a biofilm, wherein the composition comprises an enzyme that is able to convert a substrate to release hydrogen peroxide, and a substance that includes a substrate for the enzyme, wherein the composition does not include sufficient free water to allow the enzyme to convert the substrate, wherein the enzyme is additional to any enzyme activity able to convert the substrate to release hydrogen peroxide that may be present in the substance, optionally wherein the composition prevents or inhibits growth or seeding of the biofilm.

2. A composition for use according to claim 1, wherein:
a) the enzyme is a purified enzyme and the substance comprises a purified substrate for the enzyme;
b) the enzyme is a purified enzyme , the substance is an unrefined natural substance that includes a substrate for the enzyme; or
c) the substance is an unrefined natural substance that lacks catalase activity and that includes a substrate for the enzyme.

3. A composition for use according to claim 2, wherein the unrefined natural substance is a honey.

4. A composition for use according to any preceding claim, wherein the enzyme is glucose oxidase and the substrate is D-glucose.

5. A composition for use according to any preceding claim, wherein the composition is a sterile composition.

6. A composition for use according to any preceding claim, wherein the microbial infection is a chronic microbial infection.

7. A composition for use according to any preceding claim, wherein the microbial infection is a wound infection, preferably a chronic wound infection, optionally wherein the wound infection is a skin wound infection or a burn wound infection.

8. A composition for use according to any preceding claim, wherein the biofilm comprises any of the following species of bacteria: *Pseudomonas aeruginosa; Acinetobacter baumannii,* Methicillin-resistant *Staphylococcus aureus* (MRSA), or Methicillin-susceptible *Staphylococcus aureus* (MSSA).

9. A composition for use according to any preceding claim, which does not comprise ozonized honey or ozonated oil.

10. A composition for use according to any preceding claim, which does not contain any added peroxidase.

11. A composition for use according to any preceding claim, which comprises no detectable hydrogen peroxide.

12. A composition for use according to any preceding claim, wherein the composition is a medical grade or medical device grade composition, or a pharmaceutical grade composition.

13. A composition for use according to any preceding claim, which is administered internally or externally to a patient.

14. A composition for use according to any preceding claim, wherein the infection is a wound infection which is critically colonised, optionally wherein the wound has a bacterial load greater than 10⁵ organisms per gram of tissue.

## Patentansprüche

1. Zusammensetzung zum Erzeugen von antimikrobieller Aktivität zur Verwendung bei der Behandlung einer mikrobiellen Infektion, die einen Biofilm umfasst, wobei die Zusammensetzung ein Enzym umfasst, das ein Substrat umwandeln kann, um Wasserstoffperoxid freizusetzen, und eine Substanz, die ein Substrat für das Enzym beinhaltet, wobei die Zusammensetzung nicht genügend freies Wasser enthält, damit das Enzym das Substrat umwandeln kann, wobei das Enzym zusätzlich zu jeglicher Enzymaktivität das Substrat umwandeln kann, um Wasserstoffperoxid freizusetzen, das in der Substanz vorhanden sein kann, wobei die Zusammensetzung optional Wachstum oder Aussaat des Biofilms verhindert oder inhibiert.

2. Zusammensetzung zur Verwendung nach Anspruch 1, wobei:
a) das Enzym ein gereinigtes Enzym ist und die Substanz ein gereinigtes Substrat für das Enzym umfasst;
b) das Enzym ein gereinigtes Enzym ist, die Substanz eine unraffinierte natürliche Substanz ist, die ein Substrat für ein Enzym beinhaltet; oder
c) die Substanz eine unraffinierte natürliche Substanz ist, der Katalaseaktivität fehlt und die ein Substrat für das Enzym beinhaltet.

3. Zusammensetzung zur Verwendung nach Anspruch 2, wobei die unraffinierte natürliche Substanz ein Honig ist.

4. Zusammensetzung zur Verwendung nach einem vorherigen Anspruch, wobei das Enzym Glucoseoxidase ist und das Substrat D-Glucose ist.

5. Zusammensetzung zur Verwendung nach einem vorherigen Anspruch, wobei die Zusammensetzung eine sterile Zusammensetzung ist.

6. Zusammensetzung zur Verwendung nach einem vorherigen Anspruch, wobei die mikrobielle Infektion eine chronische mikrobielle Infektion ist.

7. Zusammensetzung zur Verwendung nach einem vorherigen Anspruch, wobei die mikrobielle Infektion eine Wundinfektion, vorzugsweise eine chronische Wundinfektion ist, wobei die Wundinfektion optional eine Hautwundinfektion oder eine Brandwundinfektion ist.

8. Zusammensetzung zur Verwendung nach einem vorherigen Anspruch, wobei der Biofilm eine beliebige der folgenden Bakterienspezies umfasst: *Pseudomonas aeruginosa, Acinetobacter baumannii,* Methicillin-resistente *Staphylococcus aureus* (MRSA) oder Methicillin-empfängliche *Staphylococcus aureus* (MSSA).

9. Zusammensetzung zur Verwendung nach einem vorherigen Anspruch, die nicht ozonisierten Honig oder ozonisiertes Öl umfasst.

10. Zusammensetzung zur Verwendung nach einem vorherigen Anspruch, die keine zugegebene Peroxidase enthält.

11. Zusammensetzung zur Verwendung nach einem vorherigen Anspruch, die kein nachweisbares Wasserstoffperoxid umfasst.

12. Zusammensetzung zur Verwendung nach einem vorherigen Anspruch, wobei die Zusammensetzung eine Zusammensetzung in medizinischer Qualität, eine Zusammensetzung in Medizinproduktequalität oder eine Zusammensetzung in pharmazeutischer Qualität ist.

13. Zusammensetzung zur Verwendung nach einem vorherigen Anspruch, die intern oder extern einem Patienten verabreicht wird.

14. Zusammensetzung zur Verwendung nach einem vorherigen Anspruch, wobei die Infektion eine Wundinfektion ist, die kritisch besiedelt ist, wobei die Wunde optional eine Bakterienlast von mehr als 10⁵ Organismen pro Gramm Gewebe hat.

## Revendications

1. Composition permettant de générer une activité antimicrobienne destinée à être utilisée dans le traitement d'une infection microbienne comprenant un biofilm, dans laquelle la composition comprend une enzyme capable de convertir un substrat pour libérer du peroxyde d'hydrogène, et une substance comprenant un substrat pour l'enzyme, dans laquelle la composition ne comprend pas suffisamment d'eau libre pour permettre à l'enzyme de convertir le substrat, dans laquelle l'enzyme s'ajoute à toute activité enzymatique capable de convertir le substrat pour libérer du peroxyde d'hydrogène pouvant se trouver dans la substance, éventuellement, dans laquelle la composition empêche ou inhibe la croissance ou l'ensemencement du biofilm.

2. Composition destinée à être utilisée selon la revendication 1, dans laquelle :
a) l'enzyme est une enzyme purifiée et la substance comprend un substrat purifié pour l'enzyme ;
b) l'enzyme est une enzyme purifiée, la substance est une substance naturelle non raffinée comprenant un substrat pour l'enzyme ; ou
c) la substance est une substance naturelle non raffinée, dépourvue d'activité catalase et comprenant un substrat pour l'enzyme.

3. Composition destinée à être utilisée selon la revendication 2, dans laquelle la substance naturelle non raffinée est un miel.

4. Composition destinée à être utilisée selon l'une quelconque des revendications précédentes, dans laquelle l'enzyme est la glucose oxydase et le substrat est le D-glucose.

5. Composition destinée à être utilisée selon l'une quelconque des revendications précédentes, dans laquelle la composition est une composition stérile.

6. Composition destinée à être utilisée selon l'une quelconque des revendications précédentes, dans laquelle l'infection microbienne est une infection microbienne chronique.

7. Composition destinée à être utilisée selon l'une quelconque des revendications précédentes, dans laquelle l'infection microbienne est une infection de plaie, éventuellement, une infection de plaie chronique, éventuellement, dans laquelle l'infection de plaie est une infection de plaie cutanée ou une infection de plaie causée par une brûlure.

8. Composition destinée à être utilisée selon l'une quelconque des revendications précédentes, dans laquelle le biofilm comprend l'une quelconque des espèces de bactéries suivantes : *Pseudomonas aeruginosa ; Acinetobacter baumannii, Staphylococcus aureus* résistant à la méthicilline (SARM) ou *Staphylococcus aureus* sensible à la méthicilline (MSSA).

9. Composition destinée à être utilisée selon l'une quelconque des revendications précédentes, qui ne comprend pas de miel ozonisé ou d'huile ozonée.

10. Composition destinée à être utilisée selon l'une quelconque des revendications précédentes, qui ne contient pas de peroxydase ajoutée.

11. Composition destinée à être utilisée selon l'une quelconque des revendications précédentes, qui ne comprend pas de peroxyde d'hydrogène détectable.

12. Composition destinée à être utilisée selon l'une quelconque des revendications précédentes, dans laquelle la composition est une composition de qualité médicale ou de qualité pour dispositifs médicaux, ou une composition de qualité pharmaceutique.

13. Composition destinée à être utilisée selon l'une quelconque des revendications précédentes, qui est administrée par voie interne ou externe à un patient.

14. Composition destinée à être utilisée selon l'une quelconque des revendications précédentes, dans laquelle l'infection est une infection de plaie qui est colonisée de manière critique, éventuellement dans laquelle la plaie a une charge bactérienne supérieure à 10⁵ organismes par gramme de tissu.
